# EUROPEAN PATENT APPLICATION

(11) **EP 3 778 596 A1**
(43) Date of publication of application: **17.02.2021**
(21) Application number: 19774878.3
(22) Date of filing: 25.03.2019
(51) Int. Cl.: C07D 405/12, H01L 51/50

(54) **COMPOUND, MATERIAL FOR ORGANIC ELECTROLUMINESCENT ELEMENTS, ORGANIC ELECTROLUMINESCENT ELEMENT, AND ELECTRONIC DEVICE**

(30) Priority: 28.03.2018 JP 2018063009; 09.08.2018 JP 2018150596
(71) Applicant: Idemitsu Kosan Co.,Ltd., Chiyoda-ku Tokyo 100-8321 (JP)
(72) Inventor: ITO, Hirokatsu, Sodegaura-shi, Chiba 299-0293 (JP); HAKETA, Tasuku, Sodegaura-shi, Chiba 299-0293 (JP); KUDO, Yu, Sodegaura-shi, Chiba 299-0293 (JP)
(74) Representative: Vossius & Partner Patentanwälte Rechtsanwälte mbB
(86) International application number: PCT/JP2019/012604
(87) International publication number: WO 2019/189033

(57) **Abstract**

A compound represented by formula (1) provides an organic electroluminescence device having lifetime further improved: 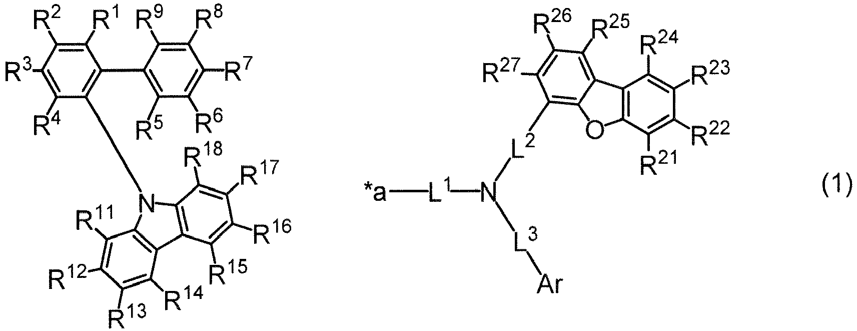 wherein R¹ to R⁹, R¹¹ to R¹⁸, R²¹ to R²⁷, Ar, L¹, L², and L³ are as defined in the description.

## Description

### TECHNICAL FIELD

The present invention relates to compounds, materials for organic electroluminescence devices comprising the compounds, organic electroluminescence devices comprising the compounds, and electronic devices comprising the organic electroluminescence devices.

### BACKGROUND ART

An organic electroluminescence device ("organic EL device") is generally composed of an anode, a cathode, and an organic layer sandwiched between the anode and the cathode. When a voltage is applied between electrodes, electrons are injected from the cathode and holes are injected from the anode into a light emitting region. The injected electrons recombine with the injected holes in the light emitting region to form excited states. When the excited states return to the ground state, the energy is released as light. Therefore, it is important for obtaining an organic EL device with high efficiency to develop a compound that transports electrons or holes into the light emitting region efficiently and facilitates the recombination of electrons and holes.

Patent Literature 1 describes an amine compound wherein one 4-biphenylyl group having a N-carbazolyl group on the terminal phenyl group at its o-position and two aryl groups are bonded to the central nitrogen atom directly or via a linker.

Patent Literature 2 describes an amine compound wherein one 2-(N-carbazolyl)phenyl group, one 2-dibenzofuranyl group, and one group having an azine ring or an aryl group having an azine ring are bonded to the central nitrogen atom.

Patent Literature 3 describes an amine compound wherein one 4-biphenylyl group having a N-carbazolyl group on the terminal phenyl group on its o-position and two 4-biphenylyl groups are bonded to the central nitrogen atom.

Patent Literature 4 describes an amine compound wherein one 4-biphenylyl group having a N-carbazolyl group on the terminal phenyl group at its m-position, one 4-(4-dibenzofuranyl)phenyl group, and one 4-biphenylyl group are bonded to the central nitrogen atom.

### CITATION LIST

### PATENT LITERATURE

Patent Literature 1: WO 2017/052261 A1
Patent Literature 2: KR 10-2017-0072041
Patent Literature 3: US 2016/0322580 A1
Patent Literature 4: US 2016/0079542 A1

### SUMMARY OF INVENTION

### TECHNICAL PROBLEM

Various compounds have been reported as materials for the production of organic EL devices. However, compounds that further improve the performance of organic EL devices have been still demanded.

The present invention has been made to solve the above problem and an object of the invention is to provide organic EL devices having further improved lifetime and novel compounds providing such organic EL devices.

### SOLUTION TO PROBLEM

The inventors have extensively studied to solve the above problems and found that the compounds represented by formula (1) provide organic EL devices having further improved lifetime.

In an aspect, the present invention provides a compound represented by formula (1) (hereinafter also referred to as "compound (1)"): 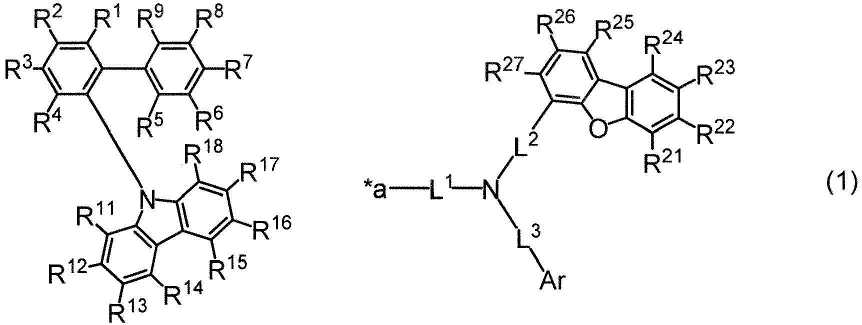 wherein:
one of R⁷, R⁸, and R⁹ is a single bond bonded to *a;
R¹ to R⁶ and R⁷ to R⁹ not the single bond bonded to *a are each independently a hydrogen atom or a substituent,
wherein the substituent is selected from the group consisting of a halogen atom, a cyano group, a nitro group, a substituted or unsubstituted alkyl group having 1 to 30 carbon atoms, a substituted or unsubstituted cycloalkyl group having 3 to 30 ring carbon atoms, a substituted or unsubstituted aryl group having 6 to 30 ring carbon atoms, a substituted or unsubstituted aralkyl group having 7 to 36 carbon atoms, a substituted or unsubstituted alkoxy group having 1 to 30 carbon atoms, a substituted or unsubstituted aryloxy group having 6 to 30 ring carbon atoms, a mono, di or tri-substituted silyl group having a substituent selected from a substituted or unsubstituted alkyl group having 1 to 30 carbon atoms and a substituted or unsubstituted aryl group having 6 to 30 ring carbon atoms, a substituted or unsubstituted haloalkyl group having 1 to 30 carbon atoms, a substituted or unsubstituted haloalkoxy group having 1 to 30 carbon atoms, and a substituted or unsubstituted heteroaryl group having 5 to 30 ring atoms,
provided that adjacent two selected from R¹ to R⁶ and R⁷ to R⁹ not the single bond bonded to *a are each independently a hydrogen atom or the substituent, or the adjacent two are bonded to each other to form a ring structure;
R¹¹ to R¹⁸ are each independently a hydrogen atom or a substituent,
wherein the substituent is selected from the group consisting of a halogen atom, a cyano group, a nitro group, a substituted or unsubstituted alkyl group having 1 to 30 carbon atoms, a substituted or unsubstituted cycloalkyl group having 3 to 30 ring carbon atoms, a substituted or unsubstituted aryl group having 6 to 30 ring carbon atoms, a substituted or unsubstituted aralkyl group having 7 to 36 carbon atoms, a substituted or unsubstituted alkoxy group having 1 to 30 carbon atoms, a substituted or unsubstituted aryloxy group having 6 to 30 ring carbon atoms, a mono, di or tri-substituted silyl group having a substituent selected from a substituted or unsubstituted alkyl group having 1 to 30 carbon atoms and a substituted or unsubstituted aryl group having 6 to 30 ring carbon atoms, a substituted or unsubstituted haloalkyl group having 1 to 30 carbon atoms, a substituted or unsubstituted haloalkoxy group having 1 to 30 carbon atoms, and a substituted or unsubstituted heteroaryl group having 5 to 30 ring atoms,
provided that adjacent two selected from R¹¹ to R¹⁸ are each independently a hydrogen atom or the substituent, or the adjacent two are bonded to each other to form a ring structure;
R²¹ to R²⁷ are each independently a hydrogen atom or a substituent,
wherein the substituent is selected from the group consisting of a halogen atom, a cyano group, a nitro group, a substituted or unsubstituted alkyl group having 1 to 30 carbon atoms, a substituted or unsubstituted cycloalkyl group having 3 to 30 ring carbon atoms, a substituted or unsubstituted aryl group having 6 to 30 ring carbon atoms, a substituted or unsubstituted aralkyl group having 7 to 36 carbon atoms, a substituted or unsubstituted alkoxy group having 1 to 30 carbon atoms, a substituted or unsubstituted aryloxy group having 6 to 30 ring carbon atoms, a mono, di or tri-substituted silyl group having a substituent selected from a substituted or unsubstituted alkyl group having 1 to 30 carbon atoms and a substituted or unsubstituted aryl group having 6 to 30 ring carbon atoms, a substituted or unsubstituted haloalkyl group having 1 to 30 carbon atoms, a substituted or unsubstituted haloalkoxy group having 1 to 30 carbon atoms, and a substituted or unsubstituted heteroaryl group having 5 to 30 ring atoms,
provided that adjacent two selected from R²¹ to R²⁷ are each independently a hydrogen atom or the substituent, or the adjacent two are bonded to each other to form a ring structure;
Ar is a substituted or unsubstituted aryl group having 6 to 30 ring carbon atoms;
L¹ is a single bond or L¹¹, wherein L¹¹ is a substituted or unsubstituted arylene group having 6 to 30 ring carbon atoms;
L² is a single bond or L²², wherein L²² is a substituted or unsubstituted arylene group having 6 to 30 ring carbon atoms; and
L³ is a single bond or L³³, wherein L³³ is a substituted or unsubstituted arylene group having 6 to 30 ring carbon atoms.

In another aspect, the present invention provides a material for organic EL device comprising the compound (1).

In another aspect, the present invention provides an organic electroluminescence device comprising an anode, a cathode, and an organic layer disposed between the anode and the cathode, wherein the organic layer comprises a light emitting layer and at least one layer of the organic layer comprises the compound (1).

In another aspect, the present invention provides an electronic device comprising the organic electroluminescence device.

### ADVANTAGEOUS EFFECTS OF INVENTION

The compound (1) provides an organic EL device having a further improved lifetime.

### BRIEF DESCRIPTION OF DRAWINGS

Fig. 1 is a schematic view showing an example of the layered structure of an organic EL device in an embodiment of the invention.
Fig. 2 is a schematic view showing another example of the layered structure of an organic EL device in an embodiment of the invention.

### DESCRIPTION OF EMBODIMENTS

The term of "XX to YY carbon atoms" referred to by "a substituted or unsubstituted group ZZ having XX to YY carbon atoms" used herein is the number of carbon atoms of the unsubstituted group ZZ and does not include any carbon atom in the substituent of the substituted group ZZ.

The term of "XX to YY atoms" referred to by "a substituted or unsubstituted group ZZ having XX to YY atoms" used herein is the number of atoms of the unsubstituted group ZZ and does not include any atom in the substituent of the substituted group ZZ.

The term of "unsubstituted group ZZ" referred to by "substituted or unsubstituted group ZZ" used herein means that no hydrogen atom in the group ZZ is substituted by a substituent.

The definition of "hydrogen atom" used herein includes isotopes different in the neutron numbers, i.e., light hydrogen (protium), heavy hydrogen (deuterium), and tritium.

The number of ring carbon atoms referred to herein means the number of the carbon atoms included in the atoms that form the ring itself of a compound in which a series of atoms are bonded to form a cyclic compound (for example, a monocyclic compound, a fused ring compound, a cross-linked compound, a carbocyclic compound, and a heterocyclic compound). If the ring has a substituent, the carbon atom in the substituent is not included in the ring carbon atom. Unless otherwise noted, the same applies to the number of ring carbon atoms mentioned below.

For example, a benzene ring has 6 ring carbon atoms, a naphthalene ring has 10 ring carbon atoms, a pyridinyl group has 5 ring carbon atoms, a furan ring has 4 ring carbon atoms. If a benzene ring or a naphthalene ring has, for example, an alkyl substituent, the carbon atom in the alkyl substituent is not counted as the ring carbon atom. When a fluorene ring has a fluorene substituent (inclusive of a spirofluorene ring), the carbon atoms in the fluorene substituent is not counted as the ring carbon atom.

The number of ring atom referred to herein means the number of the atoms which forms the ring itself (for example, a monocyclic ring, a fused ring, and a ring assembly) of a compound in which a series of atoms is bonded to form the ring (for example, a monocyclic compound, a fused ring compound, a cross-linked compound, a carbocyclic compound, and a heterocyclic compound). The atom not forming the ring (for example, hydrogen atom(s) on the ring atom) and the atom in a substituent, if the ring is substituted, are not counted as the ring atom. The same applies to the number of ring atoms described below, unless otherwise noted. For example, a pyridine ring has 6 ring atoms, a quinazoline ring has 10 ring atoms, and a furan ring has 5 ring atoms. The hydrogen atom on the ring carbon atom of a pyridine ring or a quinazoline ring and the atom in a substituent are not counted as the ring atom. When a fluorene ring has a fluorene substituent (inclusive of a spirofluorene ring), the atom in the fluorene substituent is not counted as the ring atom of the fluorene ring.

The compound (1) in an aspect of the invention is represented by formula (1):

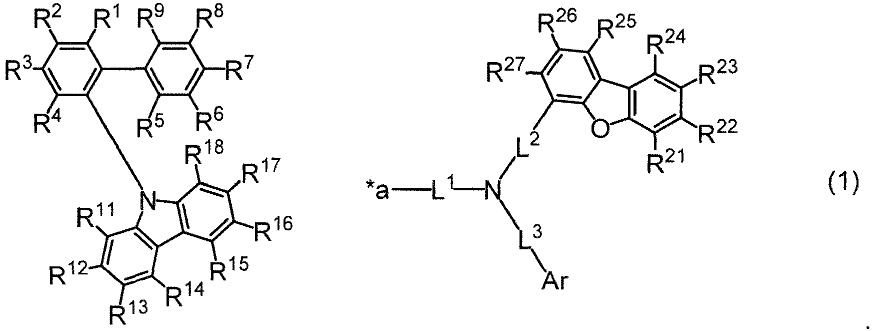

In an embodiment of the invention, the compound (1) is preferably represented by formula (2):

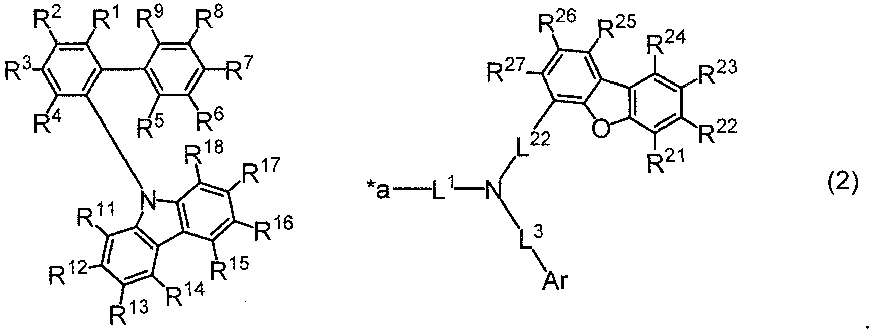

In an embodiment of the invention, the compound (1) is preferably represented by any of formulae (3) to (6):

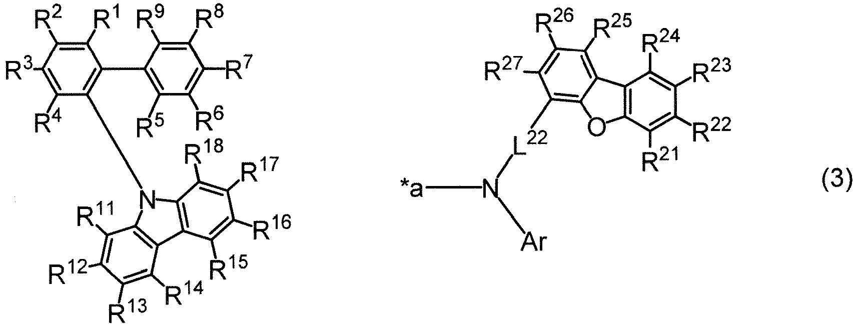

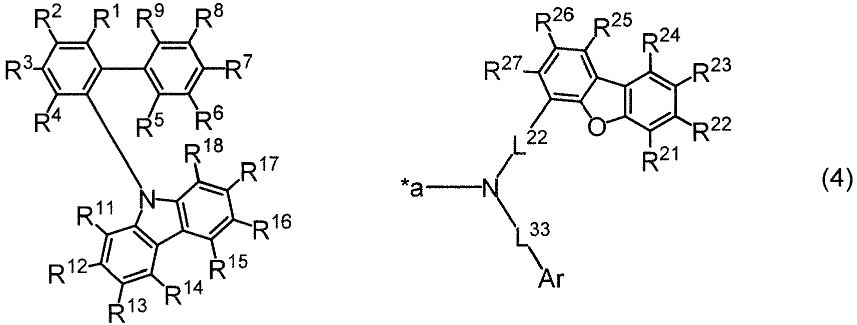

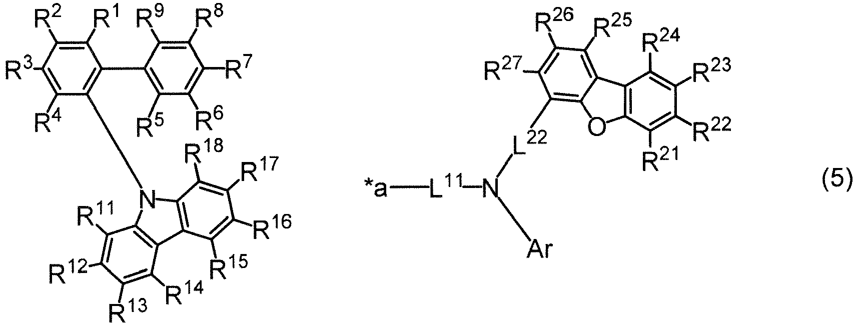

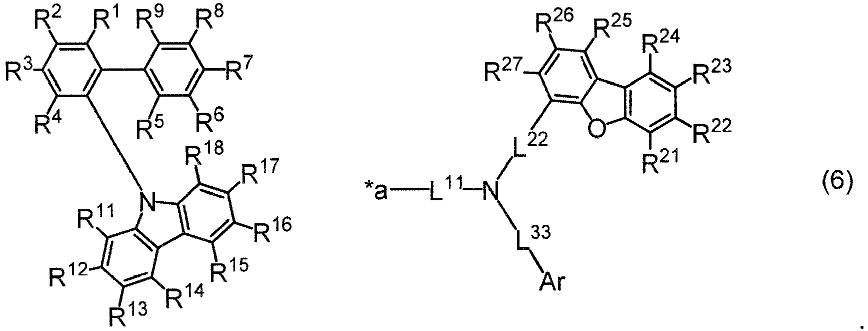

In an embodiment of the invention, the compound (1) is preferably represented by formula (3) or (4).

Each symbol in formulae (1) to (6) and formulae (7) to (12) described below will be described below.

One of R⁷, R⁸, and R⁹ is a single bond bonded to *a. Namely, the compound (1) is a compound wherein R⁷ is the single bond bonded to *a, a compound wherein R⁸ is the single bond bonded to *a, or a compound wherein R⁹ is the single bond bonded to *a. A compound wherein R⁷ or R⁸ is the single bond bonded to *a is preferred and a compound wherein R⁷ is the single bond bonded to *a is more preferred.

A compound wherein R⁷ is the single bond bonded to *a is represented by formula (7):

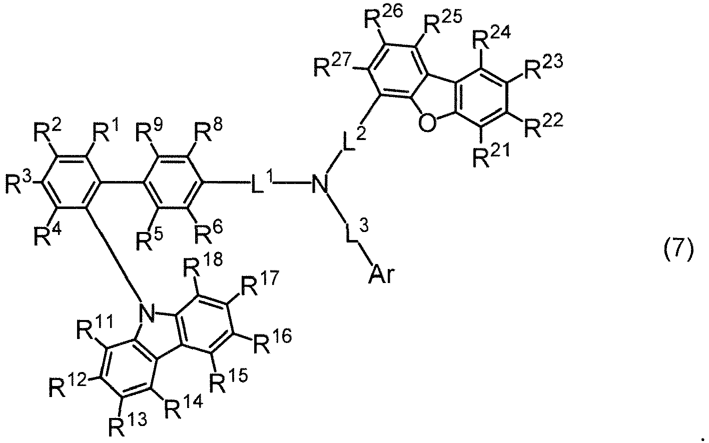

Formula (7) is preferably represented by formula (8):

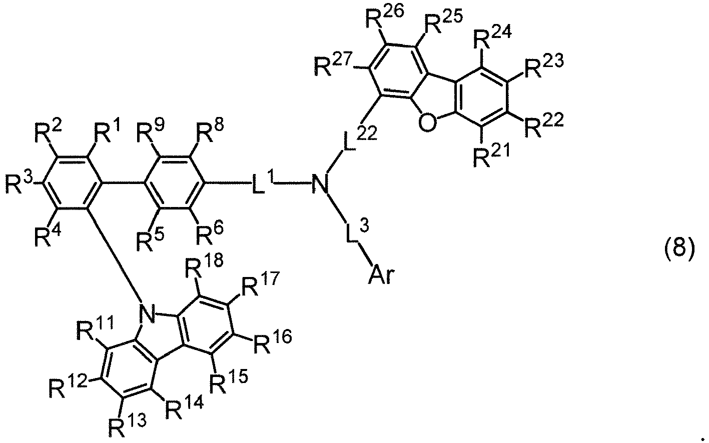

A compound wherein R⁸ is the single bond bonded to *a is represented by formula (9):

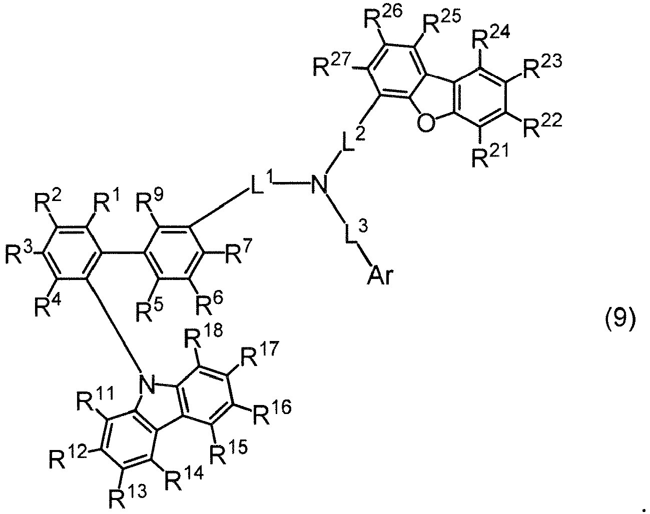

Formula (9) is preferably represented by formula (10):

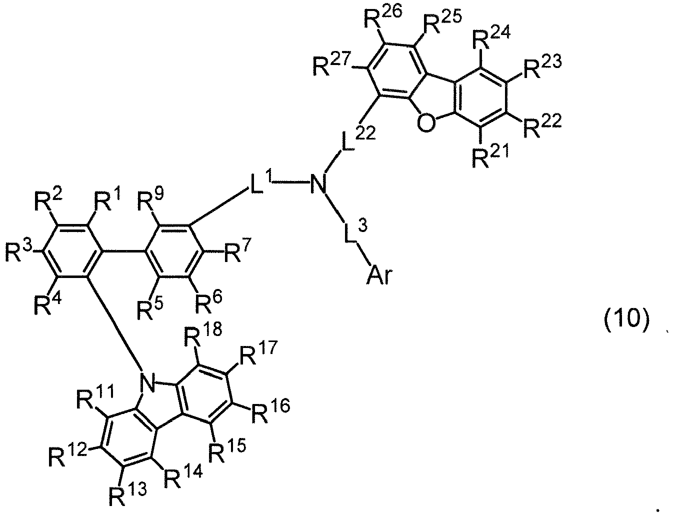

A compound wherein R⁹ is the single bond bonded to *a is represented by formula (11):

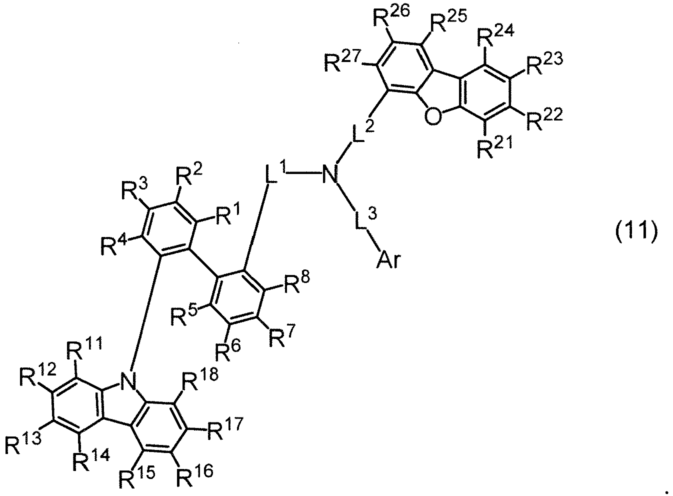

Formula (11) is preferably represented by formula (12):

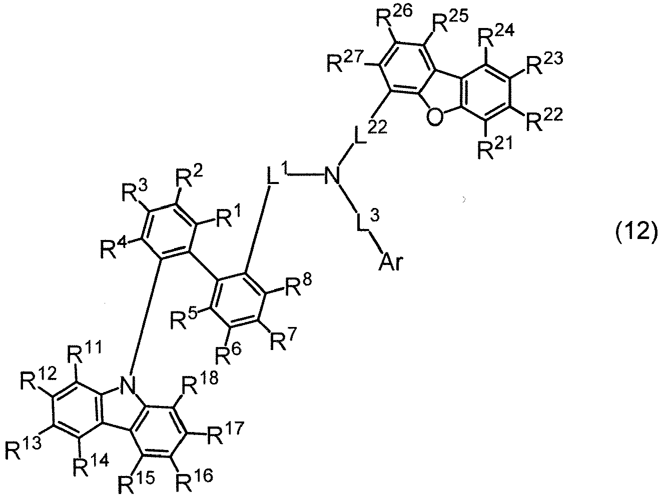

R¹ to R⁶ and R⁷ to R⁹ not the single bond bonded to *a are each independently a hydrogen atom or a substituent, wherein the substituent is selected from the group consisting of a halogen atom; a cyano group; a nitro group; a substituted or unsubstituted alkyl group having 1 to 30, preferably 1 to 18, more preferably 1 to 8 carbon atoms; a substituted or unsubstituted cycloalkyl group having 3 to 30, preferably 3 to 10, more preferably 3 to 8, still more preferably 5 or 6 ring carbon atoms; a substituted or unsubstituted aryl group having 6 to 30, preferably 6 to 25, more preferably 6 to 18 ring carbon atoms; a substituted or unsubstituted aralkyl group having 7 to 36, preferably 7 to 26, more preferably 7 to 20 carbon atoms; a substituted or unsubstituted alkoxy group having 1 to 30, preferably 1 to 18, more preferably 1 to 8 carbon atoms; a substituted or unsubstituted aryloxy group having 6 to 30, preferably 6 to 25, more preferably 6 to 18 ring carbon atoms; a mono, di or tri-substituted silyl group having a substituent selected from a substituted or unsubstituted alkyl group having 1 to 30, preferably 1 to 18, more preferably 1 to 8 carbon atoms and a substituted or unsubstituted aryl group having 6 to 30, preferably 6 to 25, more preferably 6 to 18 ring carbon atoms; a substituted or unsubstituted haloalkyl group having 1 to 30, preferably 1 to 18, more preferably 1 to 8 carbon atoms; a substituted or unsubstituted haloalkoxy group having 1 to 30, preferably 1 to 18, more preferably 1 to 8 carbon atoms; and a substituted or unsubstituted heteroaryl group having 5 to 30, preferably 5 to 24, more preferably 5 to 13 ring atoms.

Provided that adjacent two selected from R¹ to R⁶ and R⁷ to R⁹ not the single bond bonded to *a are each independently a hydrogen atom or the substituent, or the adjacent two are bonded to each other to form a ring structure. Namely, the adjacent two are each independently a hydrogen atom or the substituent without forming a ring structure or the adjacent two are bonded to each other to form a ring structure.

Thus, R¹ to R⁶ and R⁷ to R⁹ not the single bond bonded to *a are each independently a hydrogen atom or the substituent or bonded to an adjacent one selected from R¹ to R⁹ to form a ring structure.

The substituent is preferably selected from the group consisting of a substituted or unsubstituted alkyl group having 1 to 30 carbon atoms, a substituted or unsubstituted cycloalkyl group having 3 to 30 ring carbon atoms, a substituted or unsubstituted aryl group having 6 to 30 ring carbon atoms, a mono, di or tri-substituted silyl group having a substituent selected from a substituted or unsubstituted alkyl group having 1 to 30 carbon atoms and a substituted or unsubstituted aryl group having 6 to 30 ring carbon atoms, and a substituted or unsubstituted heteroaryl group having 5 to 30 ring atoms. The substituent is more preferably selected from the group consisting of a substituted or unsubstituted alkyl group having 1 to 30 carbon atoms, a substituted or unsubstituted aryl group having 6 to 30 ring carbon atoms, and a substituted or unsubstituted heteroaryl group having 5 to 30 ring atoms.

In an embodiment of the invention, adjacent two selected from R¹ to R⁶ and R⁷ to R⁹ not the single bond bonded to *a are each independently a hydrogen atom or the substituent without forming a ring structure. In another embodiment of the invention, at least one set of adjacent two are bonded to each other to form at least one ring structure.

The halogen atom is a fluorine atom, a chlorine atom, a bromine atom, or an iodine atom, with a fluorine atom is being preferred.

Examples of the alkyl group having 1 to 30 carbon atoms of the substituted or unsubstituted alkyl group having 1 to 30 carbon atoms include a methyl group, an ethyl group, a n-propyl group, an isopropyl group, a n-butyl group, an isobutyl group, a s-butyl group, a t-butyl group, a pentyl group, a hexyl group, a heptyl group, an octyl group, a nonyl group, a decyl group, an undecyl group, and a dodecyl group. Preferred are a methyl group, an ethyl group, a n-propyl group, an isopropyl group, a n-butyl group, an isobutyl group, a s-butyl group, a t-butyl group, and a pentyl group. More preferred are a methyl group, an ethyl group, a n-propyl group, an isopropyl group, a n-butyl group, an isobutyl group, a s-butyl group, and a t-butyl group. Still more preferred are a methyl group and a t-butyl group.

The substituted or unsubstituted alkyl group having 1 to 30 carbon atoms includes isomeric groups, if present.

Examples of the cycloalkyl group having 3 to 30 ring carbon atoms of the substituted or unsubstituted cycloalkyl group having 3 to 30 ring carbon atoms include a cyclopropyl group, a cyclobutyl group, a cyclopentyl group, a cyclohexyl group, and a cycloheptyl group, with a cyclopentyl group and a cyclohexyl group being preferred.

The substituted or unsubstituted cycloalkyl group having 3 to 30 ring carbon atoms includes isomeric groups, if present.

Examples of the aryl group having 6 to 30 ring carbon atoms of the substituted or unsubstituted aryl group having 6 to 30 ring carbon atoms include a phenyl group, a biphenylyl group, a terphenylyl group, a biphenylenyl group, a naphthyl group, an anthryl group, a benzanthryl group, a phenanthryl group, a benzophenanthryl group, a phenalenyl group, a picenyl group, a pentaphenyl group, a pyrenyl group, a chrysenyl group, a benzochrysenyl group, a fluorenyl group, a fluoranthenyl group, a perylenyl group, and a triphenylenyl group. Preferred are a phenyl group, a biphenylyl group, a terphenylyl group, and a naphthyl group. More preferred are a phenyl group, a 2-, 3-, or 4-biphenylyl group, a 2-, 3-, or 4-o-terphenylyl group, a 2-, 3-, or 4-m-terphenylyl group, a 2-, 3-, or 4-p-terphenylyl group, and a 1- or 2-naphthyl group. Still more preferred are a phenyl group, a 2-, 3-, or 4-biphenylyl group, and a 1- or 2-naphthyl group, with a phenyl group being particularly preferred.

Examples of the substituted aryl group having 6 to 30 ring carbon atoms include a tolyl group, a t-butylphenyl group, a 9,9-dimethylfluorenyl group, a 9,9-diphenylfluorenyl group, and a spirobifluorenyl group.

The substituted or unsubstituted aryl group having 6 to 30 ring carbon atoms includes isomeric groups, if present.

In the aralkyl group having 7 to 36 carbon atoms of the substituted or unsubstituted aralkyl group having 7 to 36 carbon atoms, the aryl portion is selected from the above aryl group having 6 to 30, preferably 6 to 25, more preferably 6 to 18 ring carbon atoms and the alkyl portion is selected from the above substituted or unsubstituted alkyl group having 1 to 30, preferably 1 to 18, more preferably 1 to 8 carbon atoms. Examples of the aralkyl group having 7 to 36 carbon atoms include a benzyl group, a phenethyl group, a phenylpropyl group, with a benzyl group being preferred.

The substituted or unsubstituted aralkyl group having 7 to 36 carbon atoms includes isomeric groups, if present.

In the alkoxy group having 1 to 30 carbon atoms of the substituted or unsubstituted alkoxy group having 1 to 30 carbon atoms, the alkyl portion is selected from the above substituted or unsubstituted alkyl group having 1 to 30, preferably 1 to 18, more preferably 1 to 8 carbon atoms. Examples of the alkoxy group having 1 to 30 carbon atoms include a t-butoxy group, a propoxy group, an ethoxy group, and a methoxy group, with an ethoxy group and a methoxy group being preferred and a methoxy group being more preferred.

The substituted or unsubstituted alkoxy group having 1 to 30 carbon atoms includes isomeric groups, if present.

In the aryloxy group having 6 to 30 ring carbon atoms of the substituted or unsubstituted aryloxy group having 6 to 30 ring carbon atoms, the aryl portion is selected from the above substituted or unsubstituted aryl group having 6 to 30, preferably 6 to 25, more preferably 6 to 18 ring carbon atoms. Examples of the aryloxy group having 6 to 30 ring carbon atoms include a terphenyloxy group, a biphenyloxy group and a phenoxy group, with a biphenyloxy group and a phenoxy group being preferred and a phenoxy group being more preferred.

The substituted or unsubstituted aryloxy group having 6 to 30 ring carbon atoms includes isomeric groups, if present.

The substituent of the mono, di or tri-substituted silyl group is selected from the above substituted or unsubstituted alkyl group having 1 to 30, preferably 1 to 18, more preferably 1 to 8 carbon atoms and the above substituted or unsubstituted aryl group having 6 to 30, preferably 6 to 25, more preferably 6 to 18 ring carbon atoms. Preferred is a tri-substituted silyl group, with a trimethylsilyl group, a triethylsilyl group, a t-butyldimethylsilyl group, a propyldimethylsilyl group, an isopropyldimethylsilyl group, a triphenylsilyl group, a phenyldimethylsilyl group, a t-butyldiphenylsilyl group, and a tritolylsilyl group being more preferred.

The mono, di or tri-substituted silyl group includes isomeric groups, if present.

The haloalkyl group having 1 to 30 carbon atoms of the substituted or unsubstituted haloalkyl group having 1 to 30 carbon atoms is a group derived from the above alkyl group having 1 to 30, preferably 1 to 18, more preferably 1 to 8 alkyl group by substituting a halogen atom for at least one hydrogen atom, preferably 1 to 7 hydrogen atoms or all hydrogen atoms. The halogen atom is selected from a fluorine atom, a chlorine atom, a bromine atom, and an iodine atom, with a fluorine atom being preferred. The haloalkyl group is preferably a fluoroalkyl group 1 to 30, preferably 1 to 18, more preferably 1 to 8 carbon atoms, more preferably a heptafluoropropyl group, a pentafluoroethyl group, a 2,2,2-trifluoroethyl group, or a trifluoromethyl group, with a pentafluoroethyl group, a 2,2,2-trifluoroethyl group, and a trifluoromethyl group being still more preferred and a trifluoromethyl group being particularly preferred.

The substituted or unsubstituted haloalkyl group having 1 to 30 carbon atoms includes isomeric groups, if present.

In the haloalkoxy group having 1 to 30 carbon atoms of the substituted or unsubstituted haloalkoxy group having 1 to 30 carbon atoms, the haloalkyl portion is selected from the above haloalkyl group having 1 to 30, preferably 1 to 18, more preferably 1 to 8 carbon atoms. The haloalkoxy group is preferably a fluoroalkoxy group having 1 to 30, preferably 1 to 18, more preferably 1 to 8 carbon atoms, more preferably a heptafluoropropoxy group, a pentafluoroethoxy group, a 2,2,2-trifluoroethoxy group, or a trifluoromethoxy group, still more preferably a pentafluoroethoxy group, a 2,2,2-trifluoroethoxy group, or a trifluoromethoxy group, with a trifluoromethoxy group being particularly preferred.

The substituted or unsubstituted haloalkoxy group having 1 to 30 carbon atoms includes isomeric groups, if present.

The heteroaryl group having 5 to 30 ring atoms of the substituted or unsubstituted heteroaryl group having 5 to 30 ring atoms includes 1 to 5, preferably 1 to 3, more preferably 1 to 2 ring hetero atoms. The ring hetero atom is selected from, for example, a nitrogen atom, a sulfur atom, and an oxygen atom. The free valence of the heteroaryl group is present on a ring carbon atom or on a ring nitrogen atom, if physically possible.

Examples of the heteroaryl group having 5 to 30 ring atoms include a pyrrolyl group, a furyl group, a thienyl group, a pyridyl group, an imidazopyridyl group, a pyridazinyl group, a pyrimidinyl group, a pyrazinyl group, a triazinyl group, an imidazolyl group, an oxazolyl group, a thiazolyl group, a pyrazolyl group, an isoxazolyl group, an isothiazolyl group, an oxadiazolyl group, a thiadiazolyl group, a triazolyl group, a tetrazolyl group, an indolyl group, an isoindolyl group, an indolizinyl group, a quinolizinyl group, a quinolyl group, an isoquinolyl group, a cinnolyl group, a phthalazinyl group, a quinazolinyl group, a quinoxalinyl group, a benzimidazolyl group, a benzoxazolyl group, a benzothiazolyl group, an indazolyl group, a benzisoxazolyl group, a benzisothiazolyl group, a phenanthridinyl group, an acridinyl group, a phenanthrolinyl group, a phenazinyl group, a phenothiazinyl group, a phenoxazinyl group, a xanthenyl group, a benzofuranyl group, an isobenzofuranyl group, a naphthobenzofuranyl group, a dibenzofuranyl group, a benzothiophenyl group (a benzothienyl group, the same applies below), an isobenzothiophenyl group (an isobenzothienyl group, the same applies below), a naphthobenzothiophenyl group (a naphthobenzothienyl group, the same applies below), a dibenzothiophenyl group (a dibenzothienyl group, the same applies below), and a carbazolyl group.

Preferred are a benzofuranyl group, an isobenzofuranyl group, a naphthobenzofuranyl group, a dibenzofuranyl group, a benzothiophenyl group, an isobenzothiophenyl group, a naphthobenzothiophenyl group, a dibenzothiophenyl group, and a carbazolyl group (9-carbazolyl group or a 1-, 2-, 3-, or 4-carbazolyl group).

Examples of a substituted heteroaryl group having 5 to 30 ring atoms include a 9-phenylcarbazolyl group, a 9-biphenylylcarbazolyl group, a 9-phenylphenylcarbazolyl group, a 9-naphthylcarbazolyl group, a phenyldibenzofuranyl group, and a phenyldibenzothiophenyl group (a phenyldibenzothienyl group, the same applies below).

The substituted or unsubstituted heteroaryl group having 5 to 30 ring atoms includes isomeric groups, if present.

The ring structure that is formed by adjacent two selected from R¹ to R⁶ and R⁷ to R⁹ not the single bond bonded to *a and two ring carbon atoms to which the adjacent two are bonded is, for example, a substituted or unsubstituted aromatic hydrocarbon ring having 6 to 18 ring carbon atoms, a substituted or unsubstituted aliphatic hydrocarbon ring having 5 to 18 ring carbon atoms, a substituted or unsubstituted aromatic heterocyclic ring having 5 to 18 ring atoms, or a substituted or unsubstituted aliphatic heterocyclic ring having 5 to 18 ring atoms.

The aromatic hydrocarbon ring having 6 to 18 ring carbon atoms is, for example, a benzene ring, a biphenylene ring, a naphthalene ring, an anthracene ring, a benzanthracene ring, a phenanthrene ring, a benzophenanthrene ring, a phenalene ring, a pyrene ring, a chrysene ring, or a triphenylene ring, with a benzene ring and a naphthalene ring being preferred.

The aliphatic hydrocarbon ring having 5 to 18 ring carbon atoms is, for example, a cyclopentene ring, a cyclopentadiene ring, a cyclohexene ring, a cyclohexadiene ring, or an aliphatic ring obtained by partially hydrogenating the above aromatic hydrocarbon ring having 6 to 18 ring carbon atoms.

The aromatic heterocyclic ring having 5 to 18 ring atoms is, for example, a pyrrole ring, a furan ring, a thiophene ring, a pyridine ring, an imidazole ring, a pyrazole ring, an indole ring, an isoindole ring, a benzofuran ring, an isobenzofuran ring, a benzothiophene ring, a benzimidazole ring, an indazole ring, a dibenzofuran ring, a naphthobenzofuran ring, a dibenzothiophene ring, a naphthobenzothiophene ring, a carbazole ring, or a benzocarbazole ring.

The aliphatic heterocyclic ring having 5 to 18 ring atoms is, for example, an aliphatic heterocyclic ring obtained by partially hydrogenating the above aromatic heterocyclic ring having 5 to 18 ring atoms.

In an embodiment of the invention, R¹ to R⁶ and R⁷ to R⁹ not the single bond bonded to *a are preferably all hydrogen atoms.

R¹¹ to R¹⁸ are each independently a hydrogen atom or a substituent, wherein the substituent is selected from the group consisting of a halogen atom; a cyano group; a nitro group; a substituted or unsubstituted alkyl group having 1 to 30, preferably 1 to 18, more preferably 1 to 8 carbon atoms; a substituted or unsubstituted cycloalkyl group having 3 to 30, preferably 3 to 10, more preferably 3 to 8, still more preferably 5 or 6 ring carbon atoms; a substituted or unsubstituted aryl group having 6 to 30, preferably 6 to 25, more preferably 6 to 18 ring carbon atoms; a substituted or unsubstituted aralkyl group having 7 to 36, preferably 7 to 26, more preferably 7 to 20 carbon atoms; a substituted or unsubstituted alkoxy group having 1 to 30, preferably 1 to 18, more preferably 1 to 8 carbon atoms; a substituted or unsubstituted aryloxy group having 6 to 30, preferably 6 to 25, more preferably 6 to 18 ring carbon atoms; a mono, di or tri-substituted silyl group having a substituent selected from a substituted or unsubstituted alkyl group having 1 to 30, preferably 1 to 18, more preferably 1 to 8 carbon atoms and a substituted or unsubstituted aryl group having 6 to 30, preferably 6 to 25, more preferably 6 to 18 ring carbon atoms; a substituted or unsubstituted haloalkyl group having 1 to 30, preferably 1 to 18, more preferably 1 to 8 carbon atoms; a substituted or unsubstituted haloalkoxy group having 1 to 30, preferably 1 to 18, more preferably 1 to 8 carbon atoms; and a substituted or unsubstituted heteroaryl group having 5 to 30, preferably 5 to 24, more preferably 5 to 13 ring atoms.

Provided that adjacent two selected from R¹¹ to R¹⁸ are each independently a hydrogen atom or the substituent, or the adjacent two are bonded to each other to form a ring structure. Namely, the adjacent two are each independently a hydrogen atom or the substituent without forming a ring structure, or the adjacent two are bonded to each other to form a ring structure.

Thus, R¹¹ to R¹⁸ are each independently a hydrogen atom or the substituent, or bonded to adjacent one selected from R¹¹ to R¹⁸ to from a ring structure.

The substituent represented by R¹¹ to R¹⁸ is preferably selected from the group consisting of a substituted or unsubstituted alkyl group having 1 to 30 carbon atoms, a substituted or unsubstituted cycloalkyl group having 3 to 30 ring carbon atoms, a substituted or unsubstituted aryl group having 6 to 30 ring carbon atoms, a mono, di or tri-substituted silyl group having a substituent selected from a substituted or unsubstituted alkyl group having 1 to 30 carbon atoms and a substituted or unsubstituted aryl group having 6 to 30 ring carbon atoms, and a substituted or unsubstituted heteroaryl group having 5 to 30 ring atoms and more preferably selected from the group consisting of a substituted or unsubstituted alkyl group having 1 to 30 carbon atoms, a substituted or unsubstituted aryl group having 6 to 30 ring carbon atoms, and a substituted or unsubstituted heteroaryl group having 5 to 30 ring atoms.

The details of the substituents represented by R¹¹ to R¹⁸ are the same as the details of the corresponding substituents mentioned above with respect to R¹ to R⁶ and R⁷ to R⁹ not the single bond bonded to *a.

In an embodiment of the invention, adjacent two selected from R¹¹ to R¹⁸ are each independently a hydrogen atom or the substituent without forming a ring structure. In another embodiment of the invention, at least one set of adjacent two are bonded to each other to form at least one ring structure.

The details of the ring structure that is formed by adjacent two selected from R¹¹ to R¹⁸ and two ring carbon atoms to which the adjacent two are bonded are the same as the details of the ring structure mentioned above with respect to R¹ to R⁶ and R⁷ to R⁹ not the single bond bonded to *a.

In an embodiment of the invention, R¹¹ to R¹⁸ are preferably all hydrogen atoms.

R²¹ to R²⁷ are each independently a hydrogen atom or a substituent, wherein the substituent is selected from the group consisting of a halogen atom; a cyano group; a nitro group; a substituted or unsubstituted alkyl group having 1 to 30, preferably 1 to 18, more preferably 1 to 8 carbon atoms; a substituted or unsubstituted cycloalkyl group having 3 to 30, preferably 3 to 10, more preferably 3 to 8, still more preferably 5 or 6 ring carbon atoms; a substituted or unsubstituted aryl group having 6 to 30, preferably 6 to 25, more preferably 6 to 18 ring carbon atoms; a substituted or unsubstituted aralkyl group having 7 to 36, preferably 7 to 26, more preferably 7 to 20 carbon atoms; a substituted or unsubstituted alkoxy group having 1 to 30, preferably 1 to 18, more preferably 1 to 8 carbon atoms; a substituted or unsubstituted aryloxy group having 6 to 30, preferably 6 to 25, more preferably 6 to 18 ring carbon atoms; a mono, di or tri-substituted silyl group having a substituent selected from a substituted or unsubstituted alkyl group having 1 to 30, preferably 1 to 18, more preferably 1 to 8 carbon atoms and a substituted or unsubstituted aryl group having 6 to 30, preferably 6 to 25, more preferably 6 to 18 ring carbon atoms; a substituted or unsubstituted haloalkyl group having 1 to 30, preferably 1 to 18, more preferably 1 to 8 carbon atoms; a substituted or unsubstituted haloalkoxy group having 1 to 30, preferably 1 to 18, more preferably 1 to 8 carbon atoms; and a substituted or unsubstituted heteroaryl group having 5 to 30, preferably 5 to 24, more preferably 5 to 13 ring atoms.

Provided that adjacent two selected from R²¹ to R²⁷ are each independently a hydrogen atom or the substituent or the adjacent two are bonded to each other to form a ring structure. Namely, the adjacent two are each independently a hydrogen atom or the substituent without forming a ring structure or the adjacent two are bonded to each other to form a ring structure.

Thus, R²¹ to R²⁷ are each independently a hydrogen atom or the substituent or bonded to adjacent one selected from R²¹ to R²⁷ to form a ring structure.

The substituent represented by R²¹ to R²⁷ is preferably selected from the group consisting of a substituted or unsubstituted alkyl group having 1 to 30 carbon atoms, a substituted or unsubstituted cycloalkyl group having 3 to 30 ring carbon atoms, a substituted or unsubstituted aryl group having 6 to 30 ring carbon atoms, a mono, di or tri-substituted silyl group having a substituent selected from a substituted or unsubstituted alkyl group having 1 to 30 carbon atoms and a substituted or unsubstituted aryl group having 6 to 30 ring carbon atoms, and a substituted or unsubstituted heteroaryl group having 5 to 30 ring atoms and more preferably selected from the group consisting of a substituted or unsubstituted alkyl group having 1 to 30 carbon atoms, a substituted or unsubstituted aryl group having 6 to 30 ring carbon atoms, and a substituted or unsubstituted heteroaryl group having 5 to 30 ring atoms.

The details of the substituents represented by R²¹ to R²⁷ are the same as the details of the corresponding substituents mentioned above with respect to R¹ to R⁶ and R⁷ to R⁹ not the single bond bonded to *a.

In an embodiment of the invention, adjacent two selected from R²¹ to R²⁷ are each independently a hydrogen atom or the substituent without forming a ring structure. In another embodiment of the invention, at least one set of adjacent two are bonded to each other to form at least one ring structure.

The details of the ring structure that is formed by adjacent two selected from R²¹ to R²⁷ and two ring carbon atoms to which the adjacent two are bonded are the same as the details of the ring structure mentioned above with respect to R¹ to R⁶ and R⁷ to R⁹ not the single bond bonded to *a.

In an embodiment of the invention, R²¹ to R²⁷ are preferably all hydrogen atoms.

Ar is a substituted or unsubstituted aryl group having 6 to 30, preferably 6 to 25, more preferably 6 to 18 ring carbon atoms. The aryl group having 6 to 30 ring carbon atoms of the substituted or unsubstituted aryl group having 6 to 30 ring carbon atoms is, for example, a phenyl group, a biphenylyl group, a terphenylyl group, a biphenylenyl group, a naphthyl group, an anthryl group, a benzanthryl group, a phenanthryl group, a benzophenanthryl group, a phenalenyl group, a picenyl group, a pentaphenyl group, a pyrenyl group, a chrysenyl group, a benzochrysenyl group, a fluorenyl group, a fluoranthenyl group, a perylenyl group, or a triphenylenyl group, preferably a phenyl group, a biphenylyl group, a terphenylyl group, a naphthyl group, a phenanthryl group, or a triphenylenyl group, more preferably a phenyl group, a biphenylyl group, a terphenylyl group, or a naphthyl group, and still more preferably a phenyl group, a biphenylyl group, or a terphenyl group.

The biphenylyl group is a 2-, 3- or 4-biphenylyl group, and a 4-biphenylyl group is particularly preferred.

The terphenylyl group is a 1,1':4',1"-terphenyl group, a 1,1':3',1"-terphenyl group, or a 1,1':2',1"-terphenyl group, preferably a 1,1':4',1"-terphenyl-2-yl group, a 1,1':4',1"-terphenyl-3-yl group, a 1,1':4',1"-terphenyl-4-yl group, a 1,1':3',1"-terphenyl-2-yl group, a 1,1':3',1"-terphenyl-3-yl group, a 1,1':3',1"-terphenyl-4-yl group, a 1,1':2',1"-terphenyl-2-yl group, a 1,1':2',1"-terphenyl-3-yl group, or a 1,1':2',1"-terphenyl-4-yl group, and more preferably a 1,1':4',1"-terphenyl-2-yl group, a 1,1':4',1"-terphenyl-3-yl group, or a 1,1':4',1"-terphenyl-4-yl group.

The naphthyl group is a 1-naphthyl group or a 2-naphthyl group.

The phenanthryl group is a 1-, 2-, 3-, 4-, or 9-phenanthryl group and preferably a 2- or 9-phenanthryl group.

The triphenylenyl group is preferably a 2-triphenylenyl group.

The substituted aryl group having 6 to 30 ring carbon atoms is, for example, a 9,9-dimethylfluorenyl group, a 9,9-diphenylfluorenyl group, or a spirobifluorenyl group.

The substituted or unsubstituted aryl group having 6 to 30 ring carbon atoms includes isomeric groups, if present.

L¹ is a single bond or L¹¹. L¹¹ is a substituted or unsubstituted arylene group having 6 to 30, preferably 6 to 25, more preferably 6 to 18 ring carbon atoms. In an embodiment of the invention, L¹ is preferably a single bond. In another embodiment of the invention, L¹ is preferably L¹¹.

The arylene group having 6 to 30 ring carbon atoms of the substituted or unsubstituted arylene group having 6 to 30 ring carbon atoms represented by L¹¹ is, for example, a phenylene group, a biphenylene group, a terphenylene group, a biphenylenylene group, a naphthylene group, an anthrylene group, a benzanthrylene group, a phenanthrylene group, a benzophenanthrylene group, a phenalenylene group, a picenylene group, a pentaphenylene group, a pyrenylene group, a chrysenylene group, a benzochrysenylene group, a fluorenylene group, a fluoranthenylene group, a perylenylene group, or a triphenylenylene group, preferably a phenylene group, a biphenylene group, or a naphthylene group, more preferably a phenylene group, still more preferably a 1,4-phenylene group, a 1,3-phenylene group, or a 1,2-phenylene group, and particularly preferably a 1,4-phenylene group.

The substituted arylene group having 6 to 30 ring carbon atoms is, for example, a 9,9-dimethylfluorenylene group, a 9,9-diphenylfluorenylene group, or a spirobifluorenylene group.

The substituted or unsubstituted arylene group having 6 to 30 ring carbon atoms includes isomeric groups, if present.

L² is a single bond or L²². L²² is a substituted or unsubstituted arylene group having 6 to 30, preferably 6 to 25, more preferably 6 to 18 ring carbon atoms. In an embodiment of the invention, L² is preferably a single bond. In another embodiment of the invention, L² is preferably L²².

The arylene group having 6 to 30 ring carbon atoms of the substituted or unsubstituted arylene group having 6 to 30 ring carbon atoms represented by L²² is, for example, a phenylene group, a biphenylene group, a terphenylene group, a biphenylenylene group, a naphthylene group, an anthrylene group, a benzanthrylene group, a phenanthrylene group, a benzophenanthrylene group, a phenalenylene group, a picenylene group, a pentaphenylene group, a pyrenylene group, a chrysenylene group, a benzochrysenylene group, a fluorenylene group, a fluoranthenylene group, a perylenylene group, or a triphenylenylene group, preferably a phenylene group, a biphenylene group, a naphthylene group, or a phenanthrylene group, more preferably a phenylene group or a biphenylene group, and still more preferably a phenylene group.

The phenylene group is a 1,2-phenylene group, a 1,3-phenylene group, or a 1,4-phenylene group, with a 1,4-phenylene group being particularly preferred.

The biphenylene group is preferably a 4,2'-biphenylene group, a 4,3'-biphenylene group, or a 4,4'-biphenylene group, with a 4,4'-biphenylene group being particularly preferred. When L²² is a 4,2'-biphenylene group or a 4,3'-biphenylene group, the central nitrogen atom is preferably bonded to 4-position and the 4-dibenzofuranyl structure is preferably bonded to 2'- or 3'-position.

The naphthylene group is preferably a 1,4-naphthylene group or a 2,6-naphthylene group.

The phenanthrylene group is preferably a 2,7-phenanthrylene group.

L³ is a single bond or L³³. L³³ is a substituted or unsubstituted arylene group having 6 to 30, preferably 6 to 25, more preferably 6 to 18 ring carbon atoms. In an embodiment of the invention, L³ is preferably a single bond. In another embodiment of the invention, L³ is L³³.

The arylene group having 6 to 30 ring carbon atoms of the substituted or unsubstituted arylene group having 6 to 30 ring carbon atoms represented by L³³ is, for example, a phenylene group, a biphenylene group, a terphenylene group, a biphenylenylene group, a naphthylene group, an anthrylene group, a benzanthrylene group, a phenanthrylene group, a benzophenanthrylene group, a phenalenylene group, a picenylene group, a pentaphenylene group, a pyrenylene group, a chrysenylene group, a benzochrysenylene group, a fluorenylene group, a fluoranthenylene group, a perylenylene group, or a triphenylenylene group, preferably a phenylene group, and more preferably a 1,4-phenylene group.

The optional substituent referred to by "substituted or unsubstituted" used herein is, unless otherwise noted, selected from the group consisting of a halogen atom; a cyano group; a nitro group; an alkyl group having 1 to 30, preferably 1 to 18, more preferably 1 to 8 carbon atoms; a cycloalkyl group having 3 to 30, preferably 3 to 10, more preferably 3 to 8, and still more preferably 5 or 6 ring carbon atoms; an aryl group having 6 to 30, preferably 6 to 25, more preferably 6 to 18 ring carbon atoms; an aralkyl group having 7 to 36, preferably 7 to 26, more preferably 7 to 20 carbon atoms; an alkoxy group having 1 to 30, preferably 1 to 18, more preferably 1 to 8 carbon atoms; an aryloxy group having 6 to 30, preferably 6 to 25, more preferably 6 to 18 ring carbon atoms; a mono, di or tri-substituted silyl group having a substituent selected from an alkyl group having 1 to 30, preferably 1 to 18, more preferably 1 to 8 carbon atoms and an aryl group having 6 to 30, preferably 6 to 25, more preferably 6 to 18 ring carbon atoms; a haloalkyl group having 1 to 30, preferably 1 to 18, more preferably 1 to 8 carbon atoms; a haloalkoxy group having 1 to 30, preferably 1 to 18, more preferably 1 to 8 carbon atoms; and a heteroaryl group having 5 to 30, preferably 5 to 24, more preferably 5 to 13 ring atoms.

The details of substituents are the same as the details of the corresponding substituents mentioned above with respect to R¹ to R⁶ and R⁷ to R⁹ not the single bond bonded to *a.

Examples of the compound (1) of the invention are shown below, although not limited thereto.

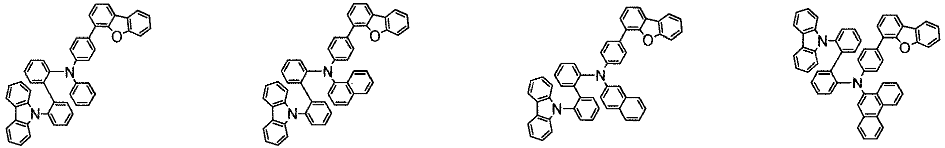

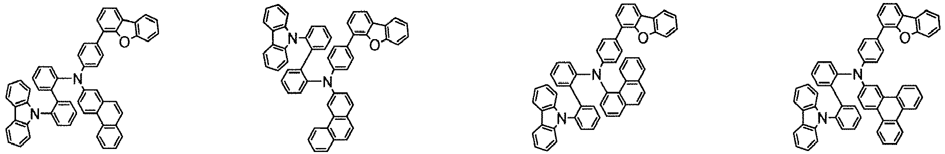

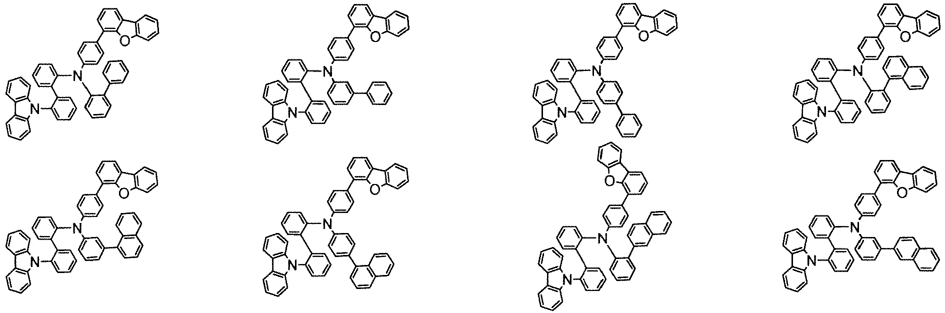

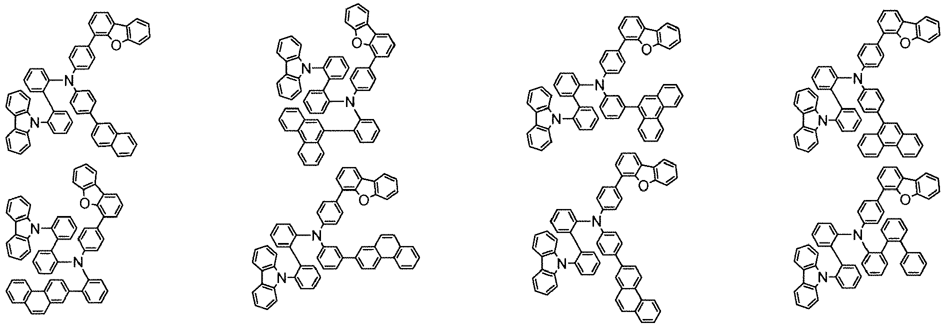

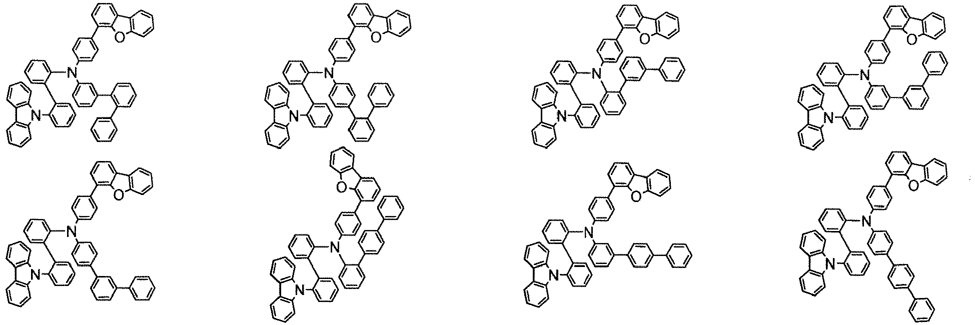

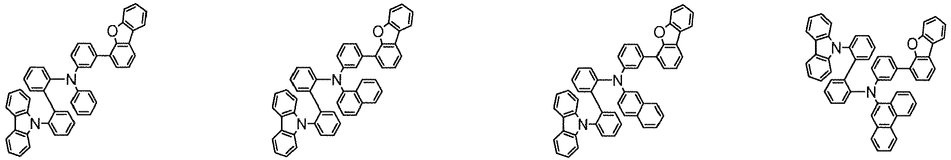

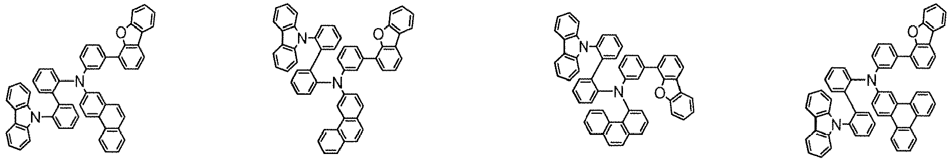

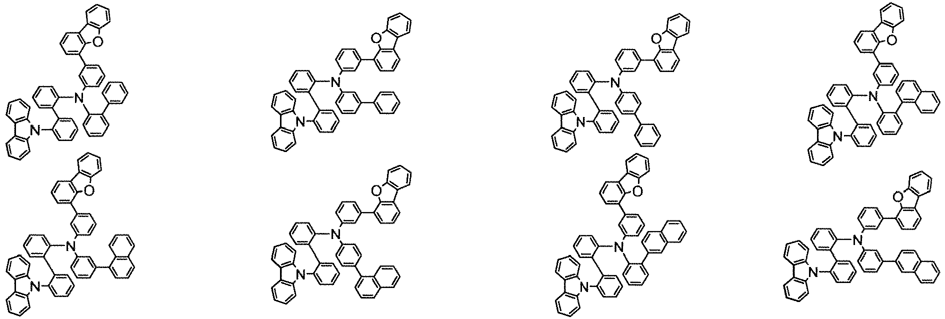

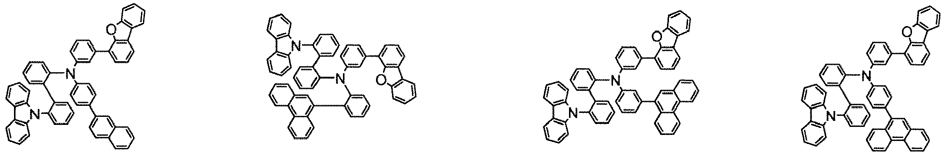

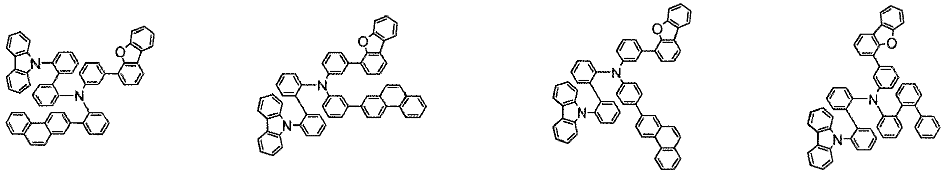

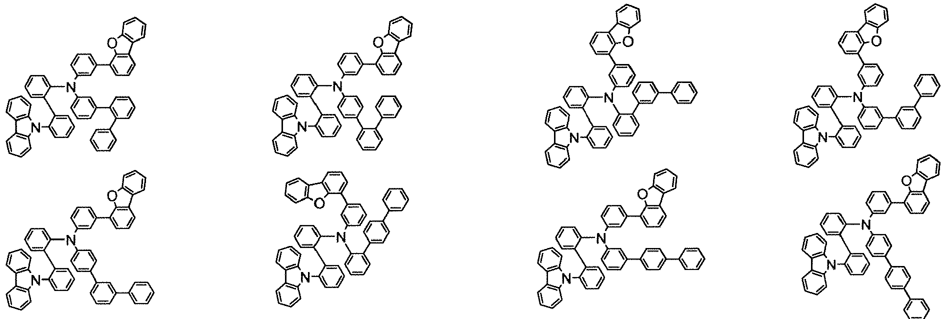

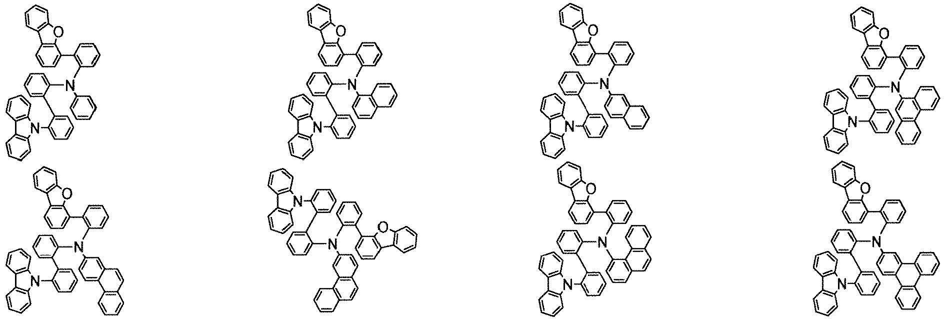

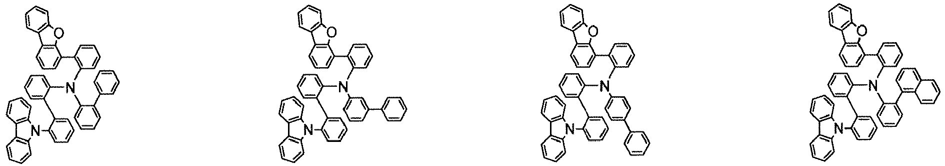

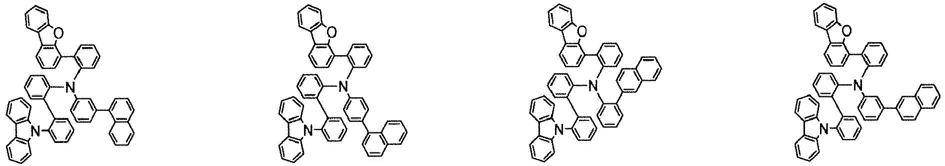

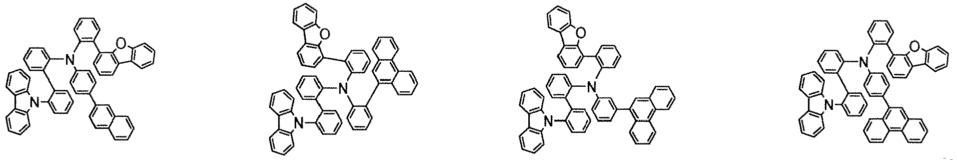

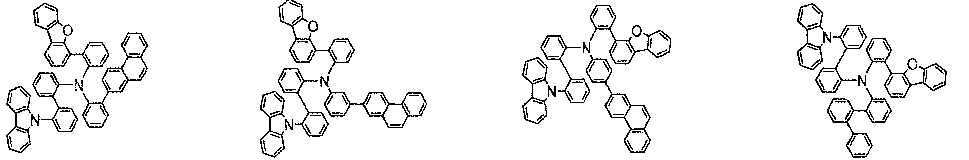

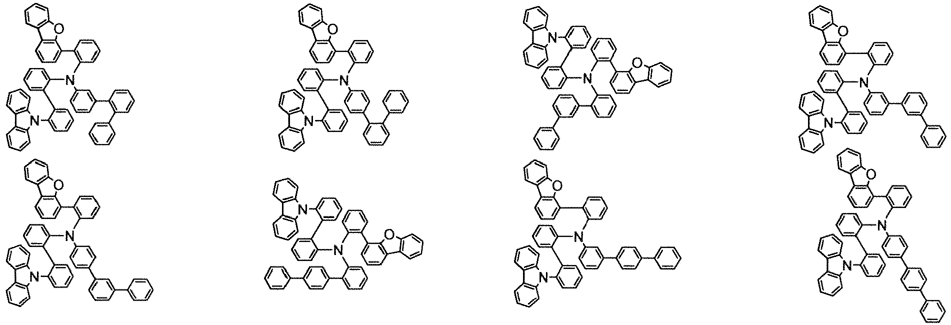

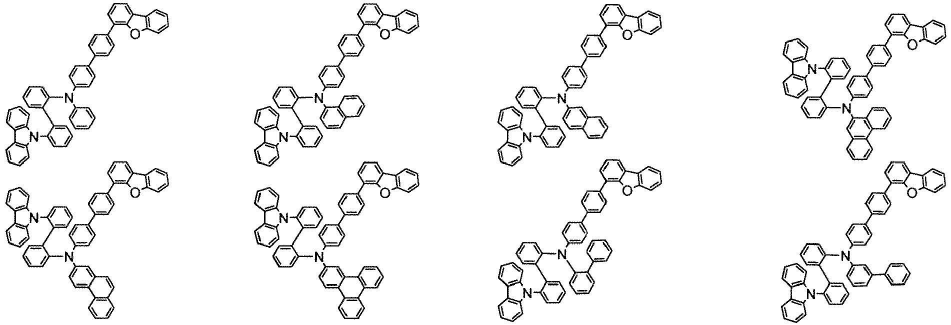

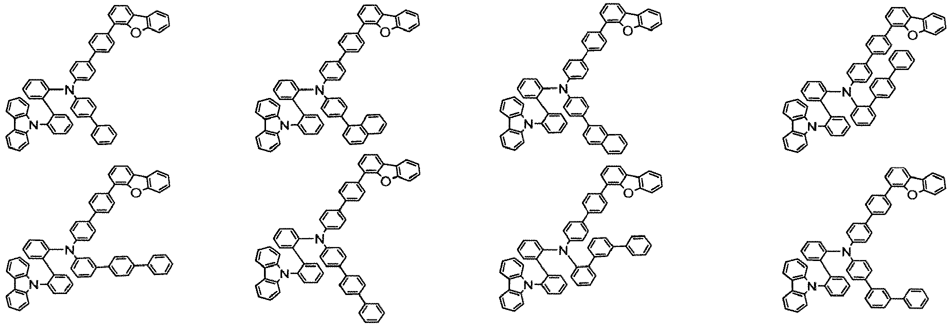

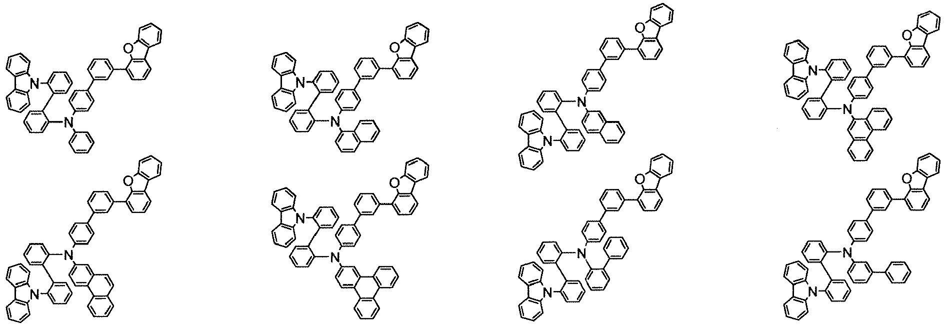

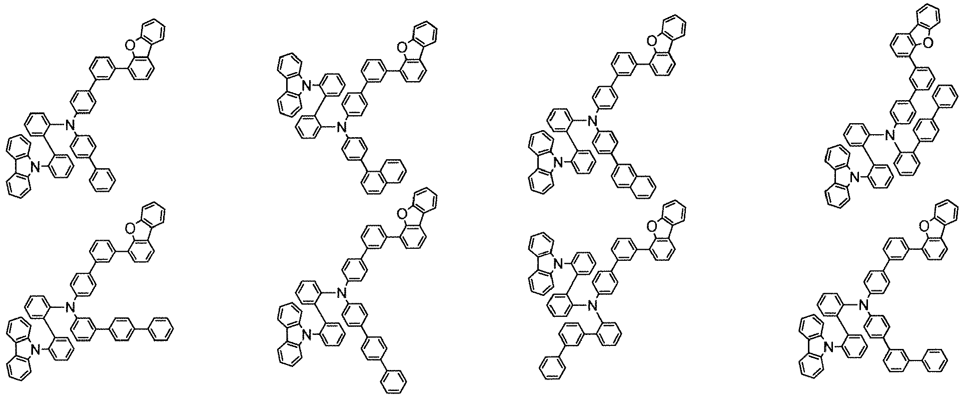

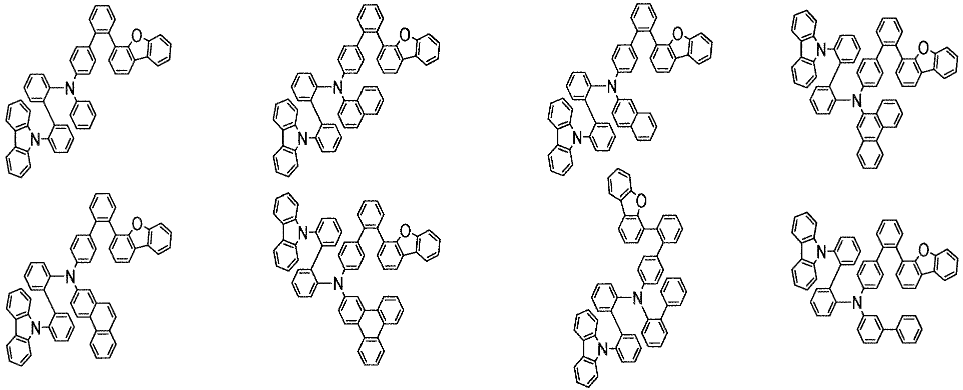

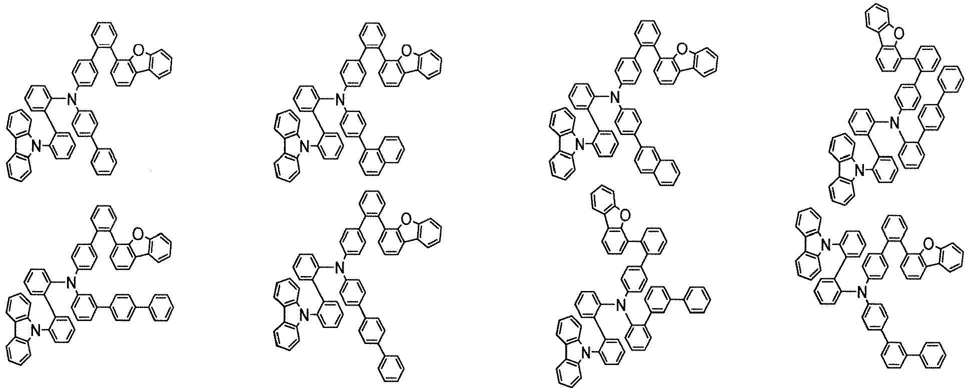

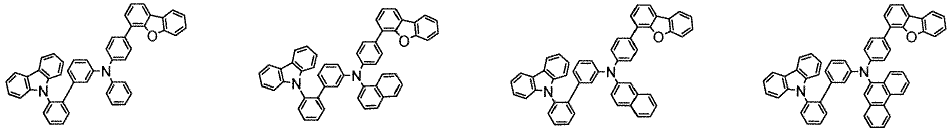

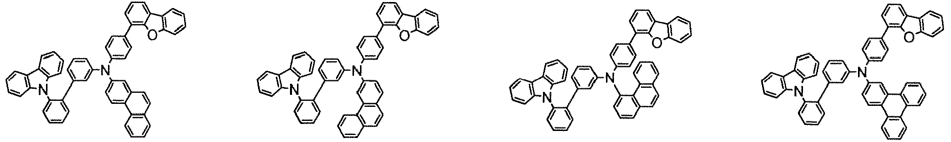

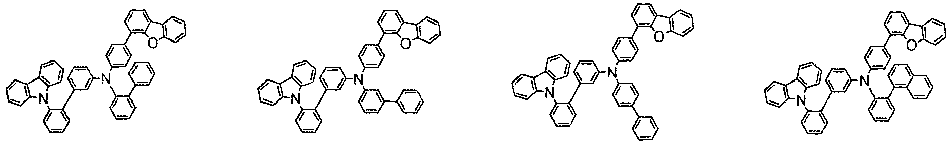

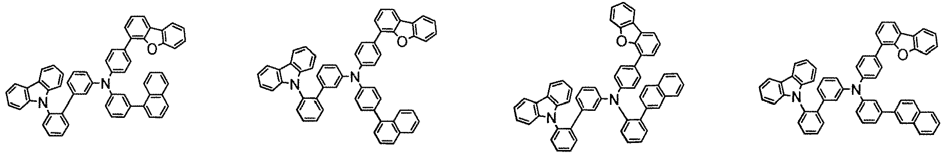

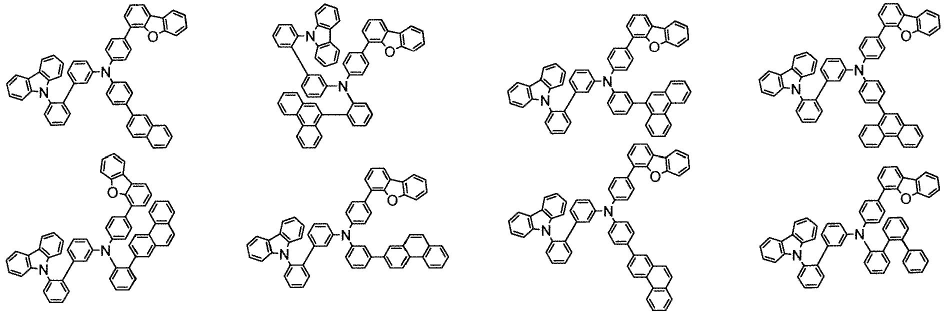

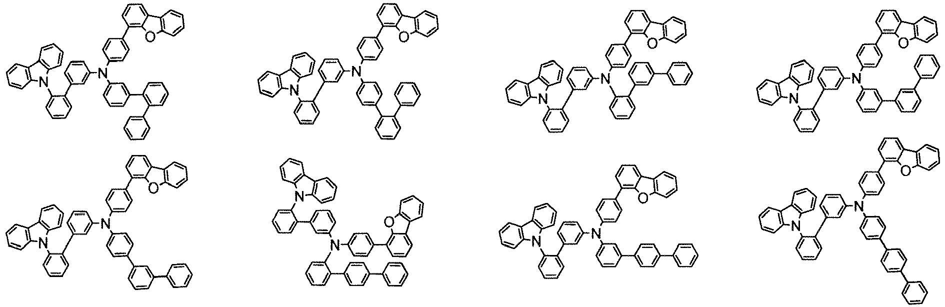

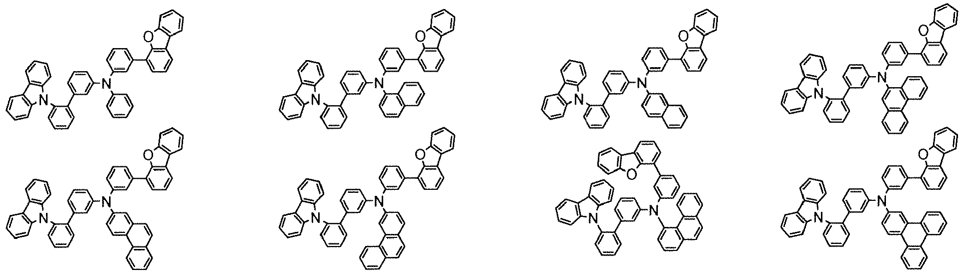

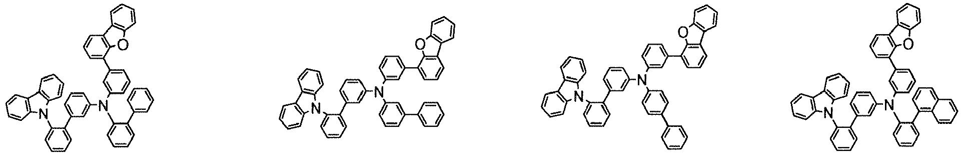

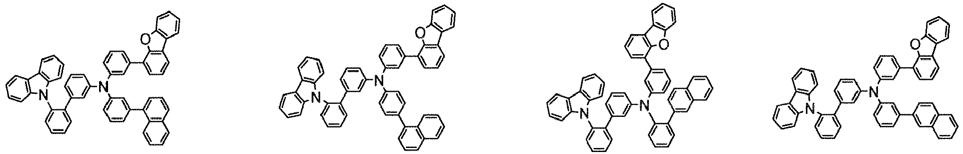

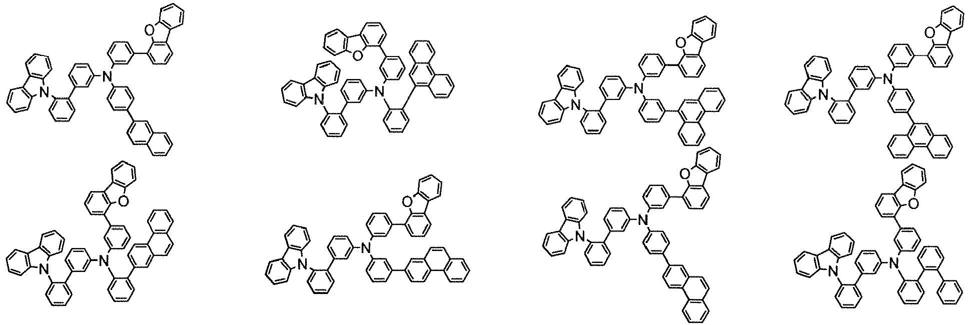

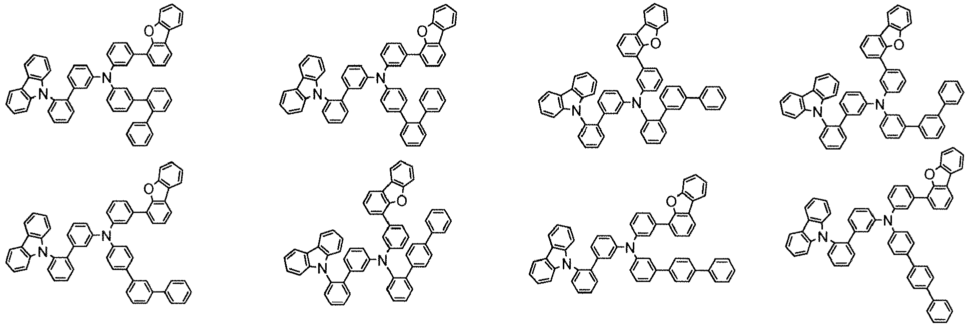

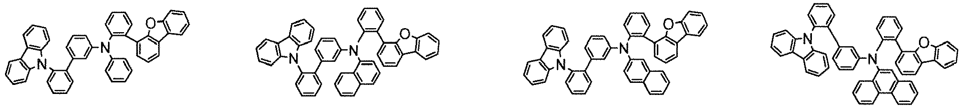

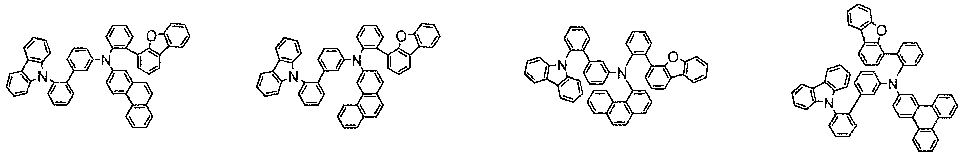

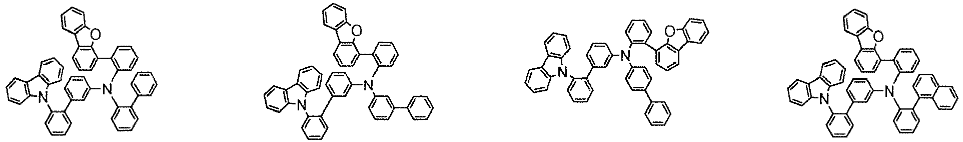

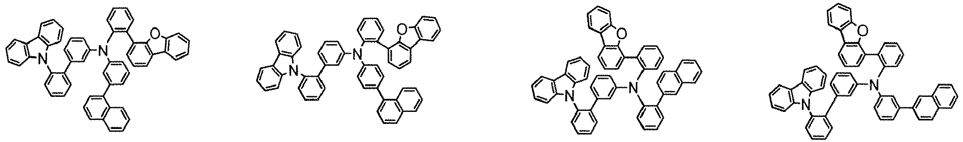

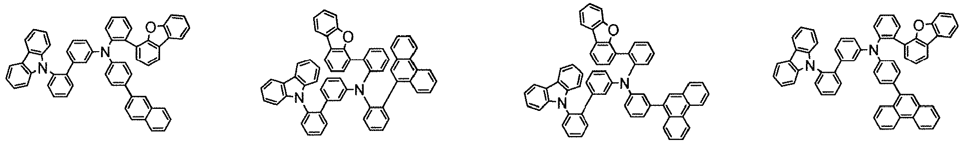

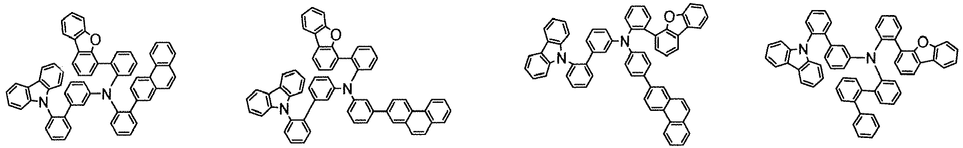

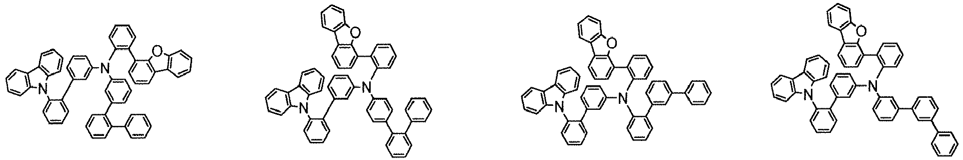

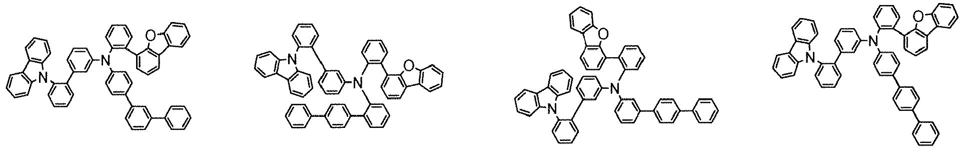

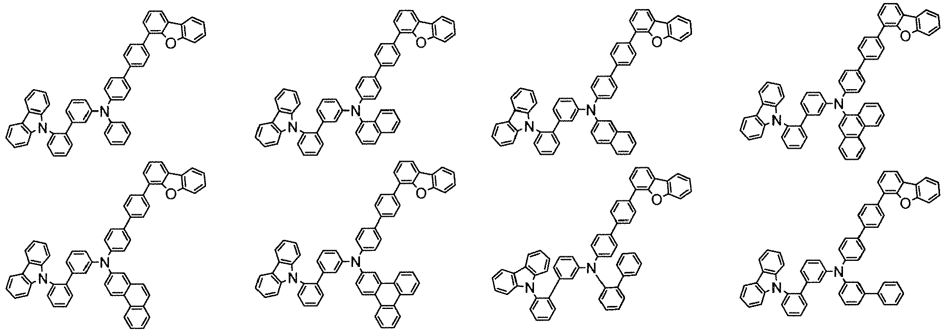

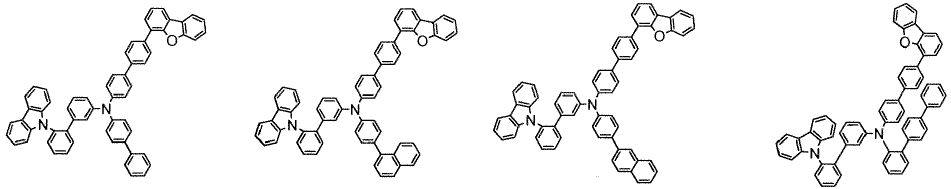

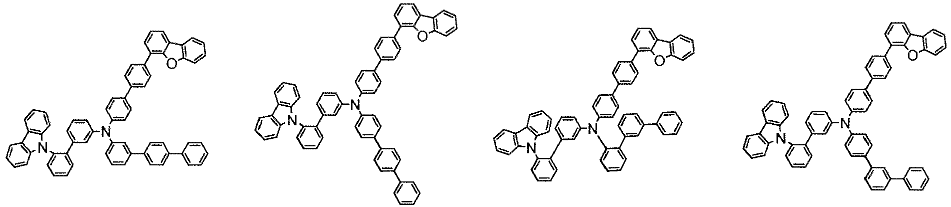

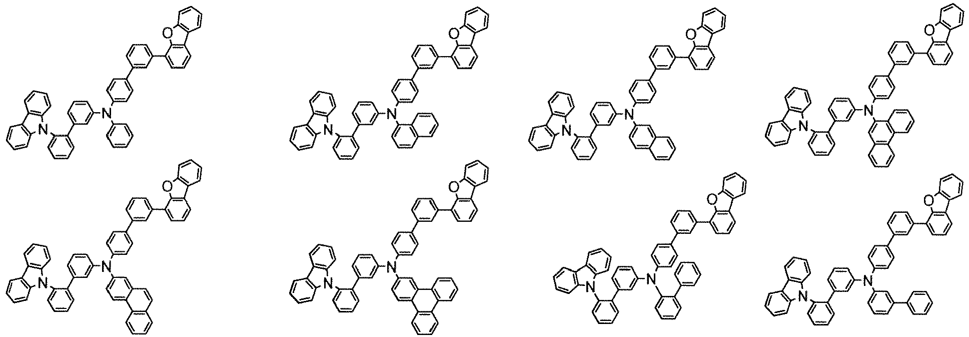

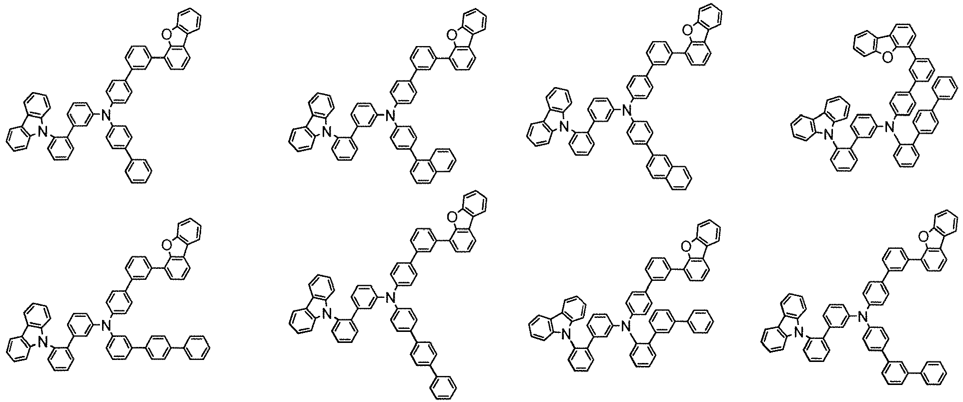

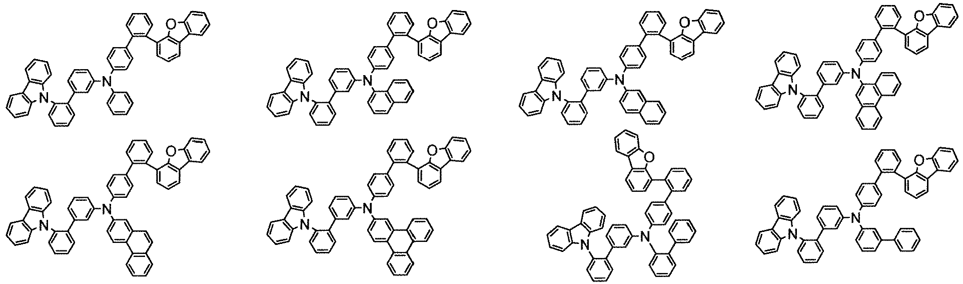

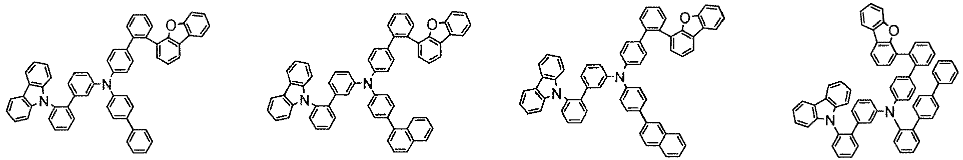

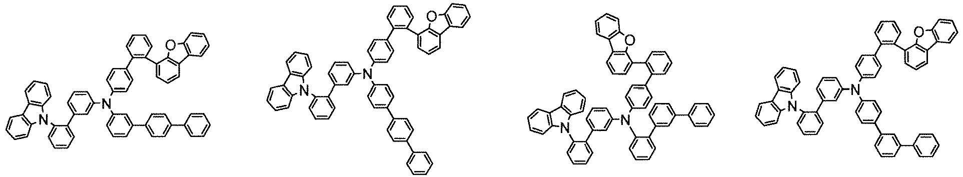

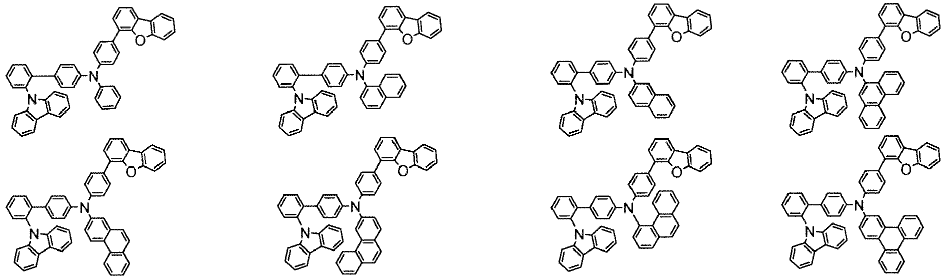

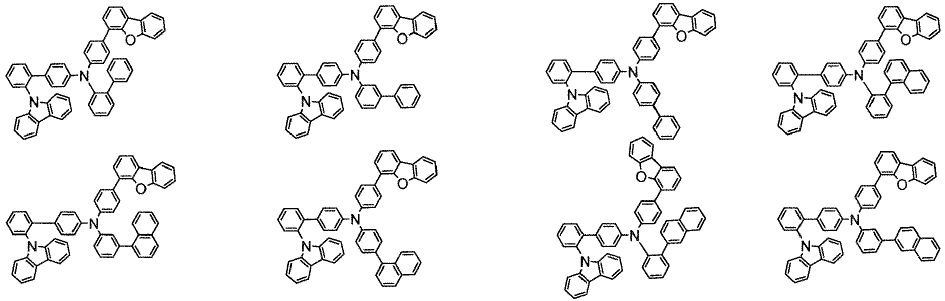

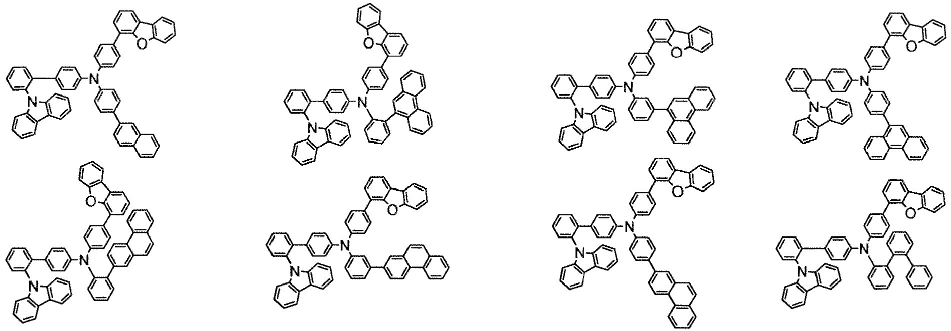

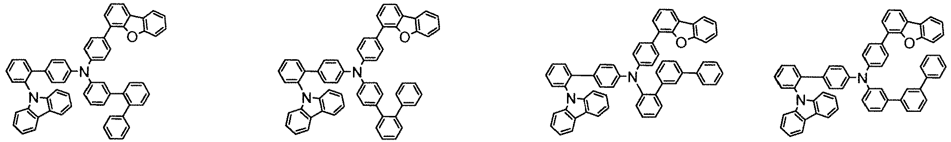

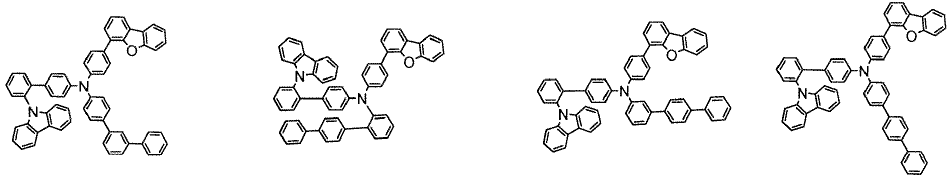

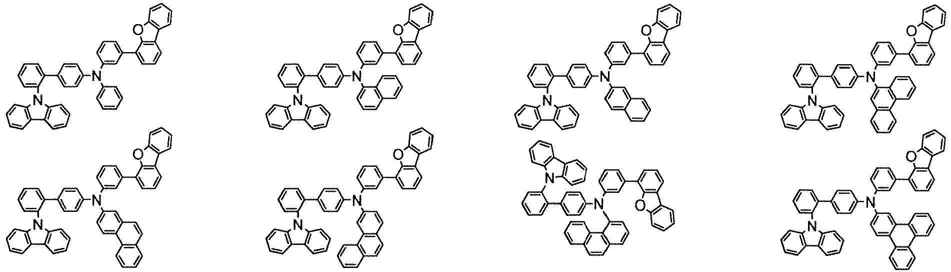

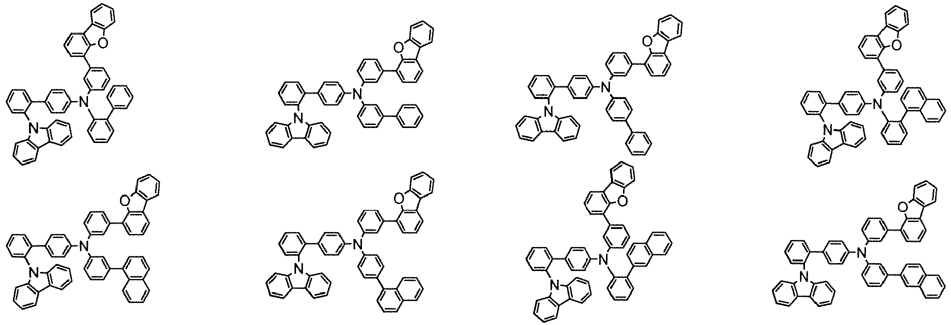

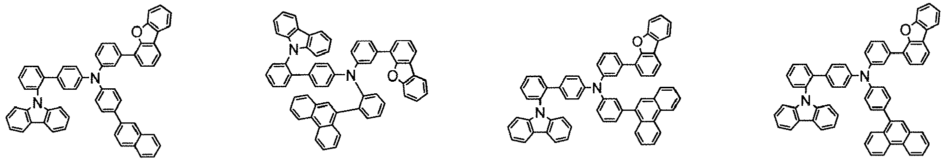

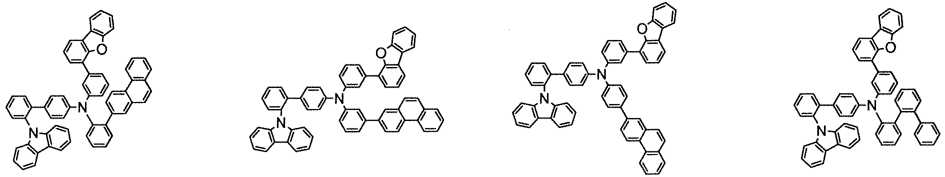

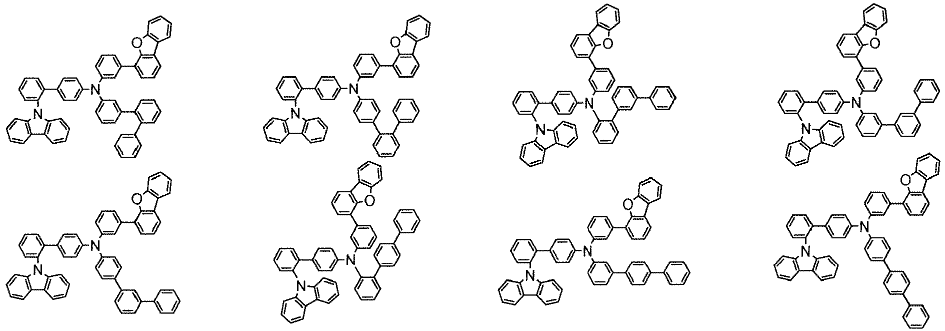

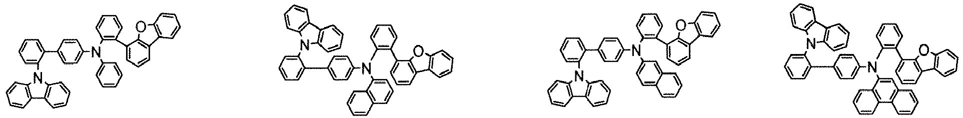

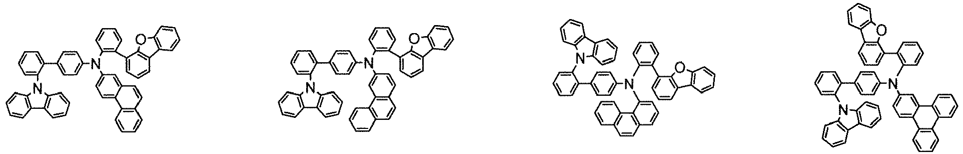

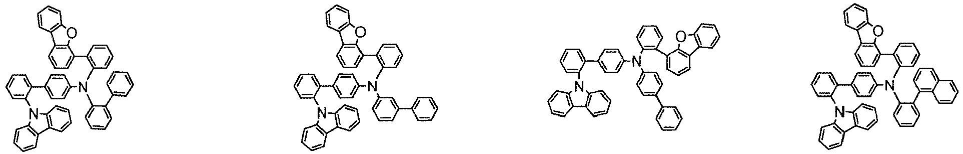

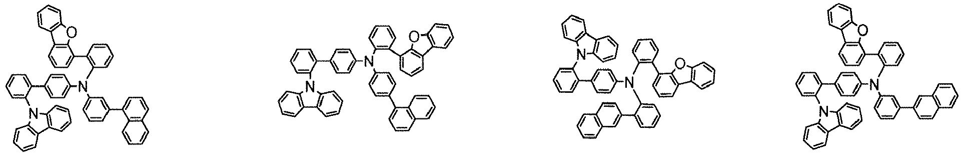

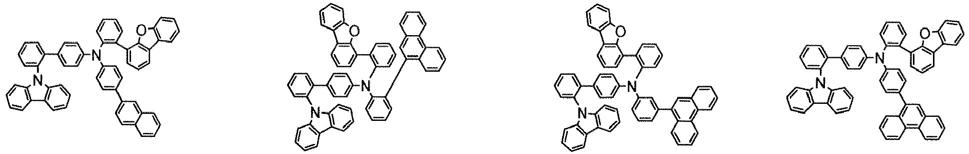

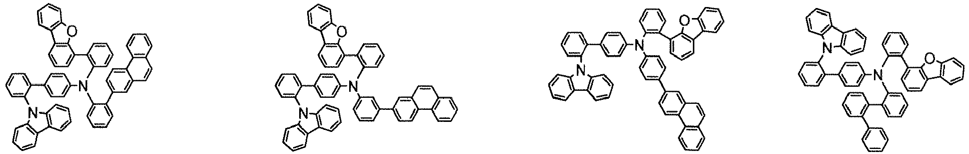

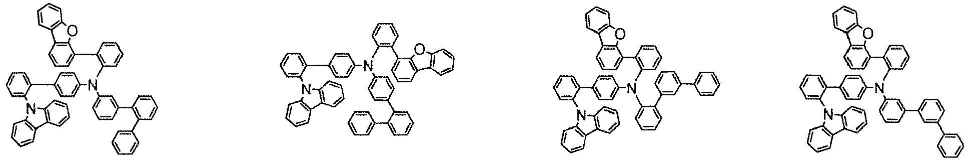

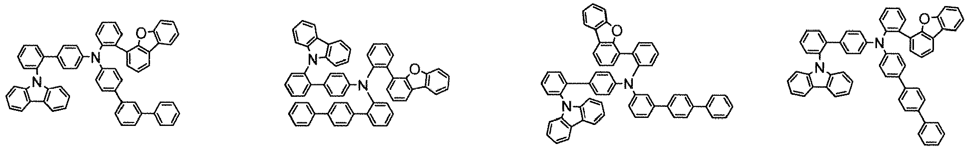

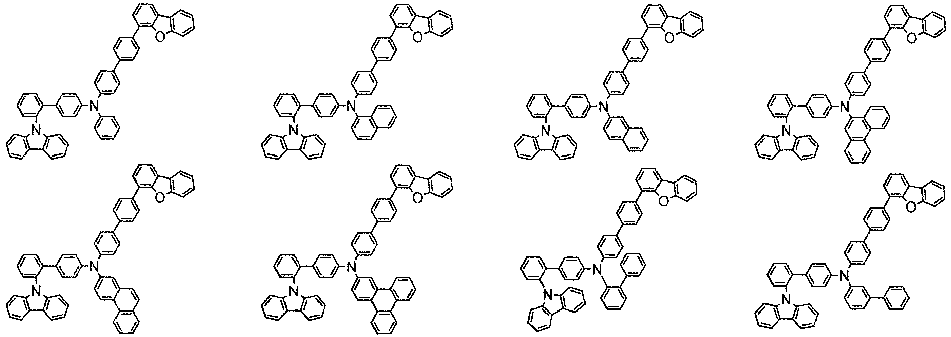

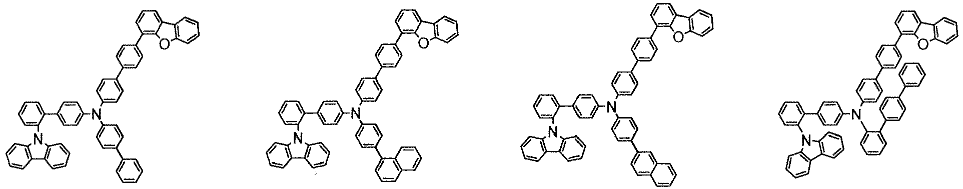

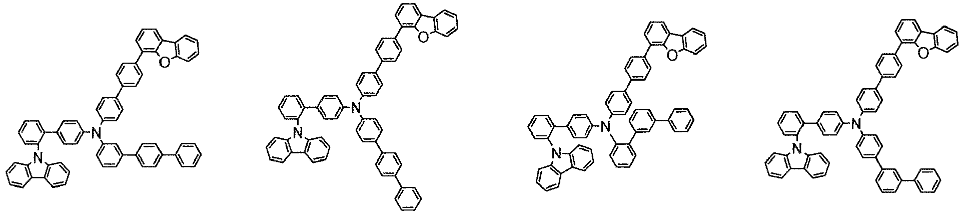

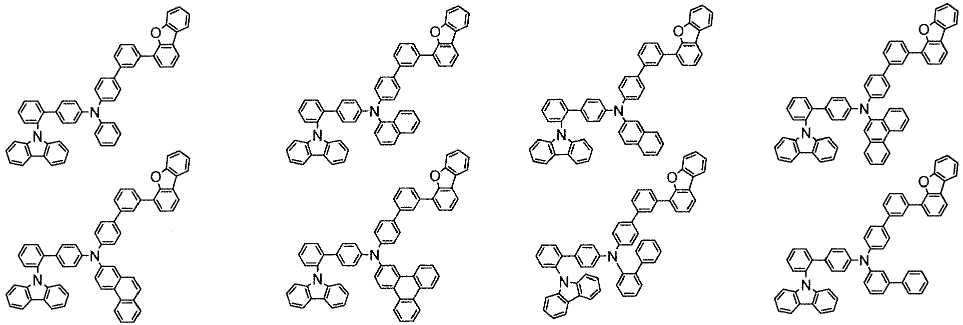

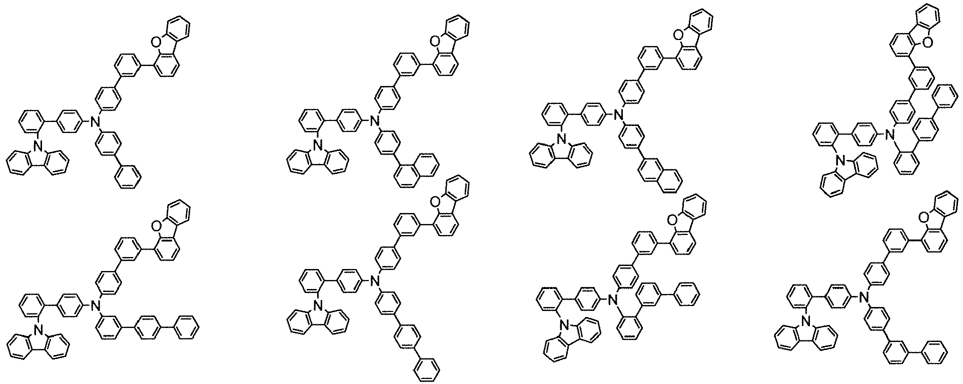

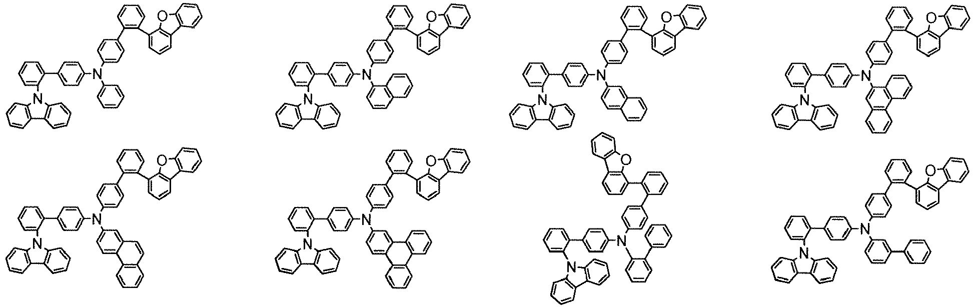

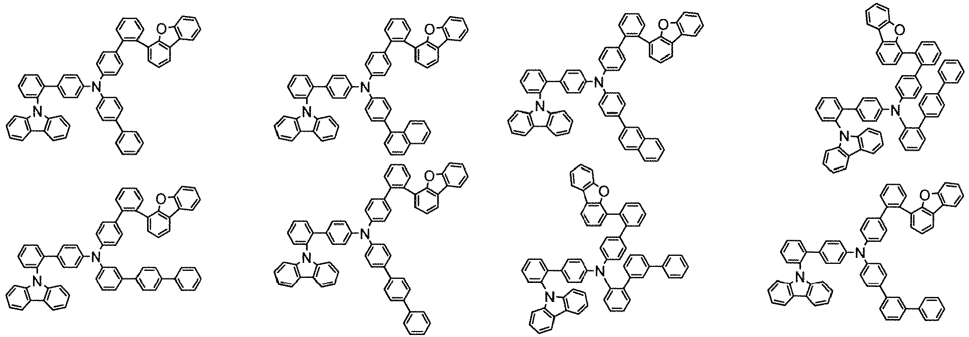

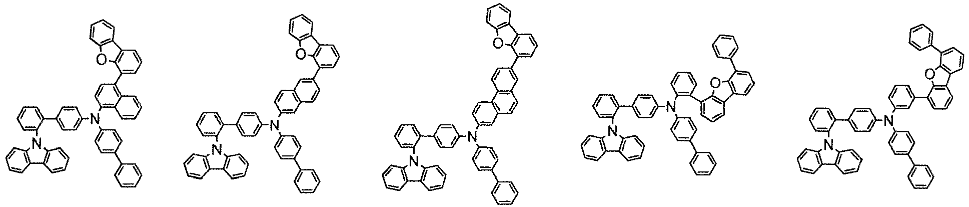

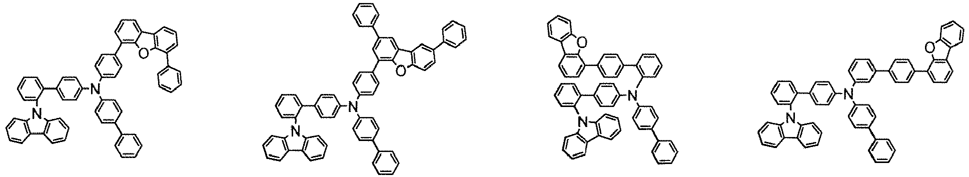

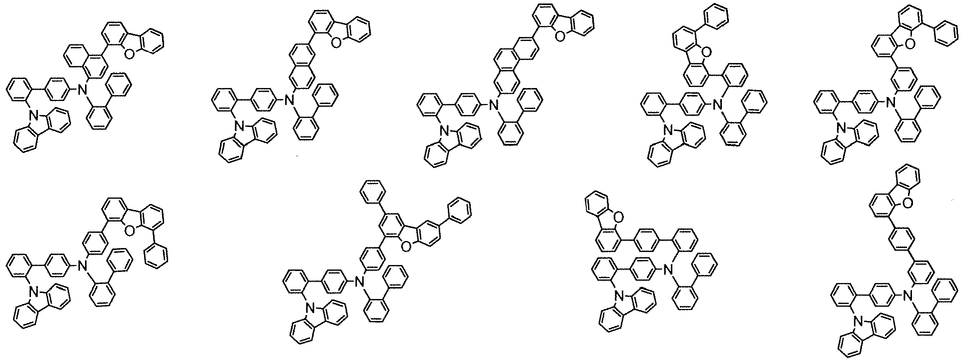

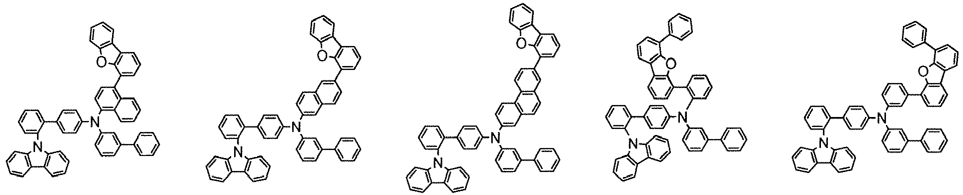

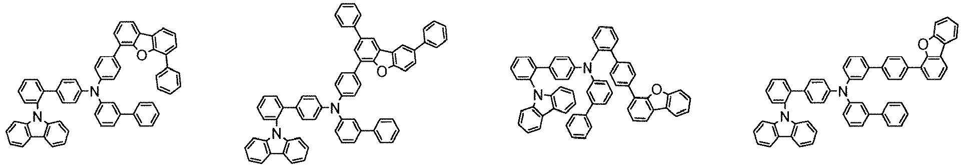

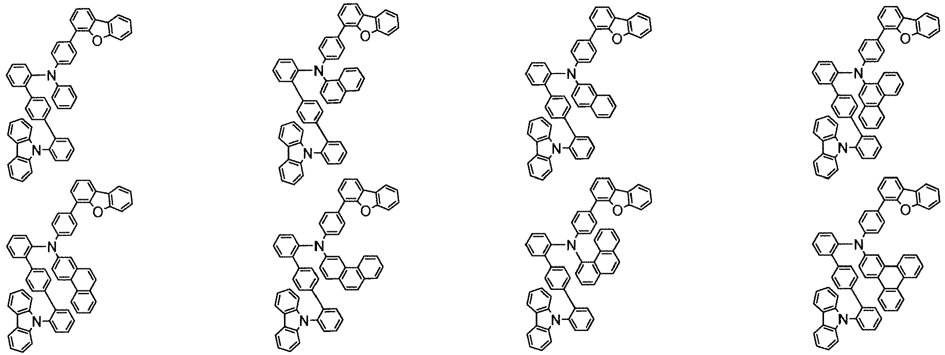

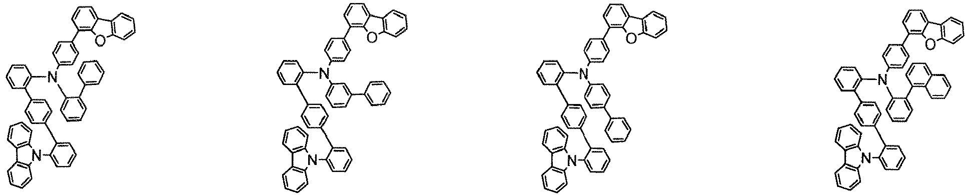

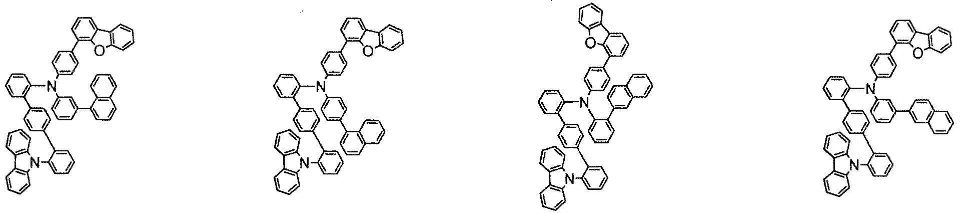

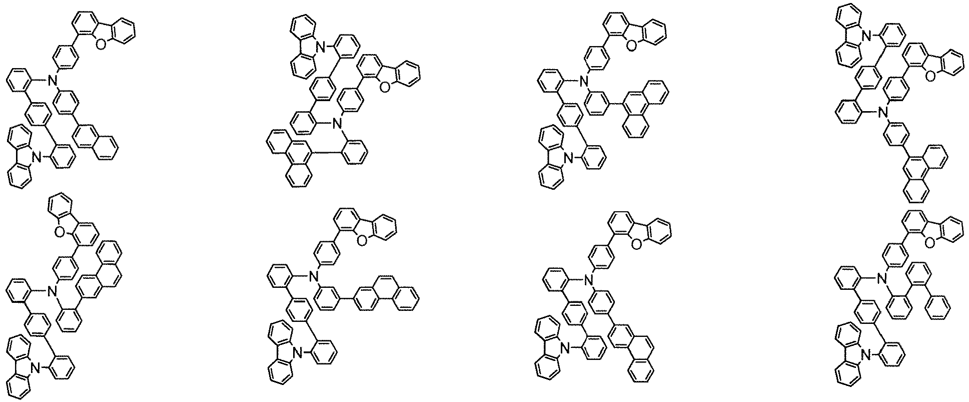

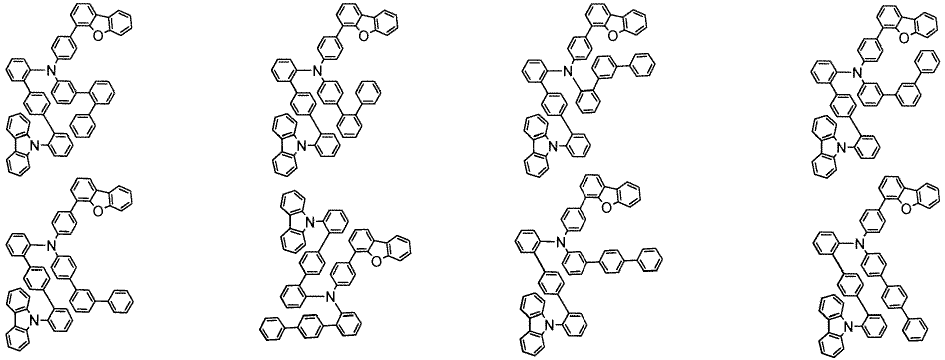

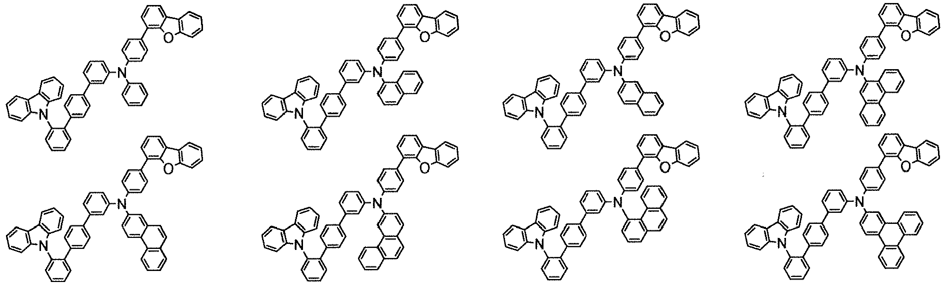

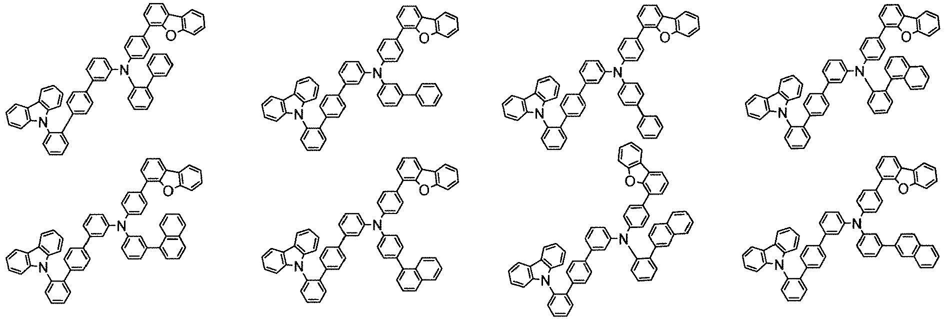

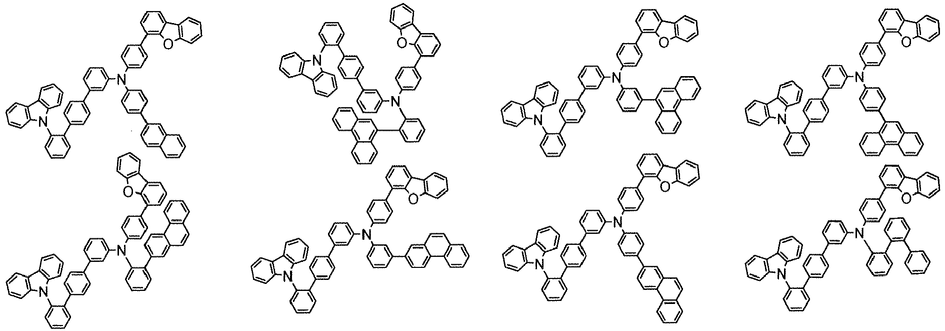

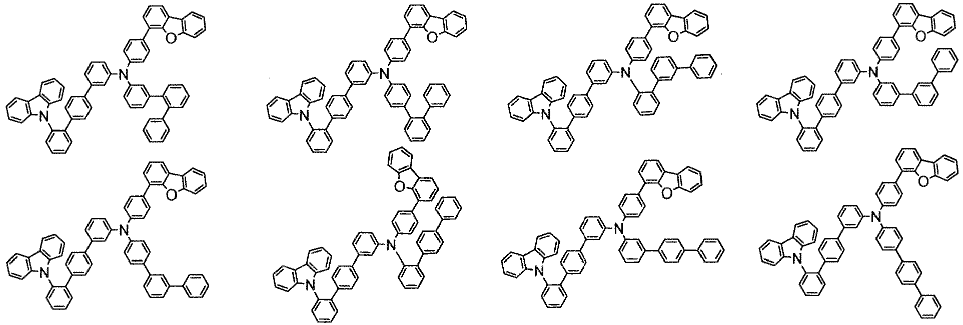

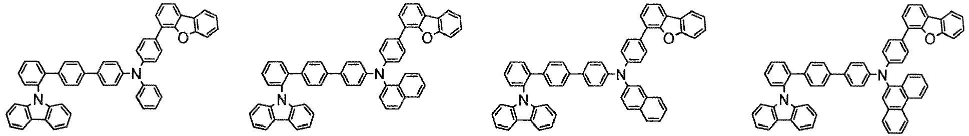

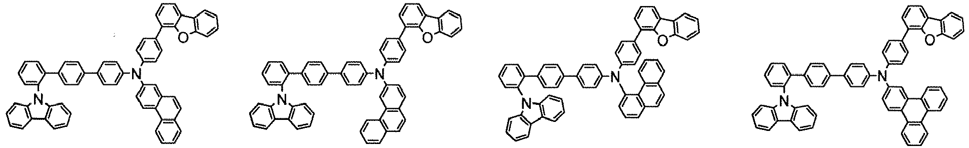

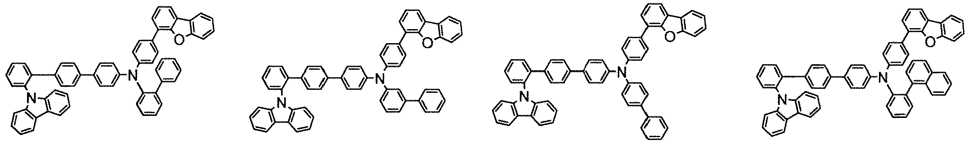

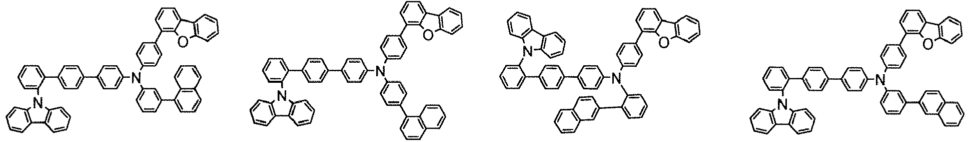

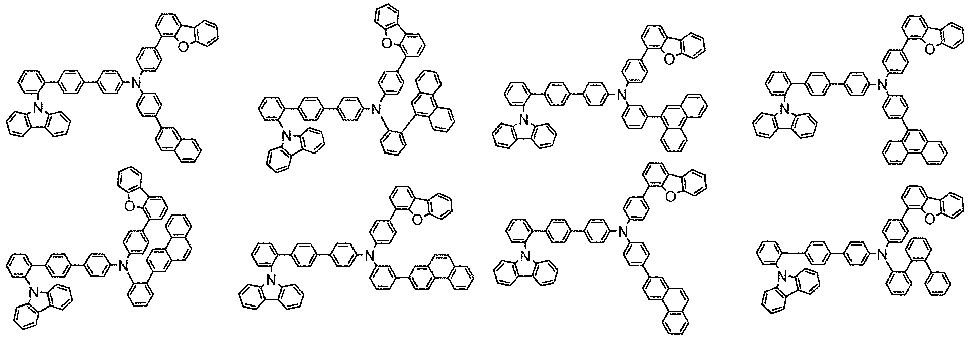

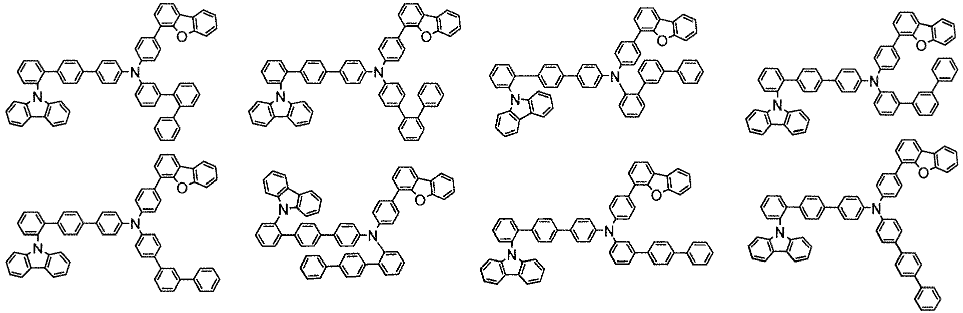

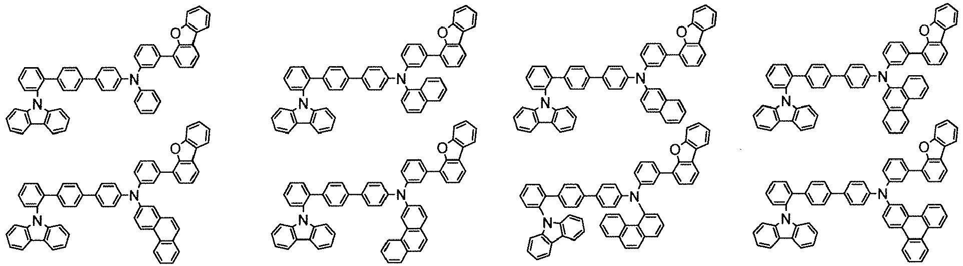

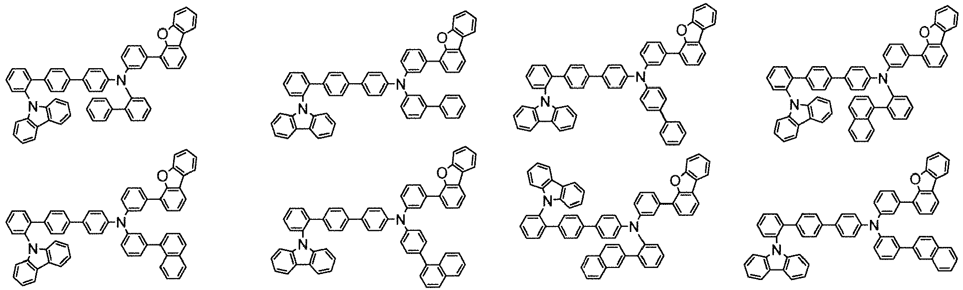

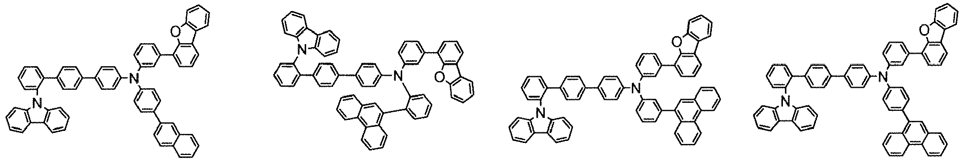

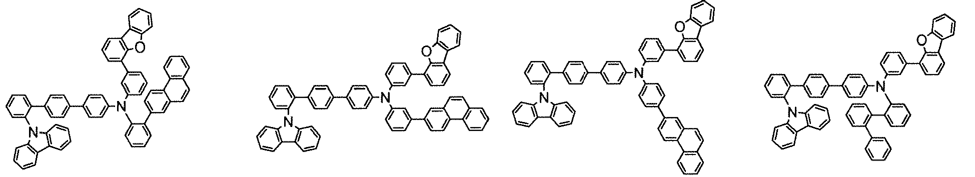

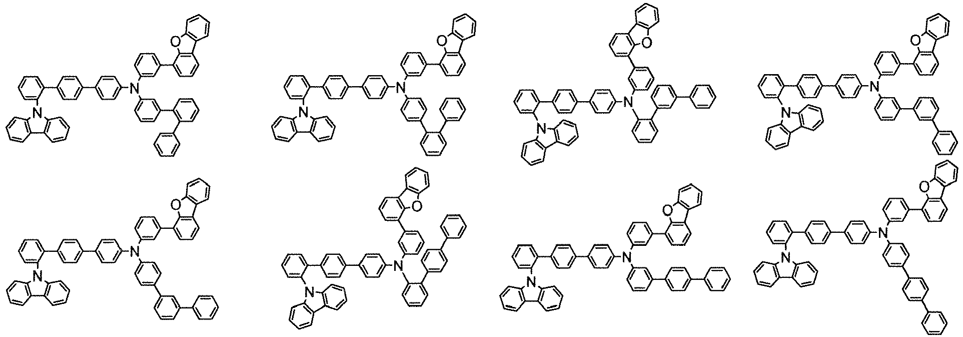

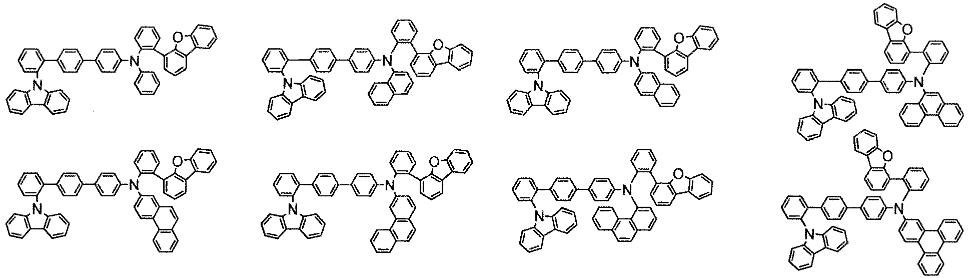

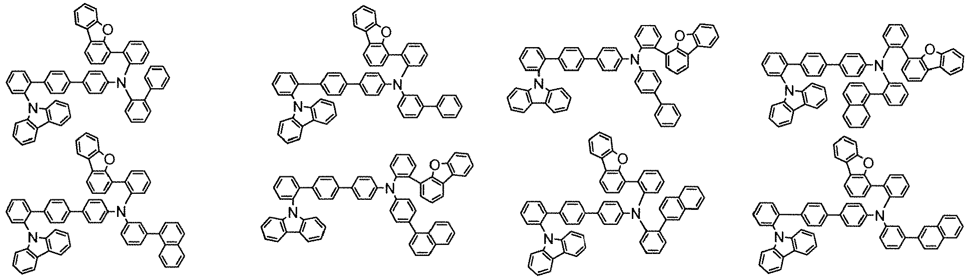

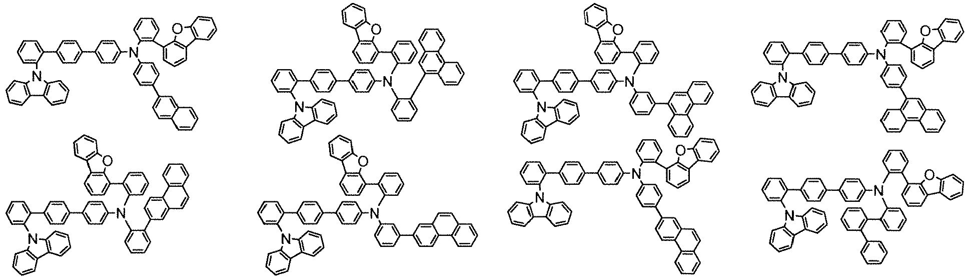

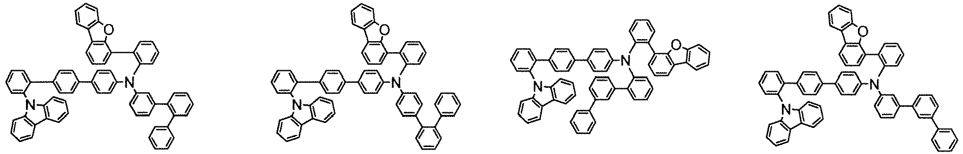

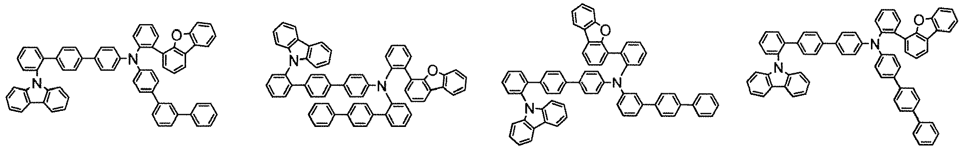

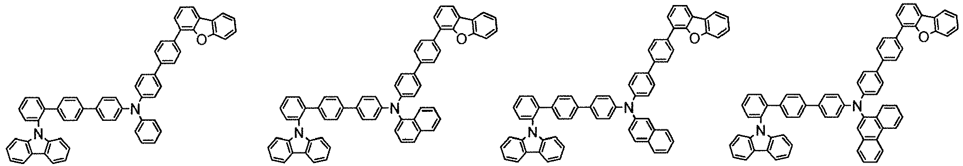

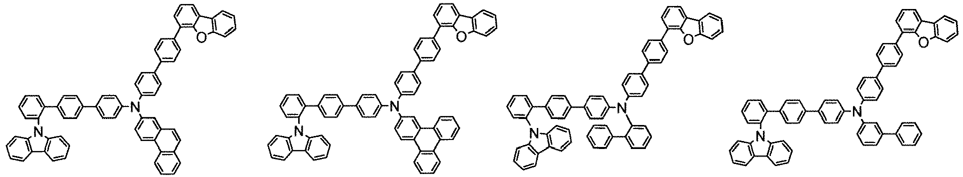

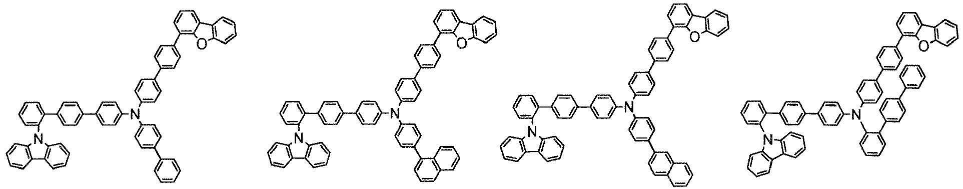

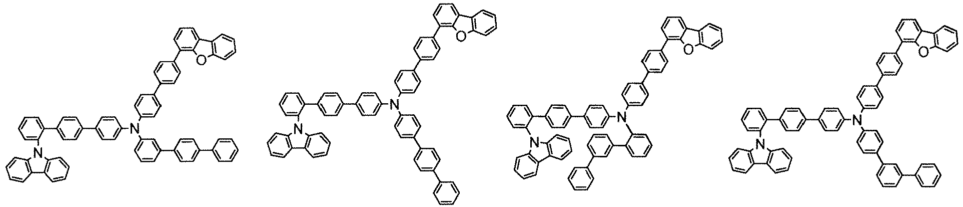

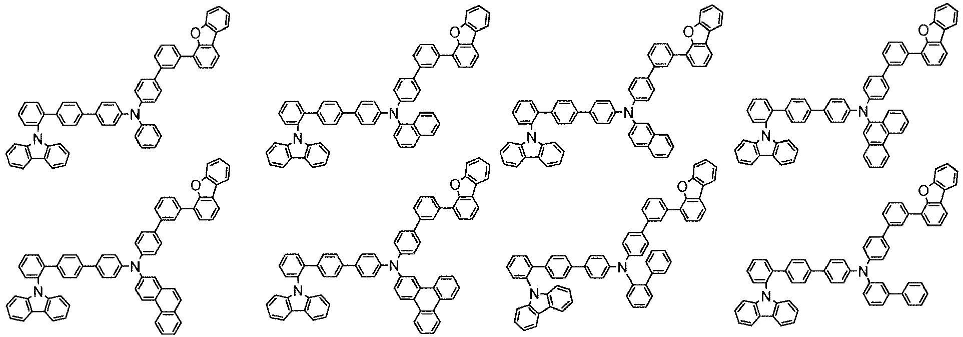

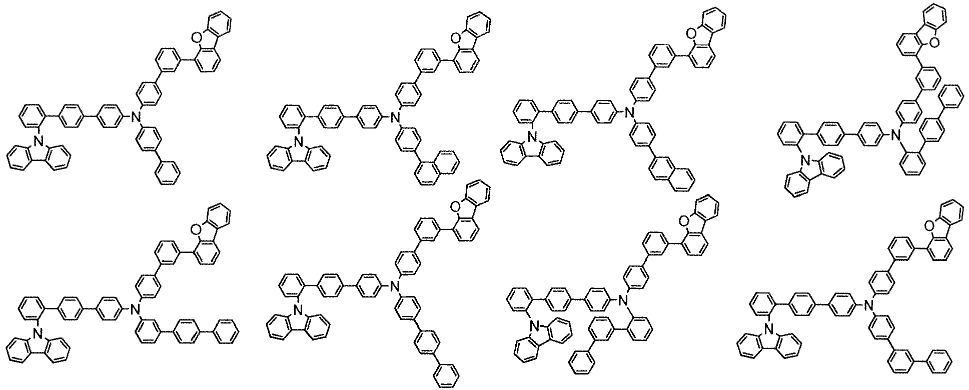

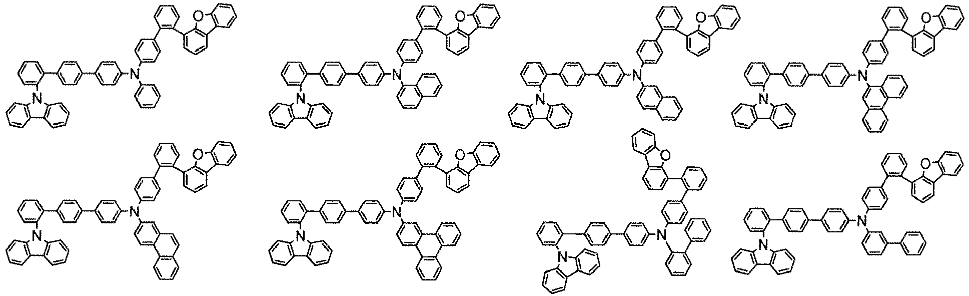

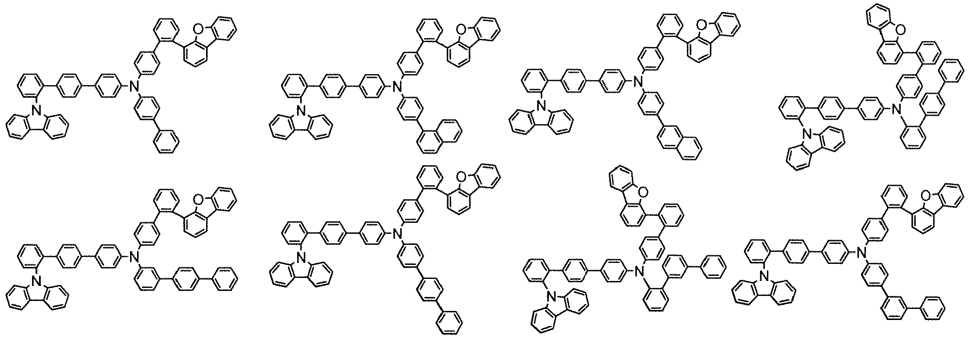

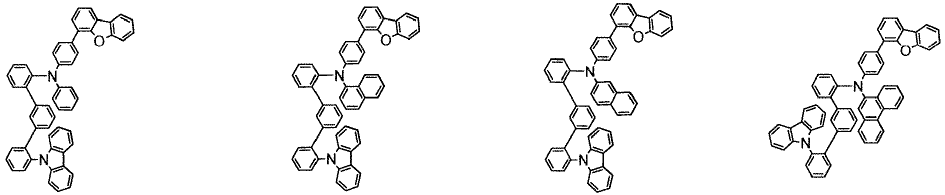

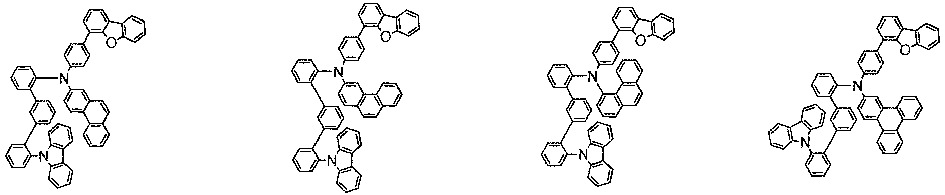

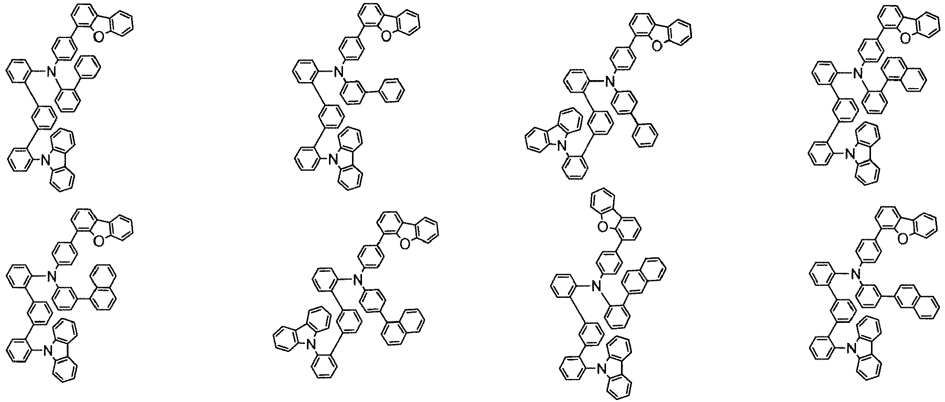

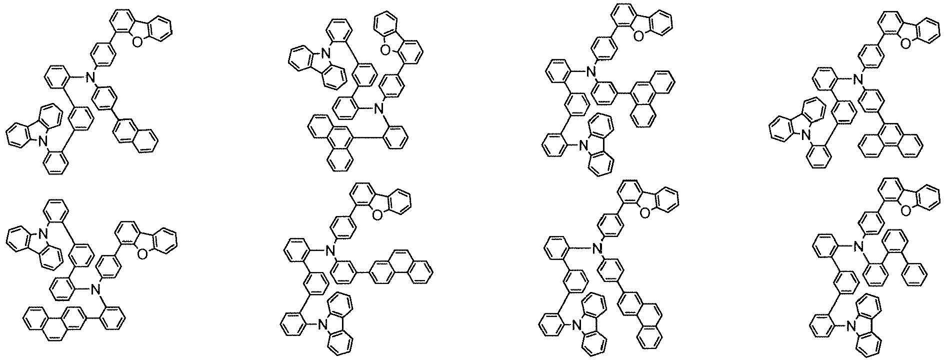

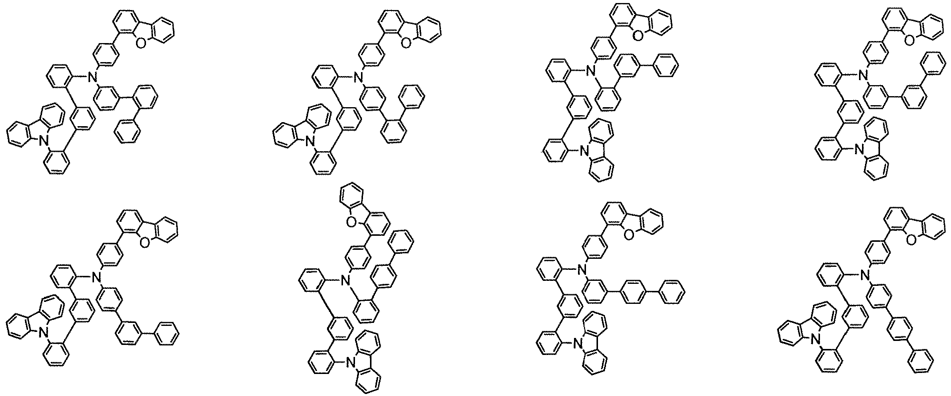

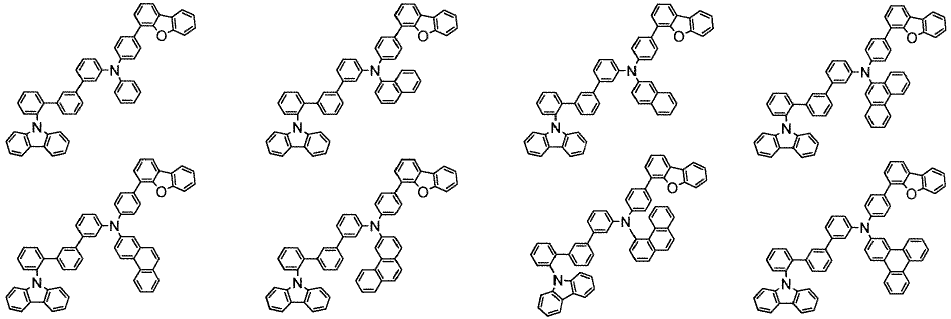

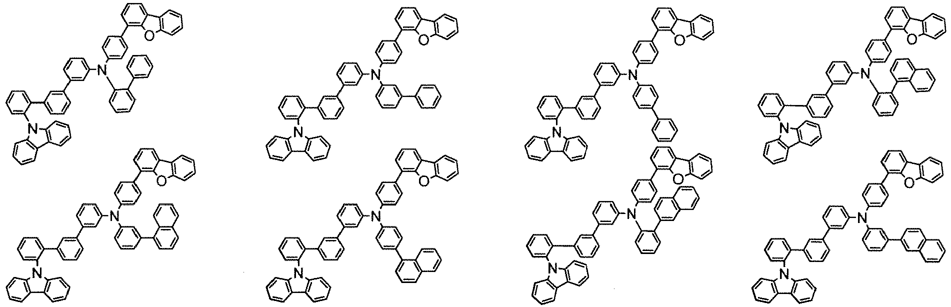

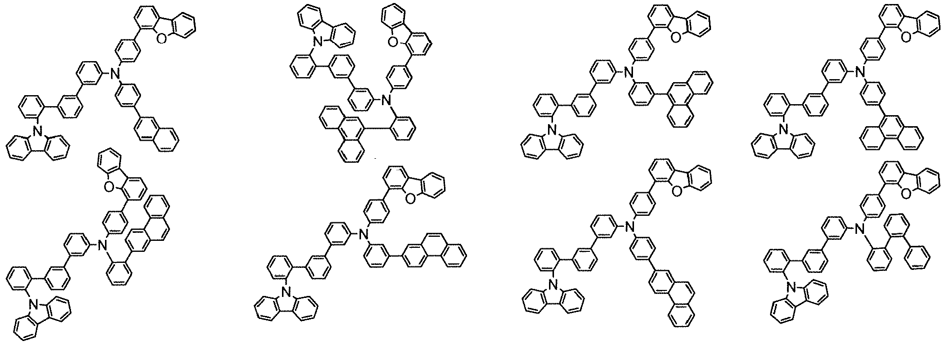

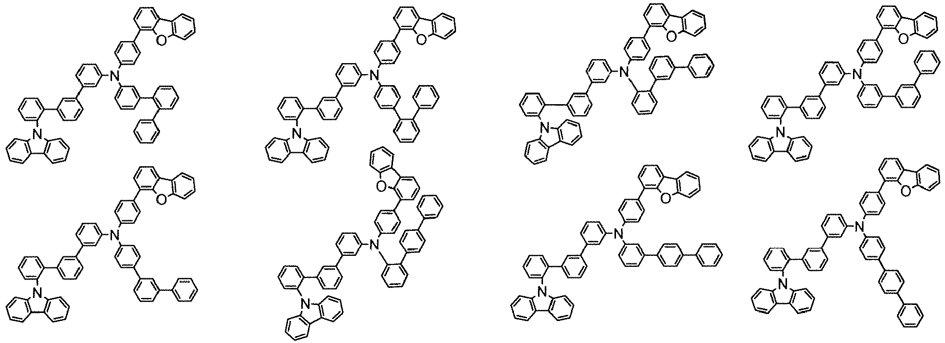

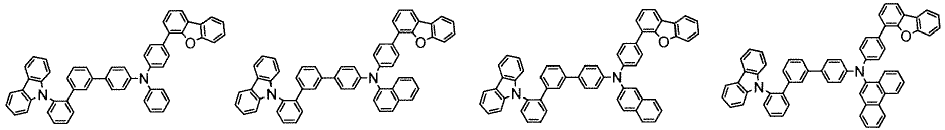

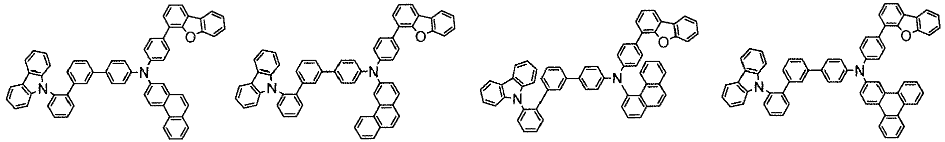

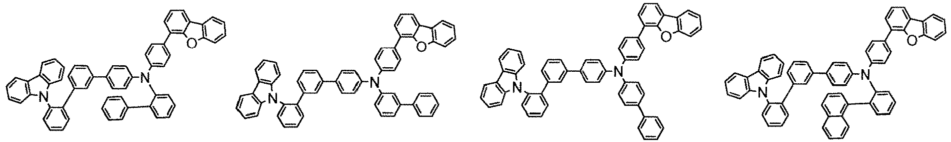

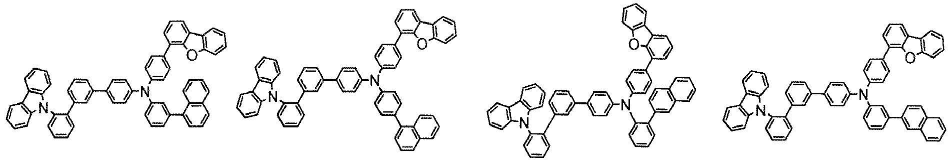

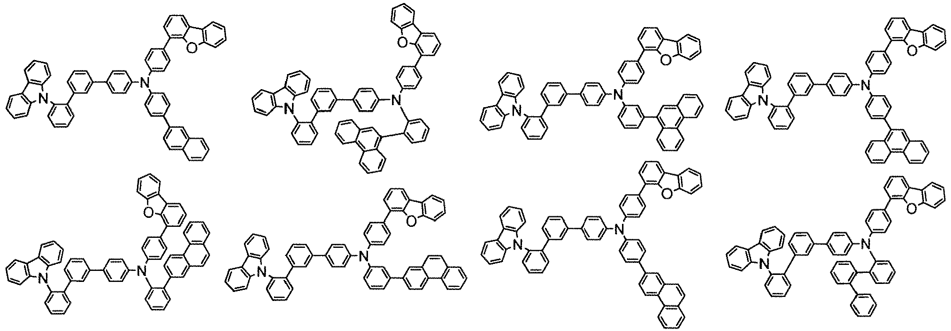

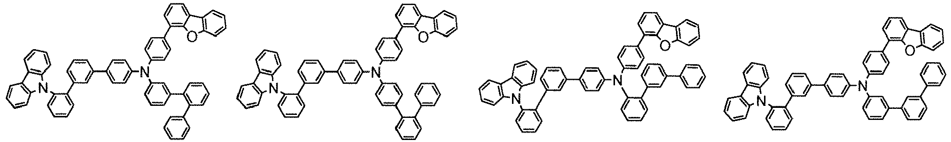

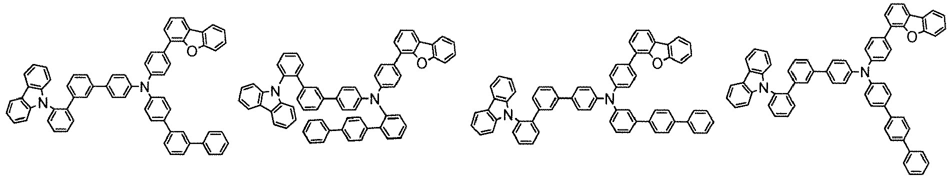

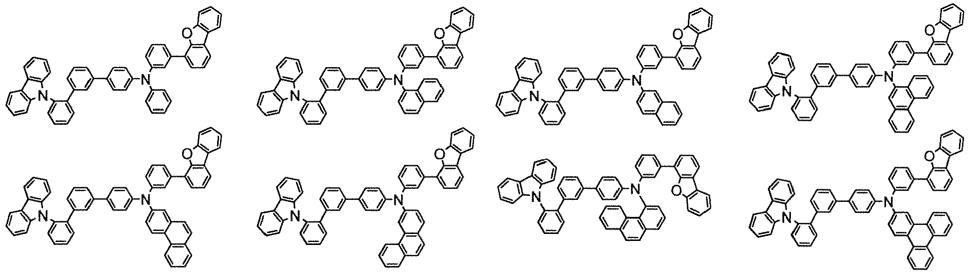

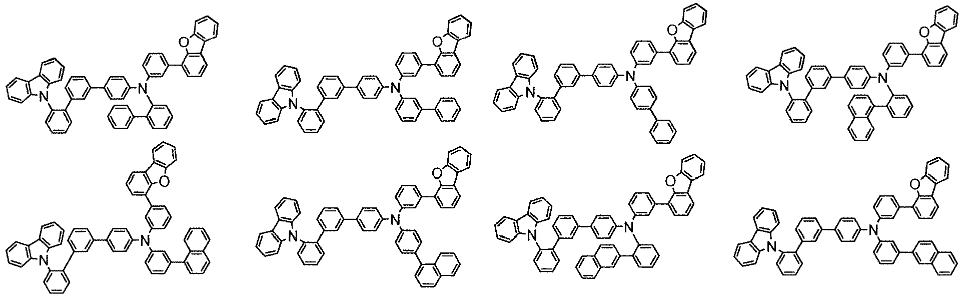

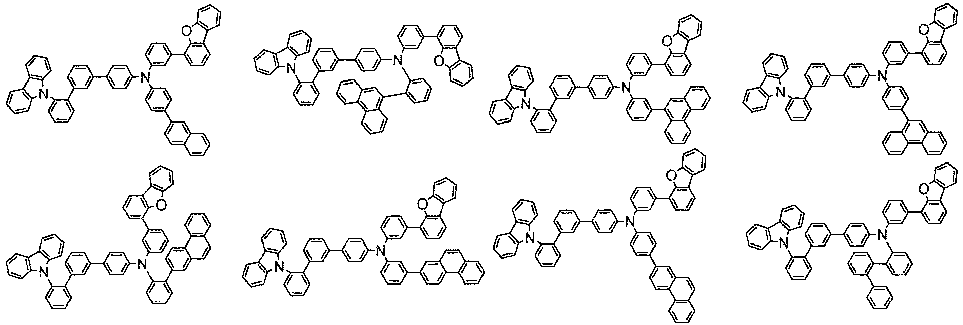

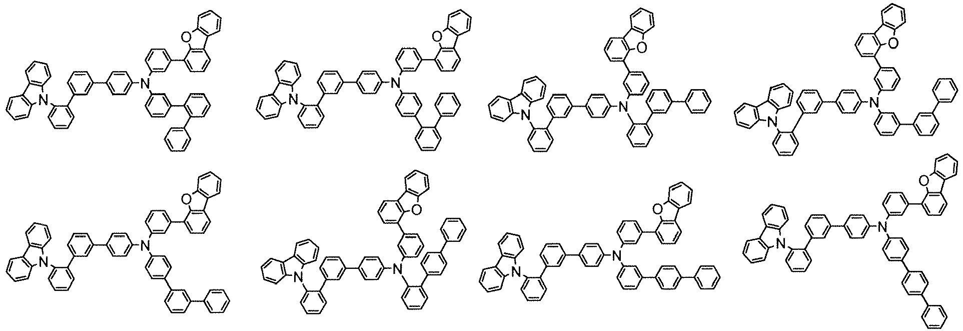

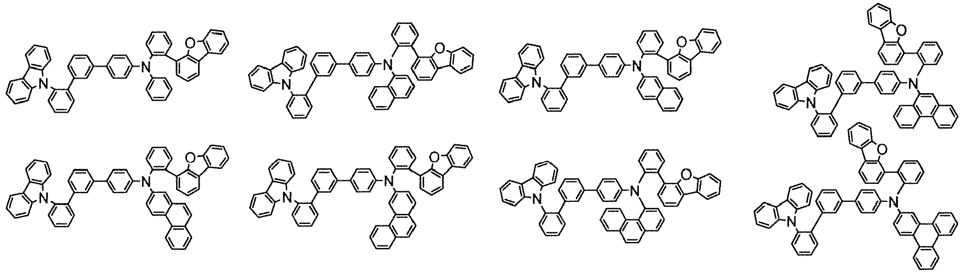

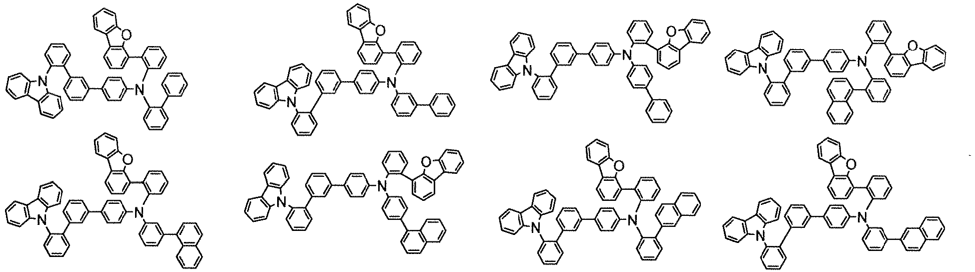

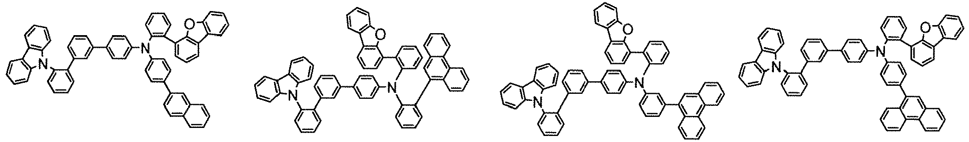

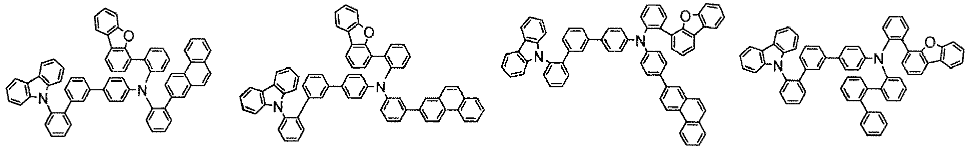

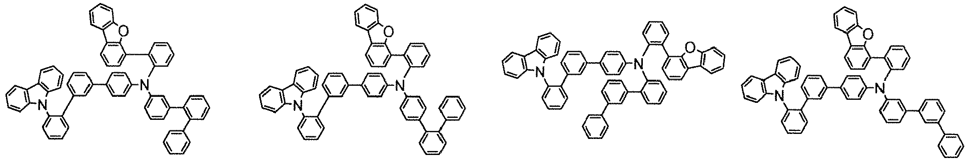

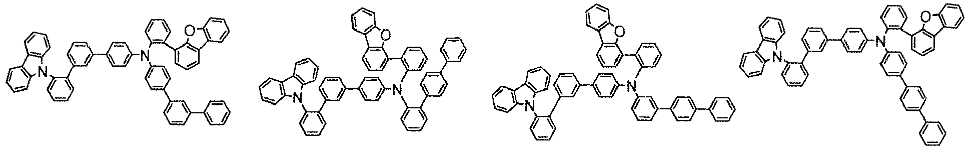

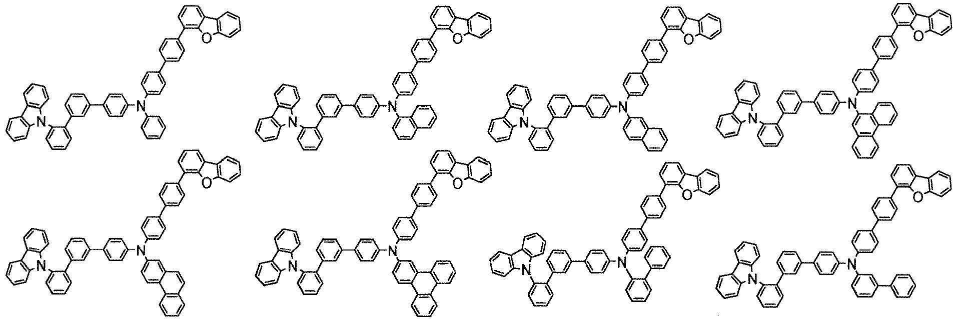

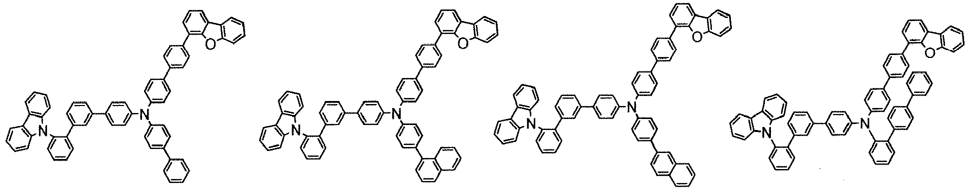

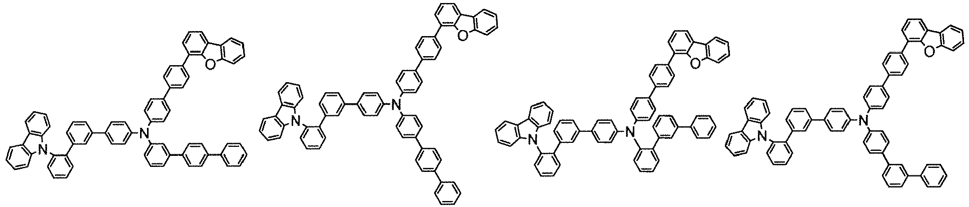

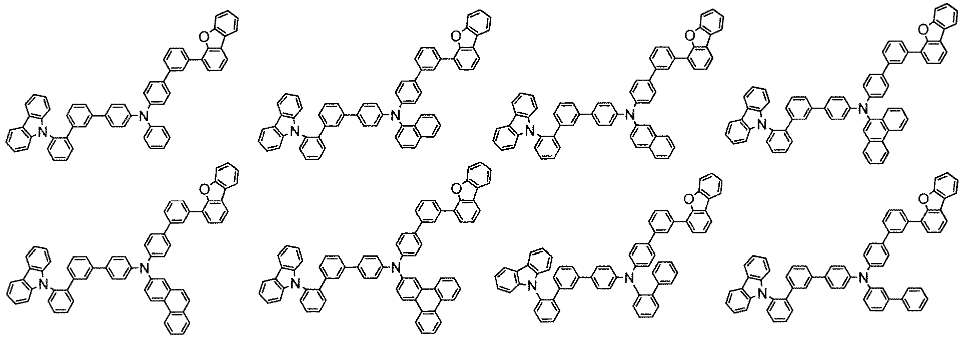

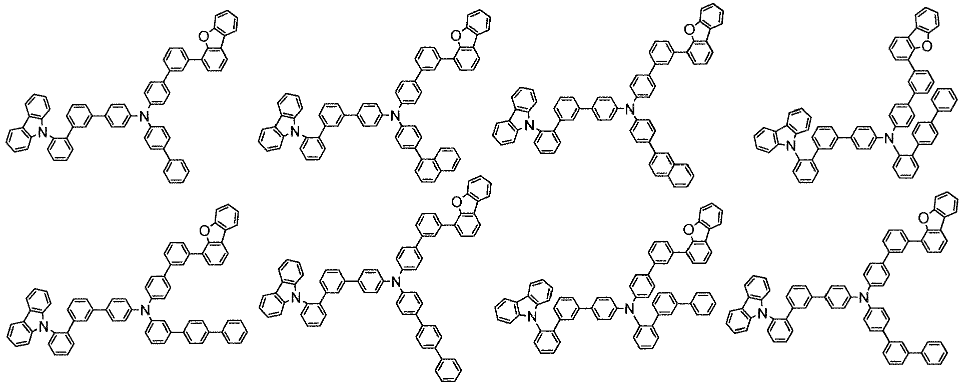

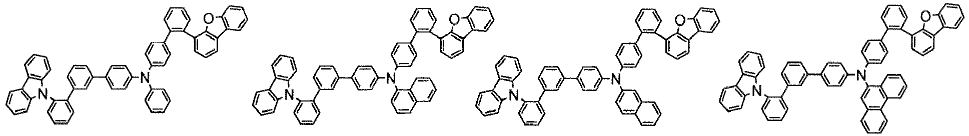

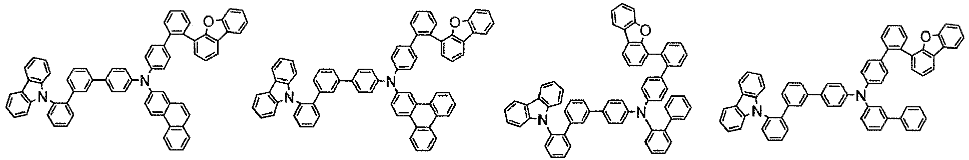

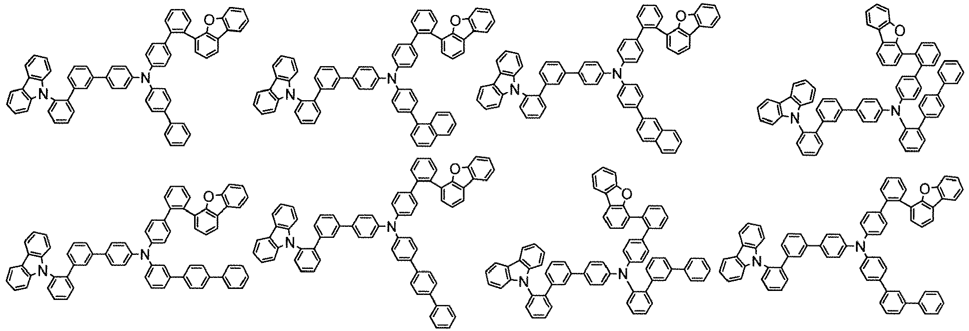

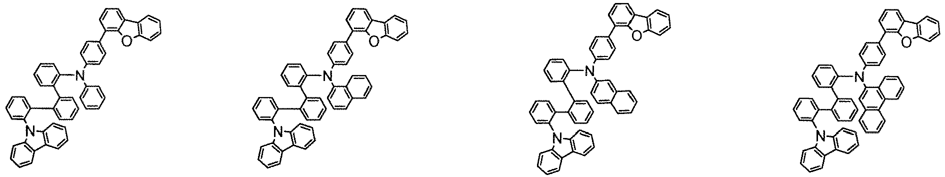

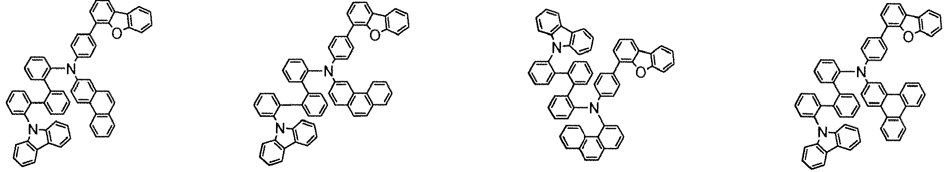

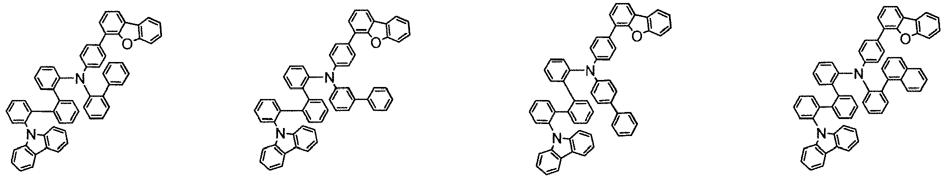

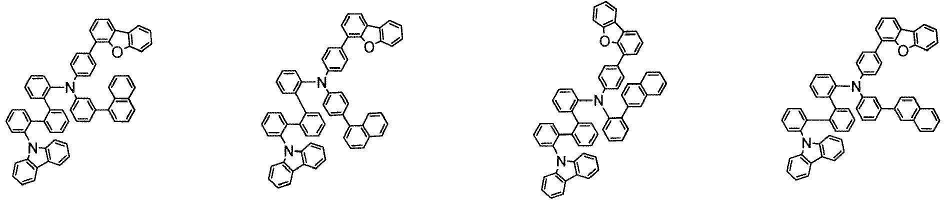

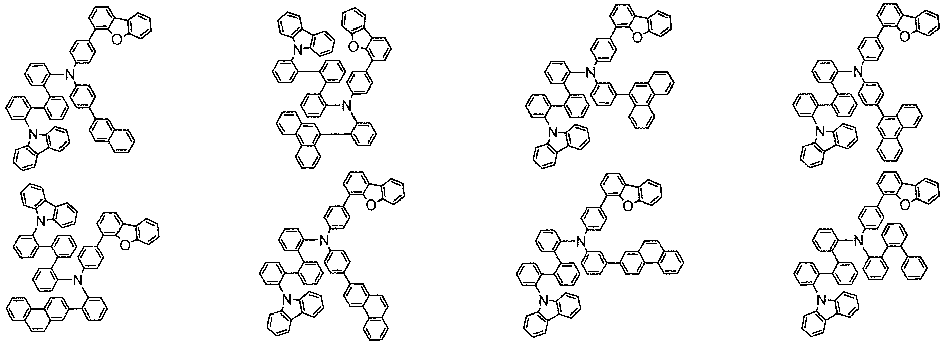

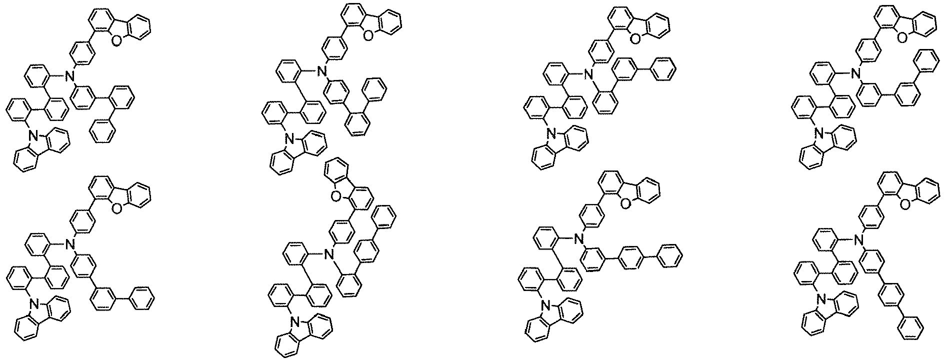

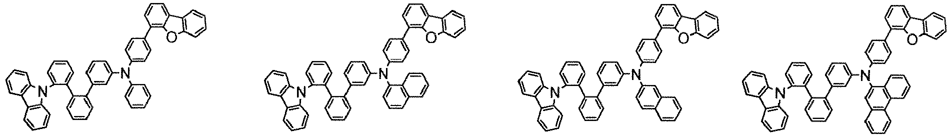

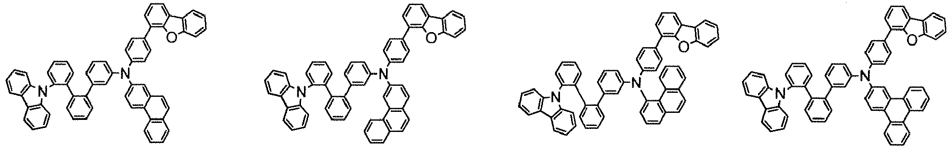

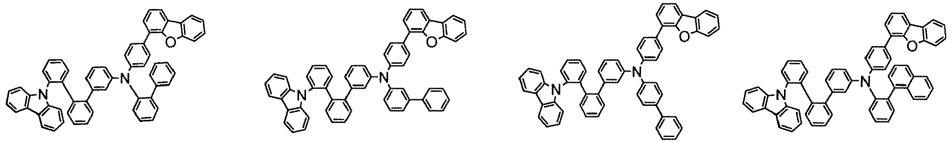

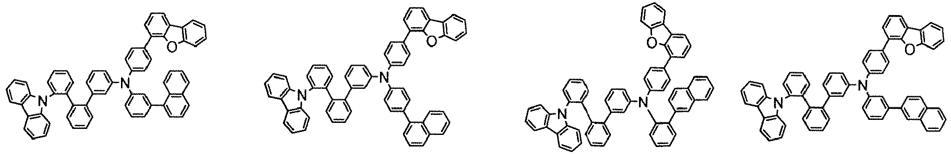

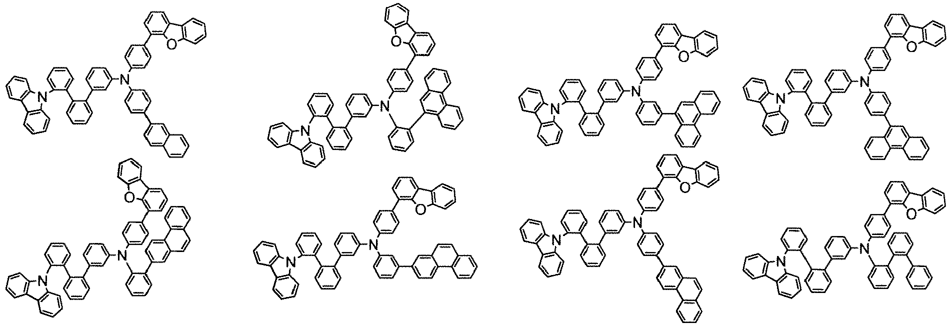

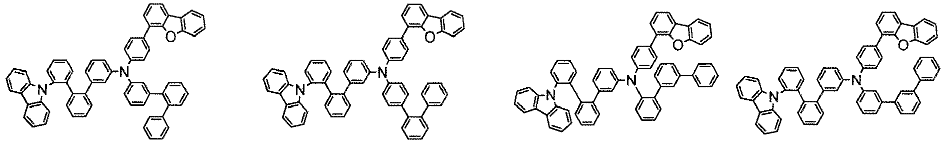

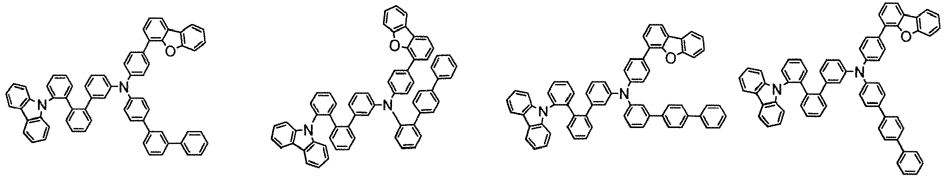

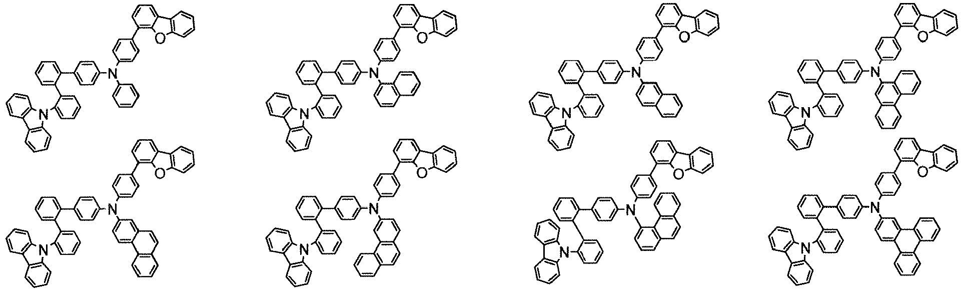

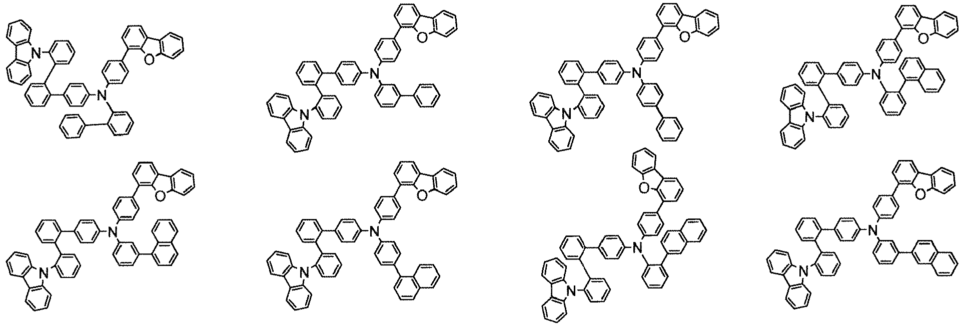

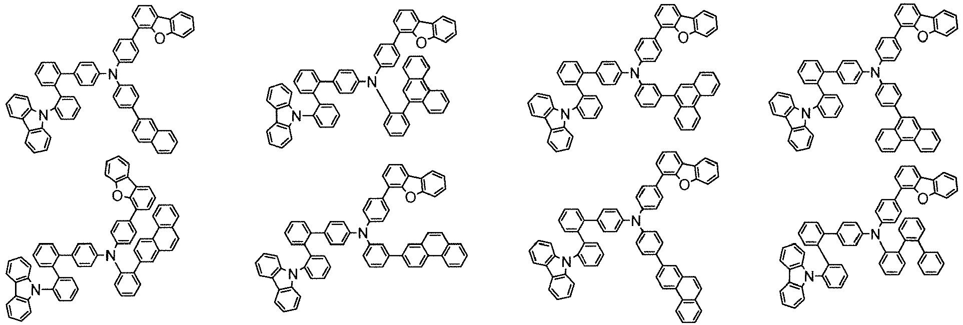

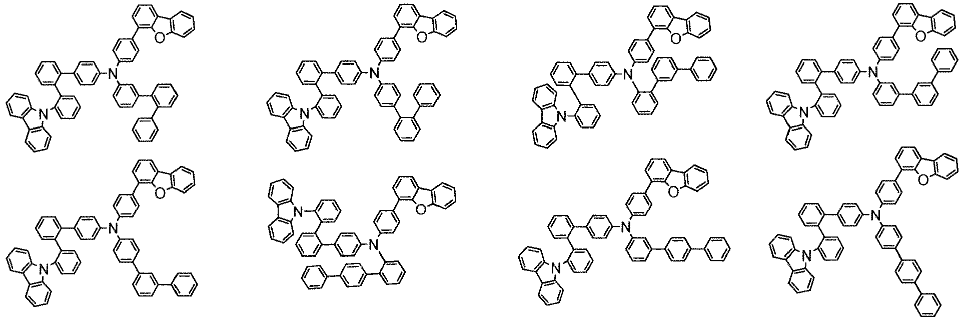

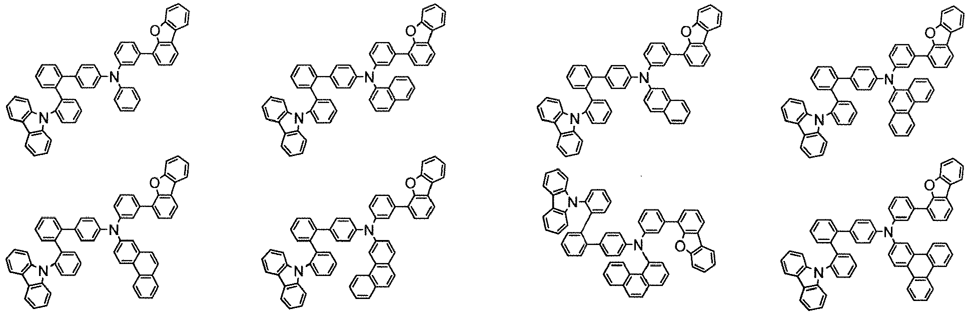

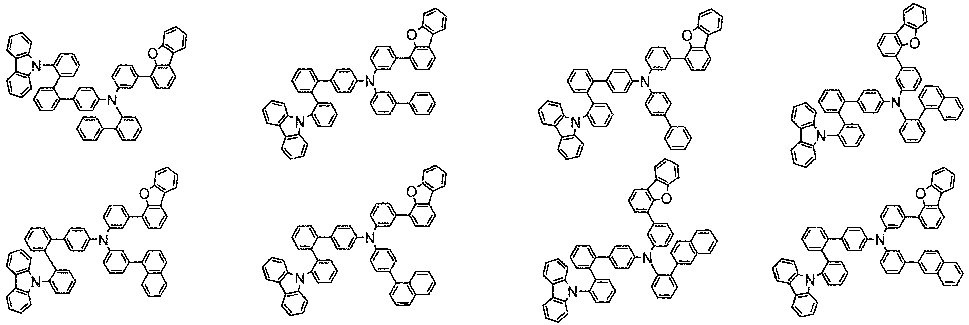

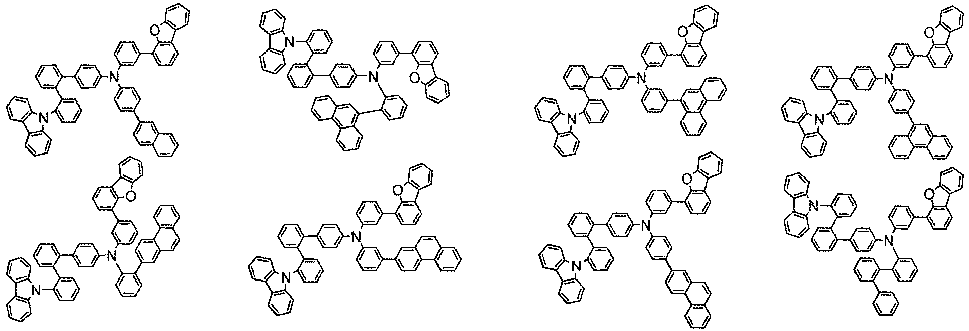

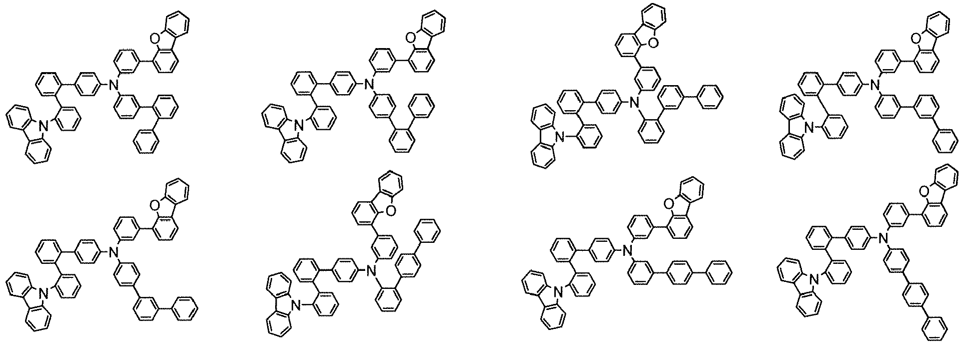

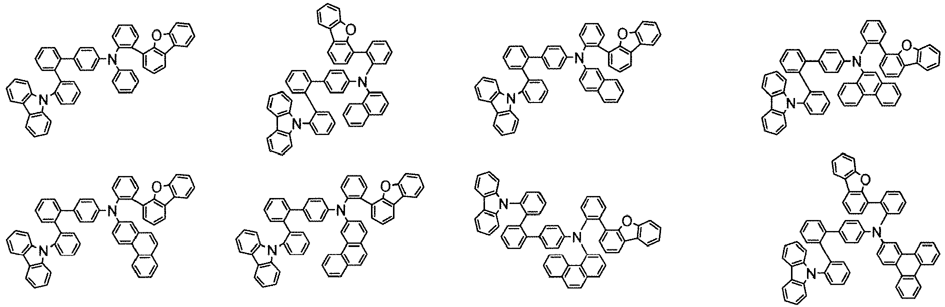

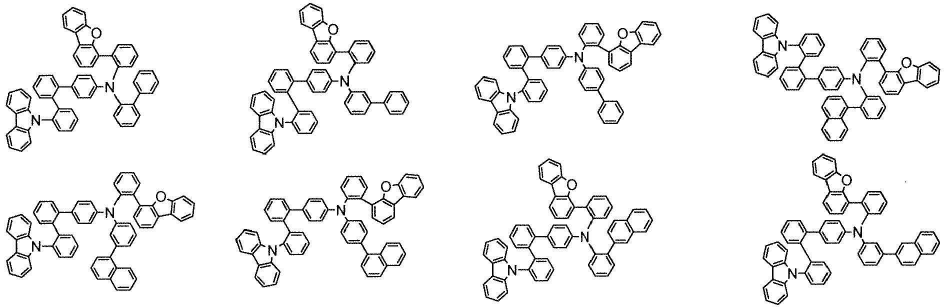

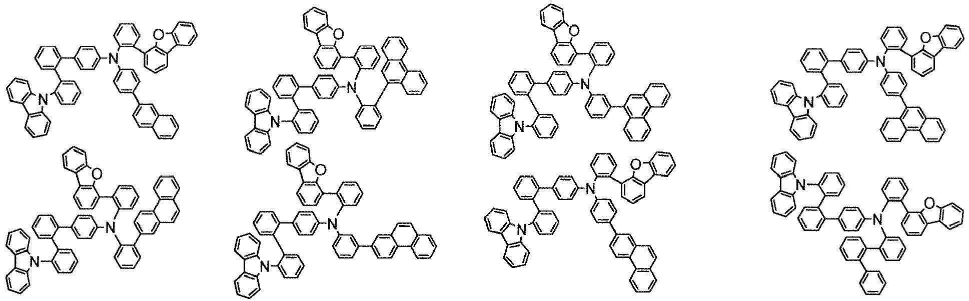

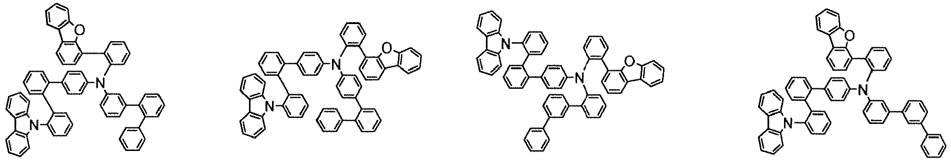

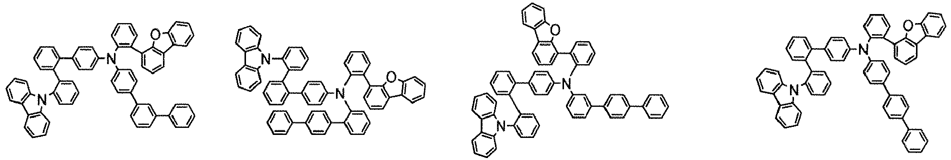

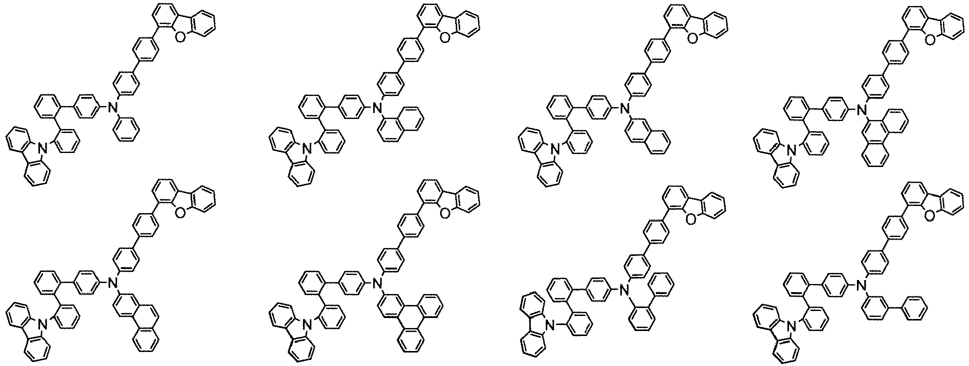

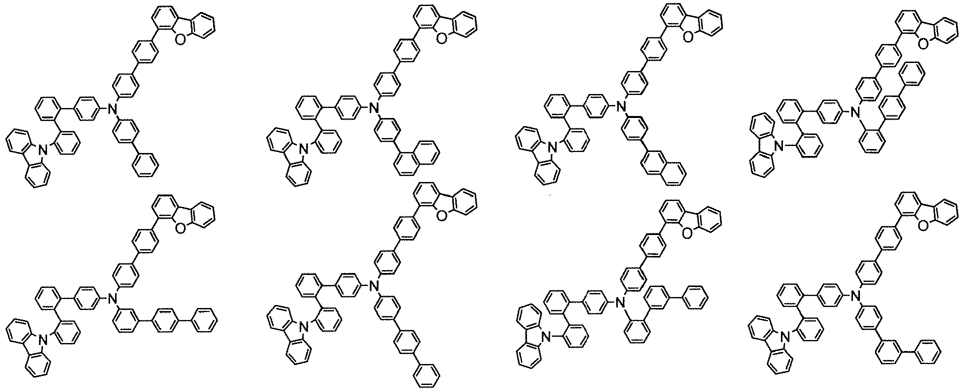

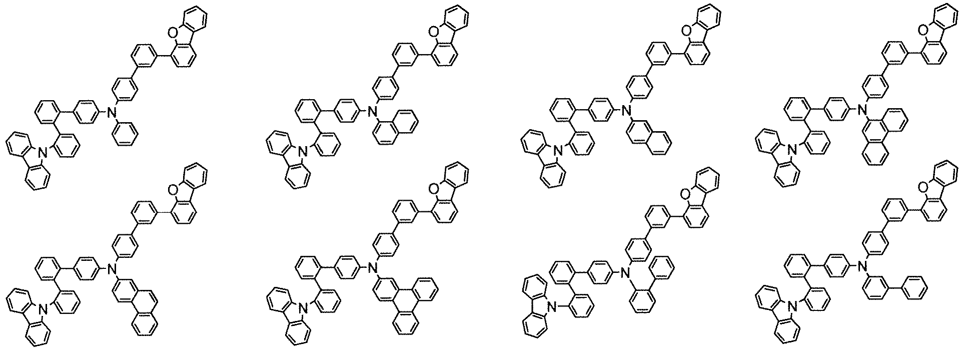

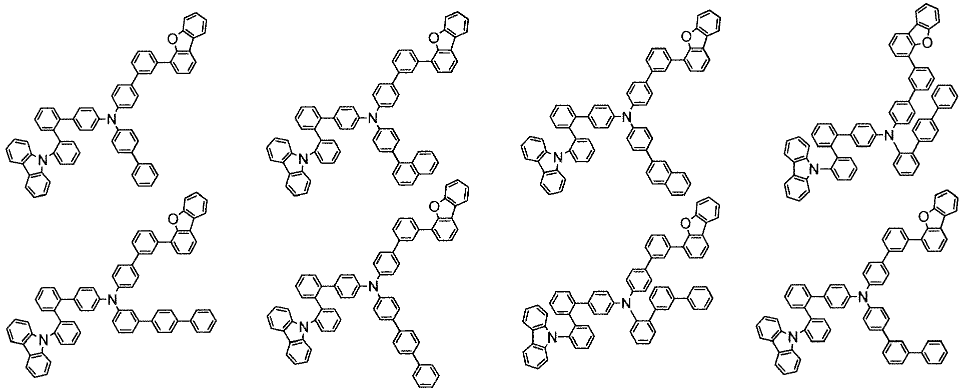

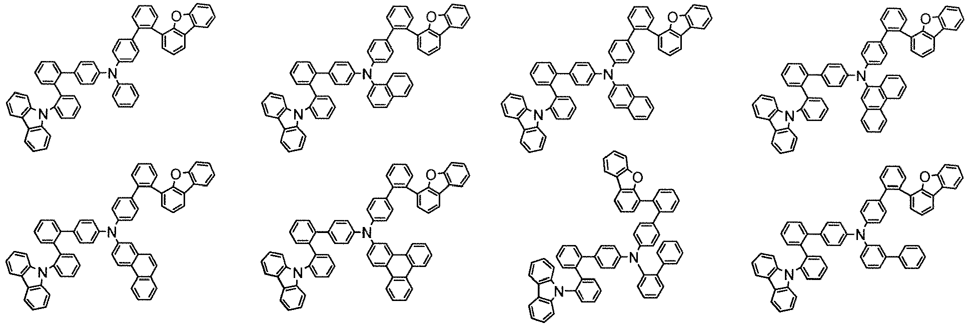

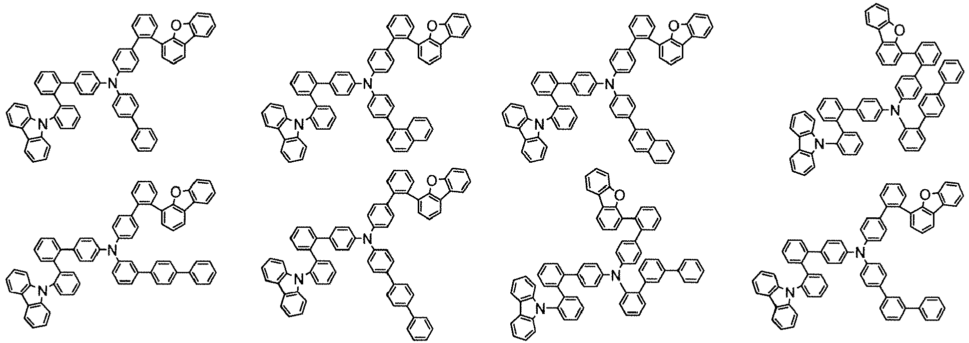

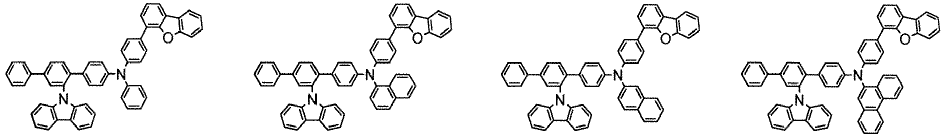

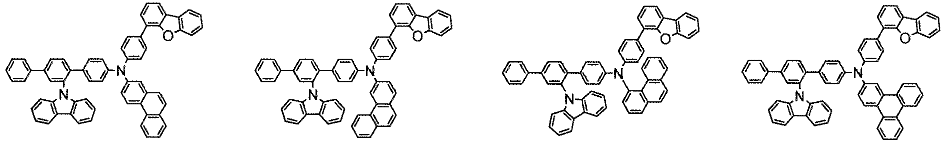

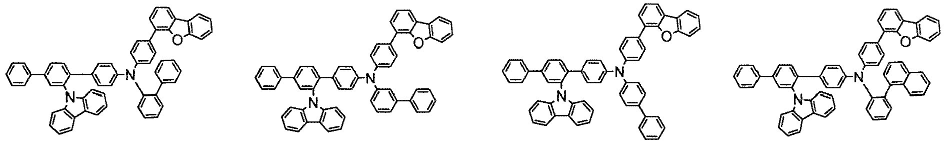

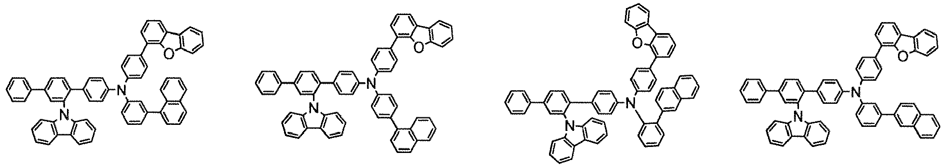

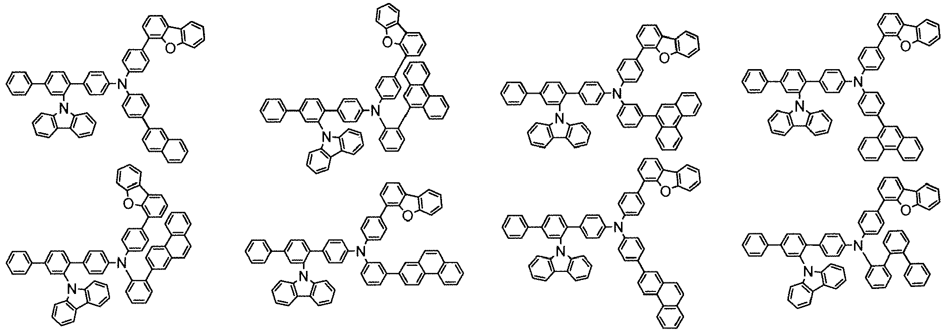

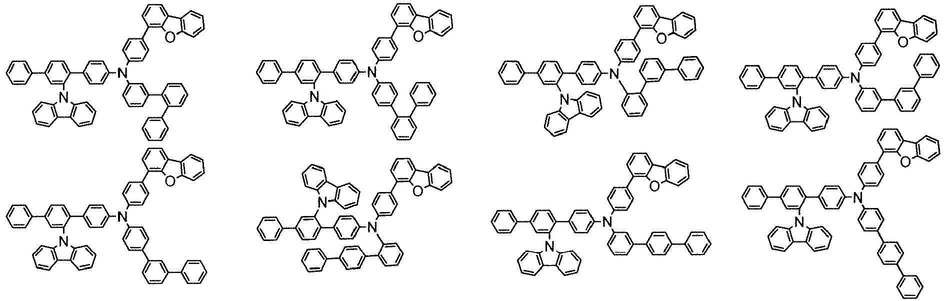

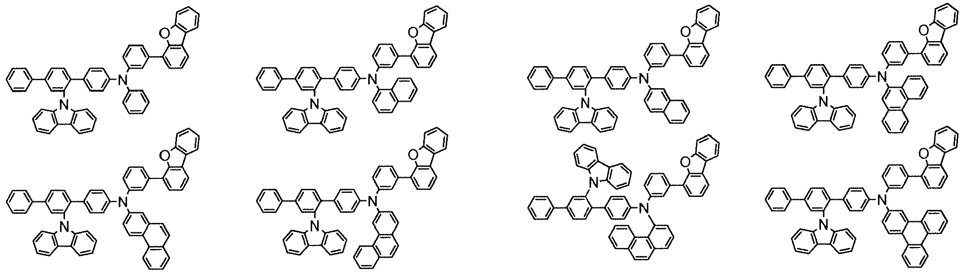

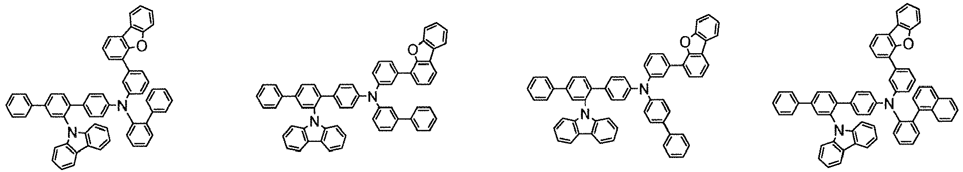

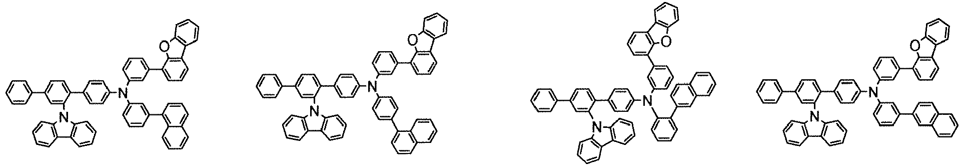

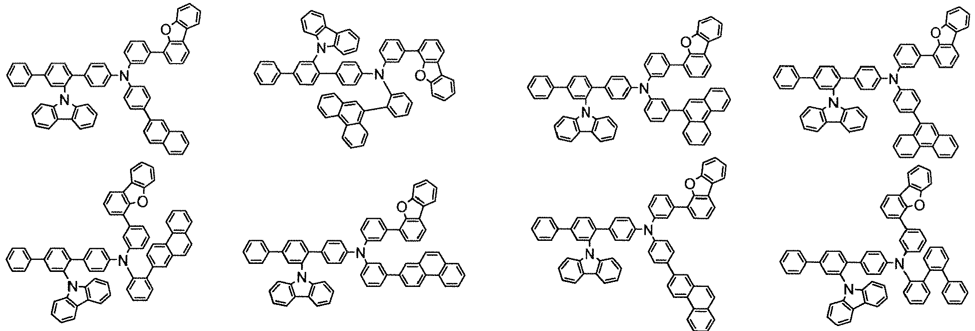

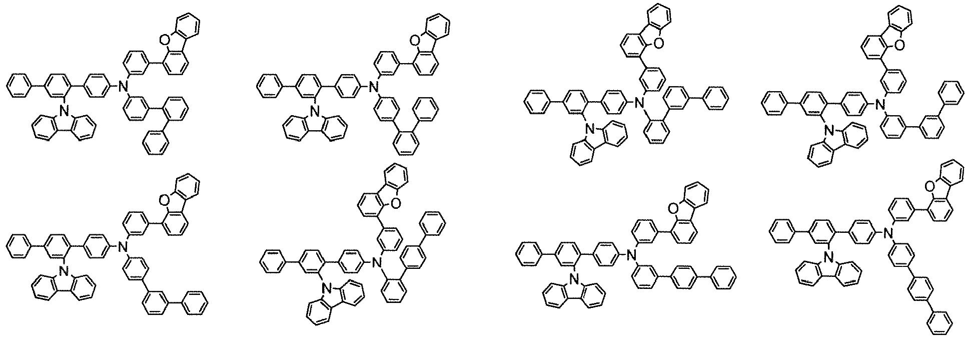

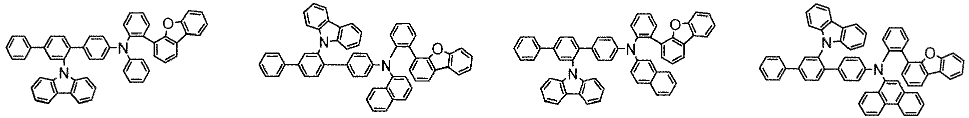

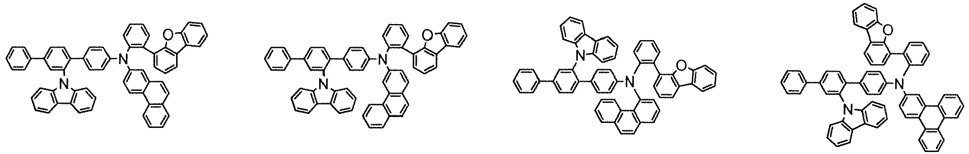

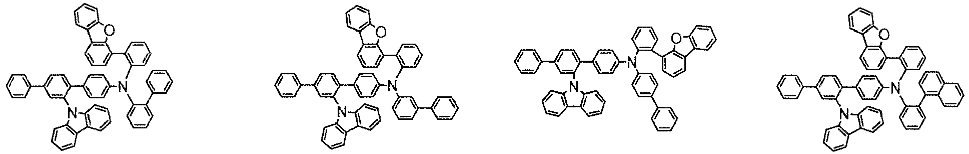

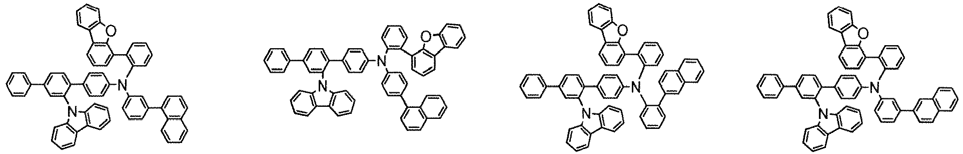

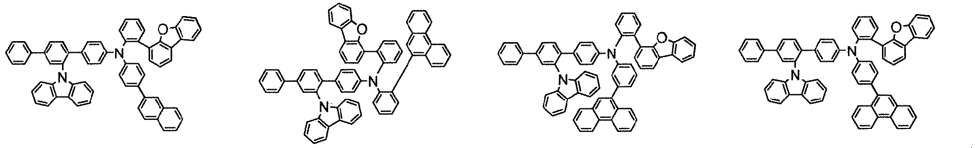

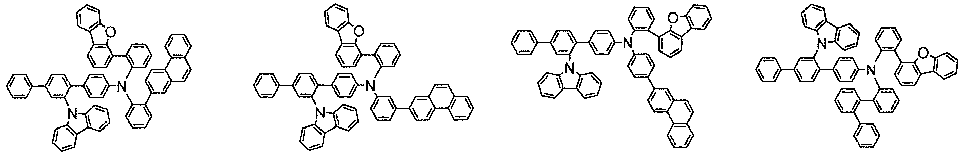

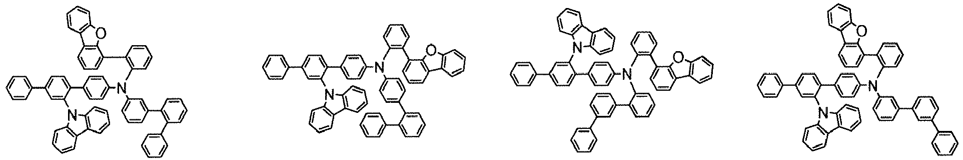

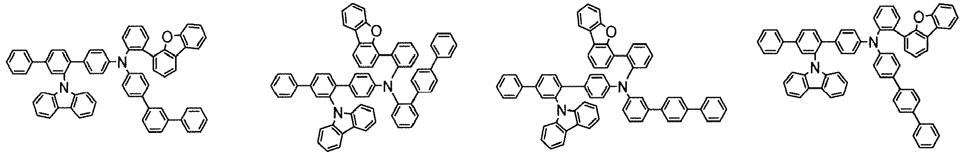

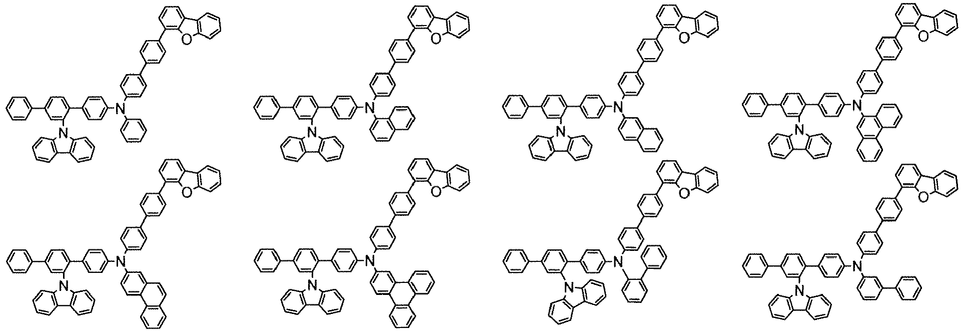

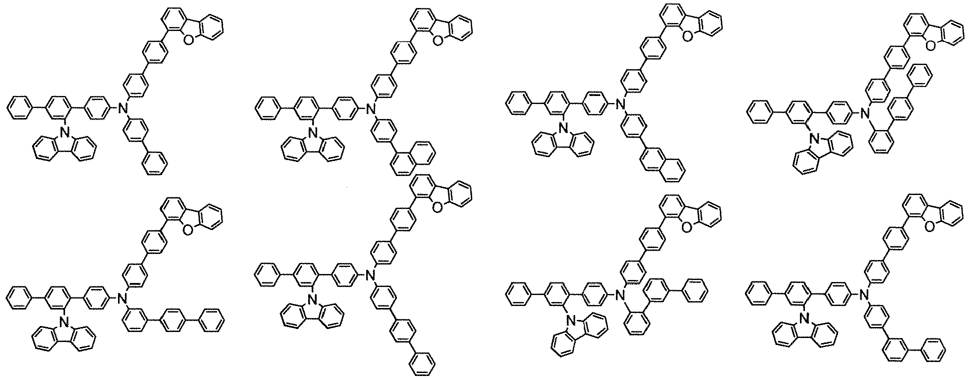

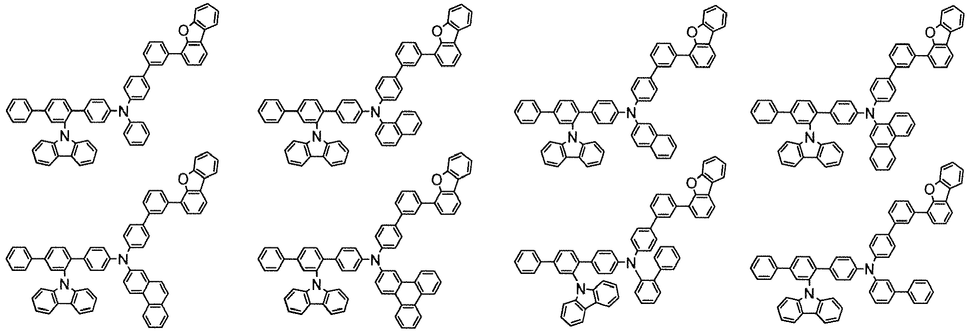

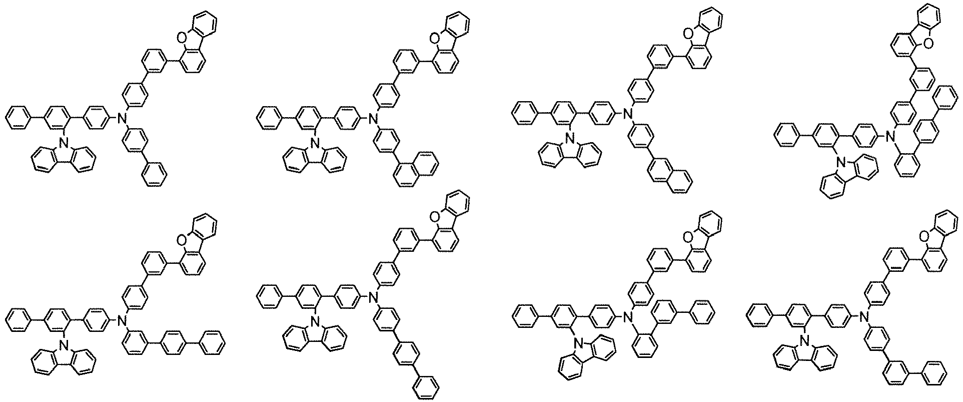

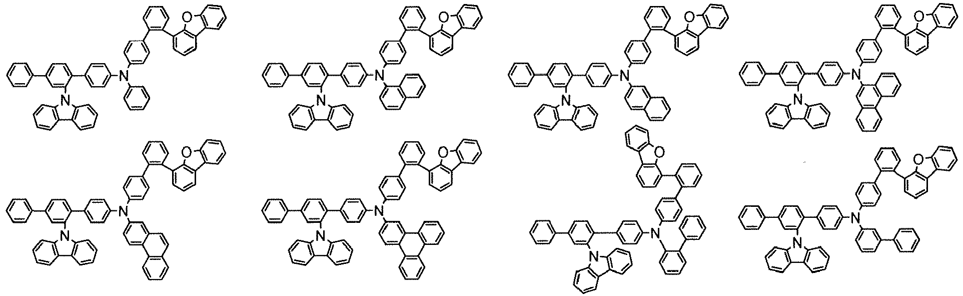

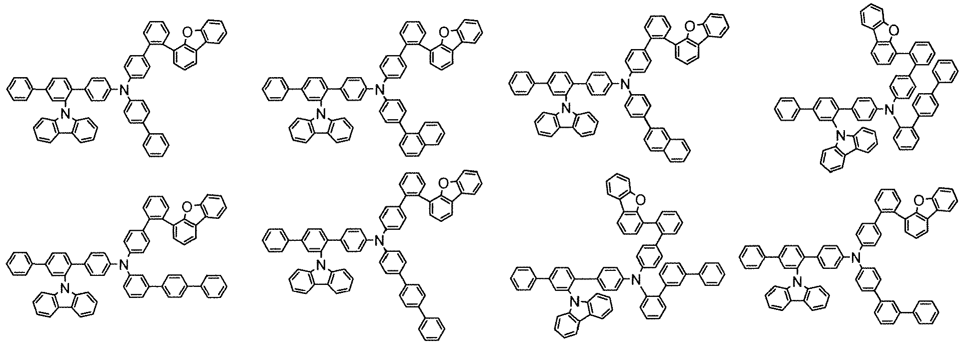

The production method of the compound (1) is not particularly limited and one of ordinary skill in the art could easily produce the compound (1) by a method described in the examples mentioned below and a modified method thereof by referring to known synthesis methods.

### Material for organic EL device

The material for organic EL device comprises the compound (1). The content of the compound (1) in the material for organic EL device is, for example, 1% by mass or more (inclusive of 100%), preferably 10% by mass or more (inclusive of 100%), more preferably 50% by mass or more (inclusive of 100%), still more preferably 80% by mass or more (inclusive of 100%), and particularly preferably 90% by mass or more (inclusive of 100%). The material for organic EL device is useful for the production of an organic EL device.

### Organic EL device

The organic EL device of the invention will be described below.

The organic EL device comprises a cathode, an anode, and an organic layer disposed between the cathode and the anode. The organic layer comprises a light emitting layer and at least one layer of the organic layer comprises the compound (1).

Examples of the organic layer which comprises the compound (1) include a hole transporting region formed between an anode and a light emitting layer, such as a hole transporting layer, a hole injecting layer, an electron blocking layer, and an exciton blocking layer, a light emitting layer, a space layer, and an electron transporting region formed between a cathode and a light emitting layer, such as an electron transporting layer, an electron injecting layer, and a hole blocking layer, although not limited thereto. The compound (1) is used for the production of a fluorescent or phosphorescent EL device preferably as a material for a hole transporting region or a light emitting layer, more preferably as a material for a hole transporting region, and still more preferably as a material for a hole transporting layer, an electron blocking layer or an exciton blocking layer.

The organic EL device of the invention may be any of a fluorescent or phosphorescent single color emitting device, a white-emitting device of fluorescent-phosphorescent hybrid type, a simple-type emitting device having a single emission unit, and a tandem emitting device having two or more emission units, with a fluorescent device being preferred. The "emission unit" referred to herein is the smallest unit for emitting light by the recombination of injected holes and injected electrons, which comprises an organic layer, wherein at least one layer is a light emitting layer.

Representative device structures of the simple-type organic EL device are shown below:

### (1) Anode/Emission unit/Cathode

The emission unit may be a multi-layered structure comprising two or more layers selected from a phosphorescent light emitting layer and a fluorescent light emitting layer. A space layer may be disposed between the light emitting layers to prevent the diffusion of excitons generated in the phosphorescent light emitting layer into the fluorescent light emitting layer. Representative layered structures of the simple-type emission unit are shown below, wherein the layers in parentheses are optional:
(a) (Hole injecting layer/)Hole transporting layer/Fluorescent emitting layer(/Electron transporting layer/Electron injecting layer);
(b) (Hole injecting layer/)Hole transporting layer/Phosphorescent emitting layer(/Electron transporting layer/Electron injecting layer);
(c) (Hole injecting layer/)Hole transporting layer/First fluorescent emitting layer/Second fluorescent emitting layer(/Electron transporting layer/Electron injecting layer);
(d) (Hole injecting layer/)Hole transporting layer/First phosphorescent emitting layer/Second phosphorescent emitting layer(/Electron transporting layer/Electron injecting layer);
(e) (Hole injecting layer/)Hole transporting layer/Phosphorescent emitting layer/Space layer/Fluorescent emitting layer(/Electron transporting layer/Electron injecting layer);
(f) (Hole injecting layer/)Hole transporting layer/First phosphorescent emitting layer/Second phosphorescent emitting layer/Space layer/Fluorescent emitting layer(/Electron transporting layer/Electron injecting layer);
(g) (Hole injecting layer/)Hole transporting layer/First phosphorescent emitting layer/Space layer/Second phosphorescent emitting layer/Space layer/Fluorescent emitting layer(/Electron transporting layer/Electron injecting layer);
(h) (Hole injecting layer/)Hole transporting layer/Phosphorescent emitting layer/Space layer/First fluorescent emitting layer/Second fluorescent emitting layer(/Electron transporting layer/Electron injecting layer);
(i) (Hole injecting layer/)Hole transporting layer/Electron blocking layer/Fluorescent emitting layer(/Electron transporting layer/Electron injecting layer);
(j) (Hole injecting layer/)Hole transporting layer/Electron blocking layer/Phosphorescent emitting layer(/Electron transporting layer/Electron injecting layer);
(k) (Hole injecting layer/)Hole transporting layer/Exciton blocking layer/Fluorescent emitting layer(/Electron transporting layer/Electron injecting layer);
(l) (Hole injecting layer/)Hole transporting layer/Exciton blocking layer/Phosphorescent emitting layer(/Electron transporting layer/Electron injecting layer);
(m) (Hole injecting layer/)First hole transporting layer/Second hole transporting layer/Fluorescent emitting layer(/Electron transporting layer/Electron injecting layer);
(n) (Hole injecting layer/)First hole transporting layer/Second hole transporting layer/Phosphorescent emitting layer(/Electron transporting layer/Electron injecting layer);
(o) (Hole injecting layer/)First hole transporting layer/Second hole transporting layer/Fluorescent emitting layer/First electron transporting layer/Second electron transporting layer(/Electron injecting layer);
(p) (Hole injecting layer/)First hole transporting layer/Second hole transporting layer/Phosphorescent emitting layer/First electron transporting layer/Second electron transporting layer(/Electron injecting layer);
(q) (Hole injecting layer/)Hole transporting layer/Fluorescent emitting layer/Hole blocking layer(/Electron transporting layer/Electron injecting layer);
(r) (Hole injecting layer/)Hole transporting layer/Phosphorescent emitting layer/Hole blocking layer(/Electron transporting layer/Electron injecting layer);
(s) (Hole injecting layer/)Hole transporting layer/Fluorescent emitting layer/Exciton blocking layer(/Electron transporting layer/Electron injecting layer); and
(t) (Hole injecting layer/)Hole transporting layer/Phosphorescent emitting layer/ Exciton blocking layer(/Electron transporting layer/Electron injecting layer).

The emission colors of phosphorescent emitting layers or fluorescent emitting layers may be different. For example, the emission unit (f) may be (Hole injecting layer)/Hole transporting layer/First phosphorescent emitting layer (red emission)/Second phosphorescent emitting layer (green emission)/Space layer/Fluorescent emitting layer (blue emission)/Electron transporting layer.

An electron blocking layer may be disposed between each light emitting layer and the hole transporting layer or between each light emitting layer and the space layer, if necessary. Also, a hole blocking layer may be disposed between each light emitting layer and the electron transporting layer, if necessary. With such an electron blocking layer or a hole blocking layer, electrons and holes are confined in the light emitting layer to increase the charge recombination in the light emitting layer, thereby improving the emission efficiency.

Representative device structure of the tandem-type organic EL device is shown below:

### (2) Anode/First emission unit/Intermediate layer/Second emission unit/Cathode.

The layered structure of the first emission unit and the second emission unit may be selected from those described above with respect to the emission unit.

Generally, the intermediate layer is also called an intermediate electrode, an intermediate conductive layer, a charge generation layer, an electron withdrawing layer, a connecting layer, or an intermediate insulating layer. The intermediate layer supplies electrons to the first emission unit and holes to the second emission unit and may be formed by known materials.

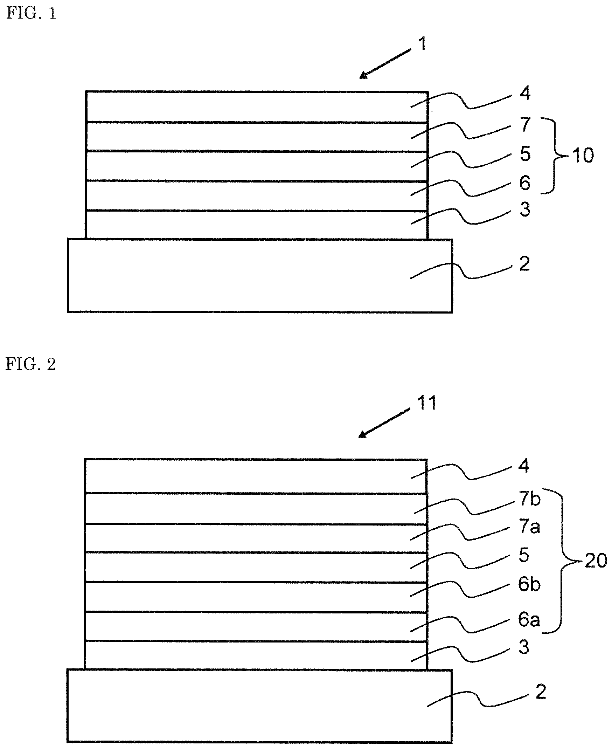

Fig. 1 is a schematic illustration showing the structure of an example of the organic EL device of the invention, wherein the organic EL device 1 comprises a substrate 2, an anode 3, a cathode 4, and an emission unit 10 disposed between the anode 3 and the cathode 4. The emission unit 10 comprises a light emitting layer 5. A hole transporting region 6 (for example, a hole injecting layer or a hole transporting layer) is disposed between the light emitting layer 5 and the anode 3, and an electron transporting region 7 (for example, an electron injecting layer or an electron transporting layer) is disposed between the light emitting layer 5 and the cathode 4. An electron blocking layer (not shown) may be disposed on the anode 3 side of the light emitting layer 5, and a hole blocking layer (not shown) may be disposed on the cathode 4 side of the light emitting layer 5. With these blocking layers, electrons and holes are confined in the light emitting layer 5 to increase the exciton generation in the light emitting layer 5.

Fig. 2 is a schematic illustration showing the structure of another example of the organic EL device, wherein the organic EL device 11 comprises a substrate 2, an anode 3, a cathode 4, and an emission unit 20 disposed between the anode 3 and the cathode 4. The emission unit 20 comprises a light emitting layer 4. The hole transporting region disposed between the anode 3 and the light emitting layer 5 is formed by a first hole transporting layer 6a and a second hole transporting layer 6b. The electron transporting region disposed between the light emitting layer 5 and the cathode 4 is formed by a first electron transporting layer 7a and a second electron transporting layer 7b.

In the present invention, a host is referred to as a fluorescent host when combinedly used with a fluorescent dopant (fluorescent emitting material) and as a phosphorescent host when combinedly used with a phosphorescent dopant (phosphorescent emitting material). Therefore, the fluorescent host and the phosphorescent host are not distinguished from each other merely by the difference in their molecular structures. Namely, in the present invention, the term "phosphorescent host" means a material for constituting a phosphorescent emitting layer containing a phosphorescent dopant and does not mean a material that cannot be used as a material for a fluorescent emitting layer. The same applies to the fluorescent host.

### Substrate

The substrate is a support for the emitting device and made of, for example, glass, quartz, and plastics. The substrate may be a flexible substrate, for example, a plastic substrate made of polycarbonate, polyarylate, polyether sulfone, polypropylene, polyester, polyvinyl fluoride, or polyvinyl chloride. An inorganic deposition film is also usable.

### Anode

The anode is formed on the substrate preferably from a metal, an alloy, an electrically conductive compound, and a mixture thereof, each having a large work function, for example, 4.0 eV or more. Examples of the material for the anode include indium oxide-tin oxide (ITO: indium tin oxide), indium oxide-tin oxide doped with silicon or silicon oxide, indium oxide-zinc oxide, indium oxide doped with tungsten oxide and zinc oxide, and graphene. In addition, gold (Au), platinum (Pt), nickel (Ni), tungsten (W), chromium (Cr), molybdenum (Mo, iron (Fe), cobalt (Co), copper (Cu), palladium (Pd), titanium (Ti), and a nitride of the above metal (for example, titanium nitride) are also usable.

These anode materials are made into a film generally by a sputtering method. For example, a film of indium oxide-zinc oxide is formed by sputtering an indium oxide target doped with 1 to 10 wt% of zinc oxide, and a film of indium oxide doped with tungsten oxide and zinc oxide is formed by sputtering an indium oxide target doped with 0.5 to 5 wt% of tungsten oxide and 0.1 to 1 wt% of zinc oxide. In addition, a vacuum vapor deposition method, a coating method, an inkjet method, and a spin coating method are usable.

A hole injecting layer to be optionally formed in contact with the anode is formed from a material which is capable of easily injecting holes independently of the work function of the anode. Therefore, the anode can be formed by a material generally known as an electrode material, for example, a metal, an alloy, an electroconductive compound, a mixture thereof, and a group 1 element and a group 2 element of the periodic table.

A material having a small work function belonging to a group 1 or a group 2 of the periodic table, for example, an alkali metal, such as lithium (Li) and cesium (Cs), an alkaline earth metal, such as magnesium (Mg), calcium (Ca), and strontium (Sr), and an alloy thereof, such as MgAg and AlLi, are also usable as an anode material. In addition, a rare earth metal, such as europium and ytterbium, and an alloy thereof are also usable. The alkali metal, the alkaline earth metal, and the alloy thereof is made into the anode by a vacuum vapor deposition or a sputtering method. When a silver paste is used, a coating method and an inkjet method are usable.

### Hole injecting layer

The hole injecting layer comprises a material having a high hole injecting ability (hole injecting material). The compound (1) may be used in a hole injecting layer alone or in combination with the material described below.

Examples of the hole injecting material other than the compound (1) include molybdenum oxide, titanium oxide, vanadium oxide, rhenium oxide, ruthenium oxide, chromium oxide, zirconium oxide, hafnium oxide, tantalum oxide, silver oxide, tungsten oxide, and manganese oxide.

The following low molecular aromatic amine compound is also usable as the hole injecting layer material: 4,4',4"-tris(N,N-diphenylamino)triphenylamine (TDATA), 4,4',4"-tris[N-(3-methylphenyl)-N-phenylamino]triphenylamine (MTDATA), 4,4'-bis[N-(4-diphenylaminophenyl)-N-phenylamino]biphenyl (DPAB), 4,4'-bis(N-{4-[N'-(3-methylphenyl)-N'-phenylamino]phenyl}-N-phenylamino)biphenyl (DNTPD), 1,3,5-tris[N-(4-diphenylaminophenyl)-N-phenylamino]benzene (DPA3B), 3-[N-(9-phenylcarbazole-3-yl)-N-phenylamino]-9-phenylcarbazole (PCzPCAl), 3,6-bis[N-(9-phenylcarbazole-3-yl)-N-phenylamino]-9-phenylcarbazole (PCzPCA2), and 3-[N-(1-naphthyl)-N-(9-phenylcarbazole-3-yl)amino]-9-phenylcarbazole (PCzPCN1).

A macromolecular compound, such as an oligomer, a dendrimer, a polymer, is also usable as the hole injecting layer material. Examples thereof include poly(N-vinylcarbazole) (PVK), poly(4-vinyltriphenylamine) (PVTPA), poly[N-(4-{N'-[4-(4-diphenylamino)phenyl]phenyl-N'-phenylamino}phenyl)methacrylamide] (PTPDMA), and poly[N,N'-bis(4-butylphenyl)-N,N'-bis(phenyl)benzidine] (Poly-TPD). A macromolecular compound doped with an acid, such as poly(3,4-ethylenedioxythiophene)/poly(styrenesulfonic acid) (PEDOT/PSS) and polyaniline/poly(styrenesulfonic acid) (PAni/PSS), is also usable.

In addition, an acceptor material, such as a hexaazatriphenylene (HAT) compound represented by formula (K), is preferably used in combination with the compound (1): 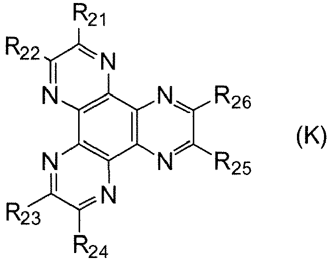 wherein:
R₂₁ to R₂₆ are each independently a cyano group, -CONH₂, a carboxyl group, or -COOR₂₇ wherein R₂₇ is an alkyl group having 1 to 20 carbon atoms or a cycloalkyl group having 3 to 20 ring carbon atoms, or
adjacent two selected from R₂₁ and R₂₂, R₂₃ and R₂₄, and R₂₅ and R₂₆ may be bonded to each other to form a group represented by -CO-O-CO-.

Examples of R₂₇ include a methyl group, an ethyl group, a n-propyl group, an isopropyl group, a n-butyl group, an isobutyl group, a t-butyl group, a cyclopentyl group, and a cyclohexyl group.

### Hole transporting layer

The hole transporting layer comprises a material having a high hole transporting ability (hole transporting material). The compound (1) is preferably used in a hole transporting layer alone or in combination with the compound described below.

Examples of the hole transporting material other than the compound (1) includes an aromatic amine compound, a carbazole derivative, and an anthracene derivative.

Examples of the aromatic amine compound include 4,4'-bis[N-(1-naphthyl)-N-phenylamino]biphenyl (NPB), N,N'-bis(3-methylphenyl)-N,N'-diphenyl-[1,1'-biphenyl]-4,4'-diamine (TPD), 4-phenyl-4'-(9-phenylfluorene-9-yl)triphenylamine (BAFLP), 4,4'-bis[N-(9,9-dimethylfluorene-2-yl)-N-phenylamino]biphenyl (DFLDPBi), 4,4',4"-tris(N,N-diphenylamino)triphenylamine (TDATA), 4,4',4"-tris[N-(3-methylphenyl)-N-phenylamino]triphenylamine (MTDATA), and 4,4'-bis[N-(spiro-9,9'-bifluorene-2-yl)-N-phenylamino]biphenyl (BSPB). The above compounds have a hole mobility of 10⁻⁶ cm²[Vs or more.

Examples of the carbazole derivative include 4,4'-di(9-carbazolyl)biphenyl (CBP), 9-[4-(9-carbazolyl)phenyl]-10-phenylanthracene (CzPA), and 9-phenyl-3-[4-(10-phenyl-9-anthryl)phenyl]-9H-carbazole (PCzPA).

Examples of the anthracene derivative include 2-t-butyl-9,10-di(2-naphthyl)anthracene (t-BuDNA), 9,10-di(2-naphthyl)anthracene (DNA), and 9,10-diphenylanthracene (DPAnth).

In addition, a macromolecular compound, such as poly(N-vinylcarbazole) (PVK) and poly(4-vinyltriphenylamine) (PVTPA) are usable.

Compounds other than those mentioned above are also usable, if their hole transporting ability is higher than their electron transporting ability.

The hole transporting layer may be a single layer or a multi-layer of two or more layers. For example, the hole transporting layer may be a two-layered structure comprising a first hole transporting layer (anode side) and a second hole transporting layer (cathode side). In an embodiment of the invention, a hole transporting layer of a single-layered structure is preferably in contact with a light emitting layer and a hole transporting layer in a multi-layered structure which is closest to a cathode, for example, the second hole transporting layer in the two-layered structure mentioned above, is preferably in contact with a light emitting layer. In another embodiment of the invention, an electron blocking layer may be disposed between the light emitting layer and the hole transporting layer of the single-layered structure or between the light emitting layer and the hole transporting layer in the multi-layered structure which is closest to the light emitting layer.

In the two-layered structure of the hole transporting layer, the compound (1) may be included in one or both of the first hole transporting layer and the second hole transporting layer. The compound (1) included in the first hole transporting layer and the compound (1) included in the second hole transporting layer are different.

In an embodiment of the invention, the compound (1) is preferably used in the first hole transporting layer. In another embodiment, the compound (1) is preferably used in the second hole transporting layer. In still another embodiment, the compound (1) is preferably used in both the first hole transporting layer and the second hole transporting layer.

### Dopant material of light emitting layer

The light emitting layer comprises a highly light-emitting material (dopant material) and may be formed from a various kind of materials. For example, a fluorescent emitting material and a phosphorescent emitting material are usable as the dopant material. The fluorescent emitting material is a compound capable of emitting light from a singlet excited state, and the phosphorescent emitting material is a compound capable of emitting light from a triplet excited state.

Examples of blue fluorescent emitting material usable in the light emitting layer include a pyrene derivative, a styrylamine derivative, a chrysene derivative, a fluoranthene derivative, a fluorene derivative, a diamine derivative, and a triarylamine derivative, such as N,N'-bis[4-(9H-carbazole-9-yl)phenyl]-N,N'-diphenylstilbene-4,4'-diamine (YGA2S), 4-(9H-carbazole-9-yl)-4'-(10-phenyl-9-anthryl)triphenylamine (YGAPA), and 4-(10-phenyl-9-anthryl)-4'-(9-phenyl-9H-carbazole-3-yl)triphenylamine (PCBAPA).

Examples of green fluorescent emitting material usable in the light emitting layer include an aromatic amine derivative, such as N-(9,10-diphenyl-2-anthryl)-N,9-diphenyl-9H-carbazole-3-amine (2PCAPA), N-[9,10-bis(1,1'-biphenyl-2-yl)-2-anthryl]-N,9-diphenyl-9H-carbazole-3-amine (2PCABPhA), N-(9,10-diphenyl-2-anthryl)-N,N',N'-triphenyl-1,4-phenylenediamine (2DPAPA), N-[9,10-bis(1,1'-biphenyl-2-yl)-2-anthryl]-N,N',N'-triphenyl-1,4-phenylenediamine (2DPABPhA), N-[9,10-bis(1,1'-biphenyl-2-yl)]-N-[4-(9H-carbazole-9-yl)phenyl]-N-phenylanthracene-2-amine (2YGABPhA), and N,N,9-triphenylanthracene-9-amine (DPhAPhA).

Examples of red fluorescent emitting material usable in the light emitting layer include a tetracene derivative and a diamine derivative, such as N,N,N',N'-tetrakis(4-methylphenyl)tetracene-5,11-diamine (p-mPhTD) and 7,14-diphenyl-N,N,N',N'-tetrakis(4-methylphenyl)acenaphtho[1,2-alfluoranthene-3,10-diamine (p-mPhAFD).

Examples of blue phosphorescent emitting material usable in the light emitting layer include a metal complex, such as an iridium complex, an osmium complex, and a platinum complex. Examples thereof include bis[2-(4',6'-difluorophenyl)pyridinato-N,C2']iridium(III) tetrakis(1-pyrazolyl)borato (Fir₆), bis[2-(4',6'-difluorophenyl)pyridinato-N,C2']iridium(III) picolinato (FIrpic), bis[2-(3',5'-bistrifluoromethylphenyl)pyridinato-N,C2']iridium(III) picolinato (Ir(CF₃ppy)₂(pic)), and bis[2-(4',6'-difluorophenyl)pyridinato-N,C2']iridium(III) acetylacetonato (FIracac).

Examples of green phosphorescent emitting material usable in the light emitting layer include an iridium complex, such as tris(2-phenylpyridinato-N,C2')iridium(III) (Ir(ppy)₃), bis(2-phenylpyridinato-N,C2')iridium(III) acetylacetonato (Ir(ppy)₂(acac)), bis(1,2-diphenyl-1H-benzimidazolato)iridium(III) acetylacetonato (Ir(pbi)₂(acac)), and bis(benzo[h]quinolinato)iridium(III) acetylacetonato (Ir(bzq)₂(acac)).

Examples of red phosphorescent emitting material usable in the light emitting layer include a metal complex, such as an iridium complex, a platinum complex, a terbium complex, and a europium complex. Examples thereof include an organometallic complex, such as bis[2-(2'-benzo[4,5-α]thienyl)pyridinato-N,C3']iridium(III) acetylacetonato (Ir(btp)2(acac)), bis(1-phenylisoquinolinato-N,C2')iridium(III) acetylacetonato (Ir(piq)2(acac)), (acetylacetonato)bis[2,3-bis(4-fluorophenyl)quinoxalinato]iridium(III) (Ir(Fdpq)₂(acac)), and 2,3,7,8,12,13,17,18-octaethyl-21H,23H-porphyrin platinum(II) (PtOEP).

A rare earth metal complex, such as tris(acetylacetonato) (monophenanthroline)terbium(III) (Tb(acac)₃(Phen)), tris(1,3-diphenyl-1,3-propanedionato)(monophenanthroline)europium(III) (Eu(DBM)₃(Phen)), and tris[1-(2-thenoyl)-3,3,3-trifluoroacetonato](monophenanthroline)europium(III) (Eu(TTA)₃(Phen)), emits light from the rare earth metal ion (electron transition between different multiple states), and therefore, usable as a phosphorescent emitting material.

### Host material for light emitting layer

The light emitting layer may be a layer wherein the above dopant material is dispersed in another material (host material). The compound (1) may be used as a host material and other materials may be usable. The host material preferably has a lowest unoccupied molecular orbital level (LUMO level) higher than that of the dopant material and a highest occupied molecular orbital level (HOMO level) lower than that of the dopant material.

The host material other the compound (1) may include, for example,
(1) a metal complex, such as an aluminum complex, a beryllium complex, and a zinc complex;
(2) a heterocyclic compound, such as an oxadiazole derivative, a benzimidazole derivative, and a phenanthroline derivative;
(3) a fused aromatic compound, such as a carbazole derivative, an anthracene derivative, a phenanthrene derivative, a pyrene derivative, and a chrysene derivative; and
(4) an aromatic amine compound, such as a triarylamine derivative and a fused aromatic polycyclic amine derivative.

Examples thereof include:
a metal complex, such as tris(8-quinolinolato)aluminum(III) (Alq), tris(4-methyl-8-quinolinolato)aluminum(III) (Almq₃), bis(10-hydroxybenzo[h]quinolinato)beryllium(II) (BeBq₂), bis(2-methyl-8-quinolinolato)(4-phenylphenolato)aluminum(III) (BAlq), bis(8-quinolinolato)zinc(II) (Znq), bis[2-(2-benzoxazolyl)phenolato]zinc(II) (ZnPBO), and bis[2-(2-benzothiazolyl)phenolato]zinc(II) (ZnBTZ);
a heterocyclic compound, such as 2-(4-biphenylyl)-5-(4-tert-butylphenyl)-1,3,4-oxadiazole (PBD), 1,3-bis[5-(p-tert-butylphenyl)-1,3,4-oxadiazole-2-yl]benzene (OXD-7), 3-(4-biphenylyl)-4-phenyl-5-(4-tert-butylphenyl)-1,2,4-triazole (TAZ), 2,2',2"-(1,3,5-benzenetriyl)tris(1-phenyl-1H-benzimidazole) (TPBI), bathophenanthroline (BPhen), and bathocuproin (BCP);
a fused aromatic compound, such as 9-[4-(10-phenyl-9-anthryl)phenyl]-9H-carbazole (CzPA), 3,6-diphenyl-9-[4-(10-phenyl-9-anthryl)phenyl]-9H-carbazole (DPCzPA), 9,10-bis(3,5-diphenylphenyl)anthracene (DPPA), 9,10-di(2-naphthyl)anthracene (DNA), 2-tert-butyl-9,10-di(2-naphthyl)anthracene (t-BuDNA), 9,9'-bianthryl (BANT), 9,9'-(stilbene-3,3'-diyl)diphenanthrene (DPNS), 9,9'-(stilbene-4,4'-diyl)diphenanthrene (DPNS2), 33',3"-(benzene-1,3,5-triyl)tripyrene (TPB3), 9,10-diphenylanthracene (DPAnth), and 6,12-dimethoxy-5,11-diphenylchrysene; and
an aromatic amine compound, such as N,N-diphenyl-9-[4-(10-phenyl-9-anthryl)phenyl]-9H-carbazole-3-amine (CzA1PA), 4-(10-phenyl-9-anthryl)triphenylamine (DPhPA), N,9-diphenyl-N-[4-(10-phenyl-9-anthryl)phenyl]-9H-carbazole-3-amine (PCAPA), N,9-diphenyl-N-{4-[4-(10-phenyl-9-anthryl)phenyl]phenyl}-9H-carbazole-3-amine (PCAPBA), N-(9,10-diphenyl-2-anthryl)-N,9-diphenyl-9H-carbazole-3-amine (2PCAPA), 4,4'-bis[N-(1-anthryl)-N-phenylamino]biphenyl (NPB or α-NPD), N,N'-bis(3-methylphenyl)-N,N'-diphenyl-[1,1'-biphenyl]-4,4'-diamine (TPD), 4,4'-bis[N-(9,9-dimethylfluorene-2-yl)-N-phenylamino]biphenyl (DFLDPBi), and 4,4'-bis[N-(spiro-9,9'-bifluorene-2-yl)-N-phenylaminolbiphenyl (BSPB).

The host material may be used alone or in combination of two or more.

In particular, as a host material for a blue fluorescent device, the following anthracene compound is preferably used.

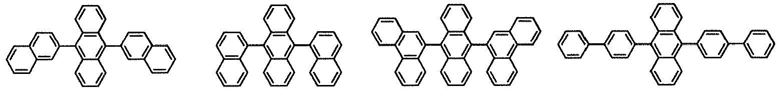

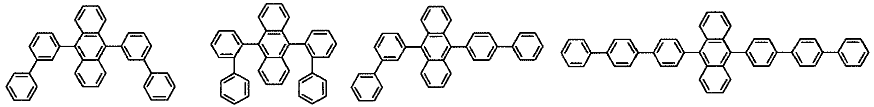

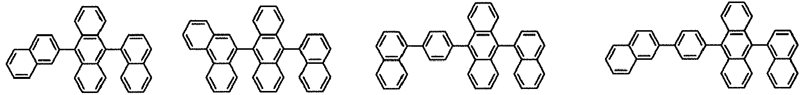

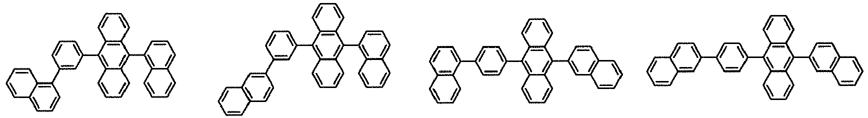

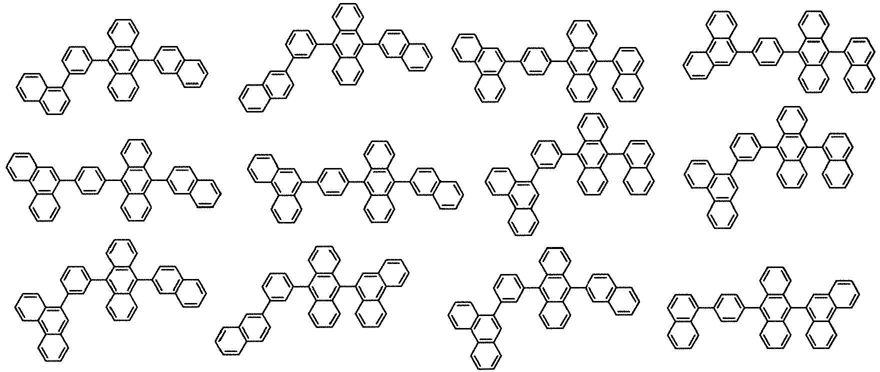

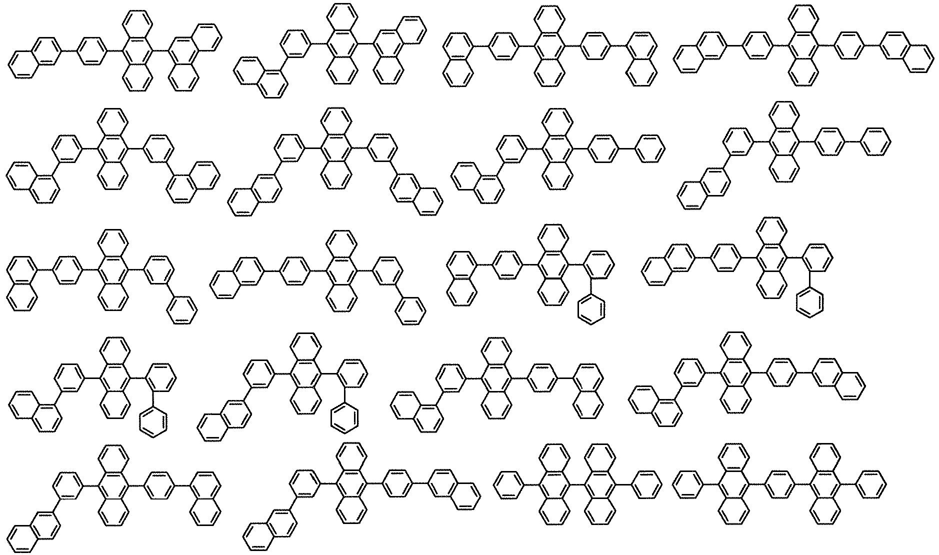

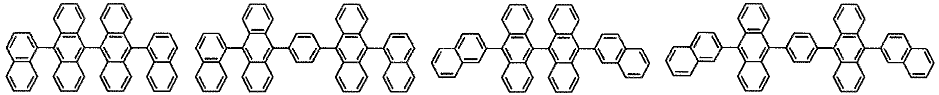

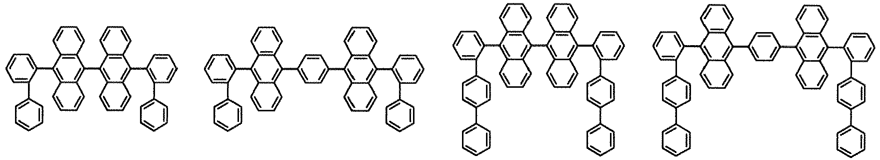

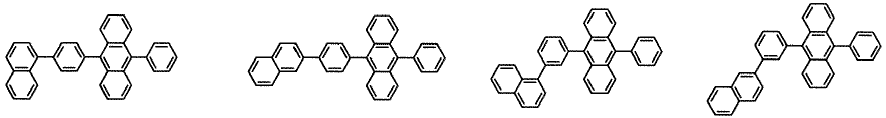

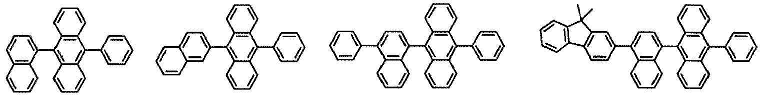

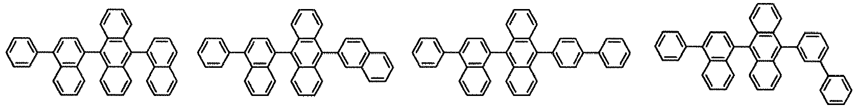

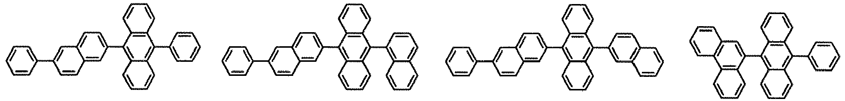

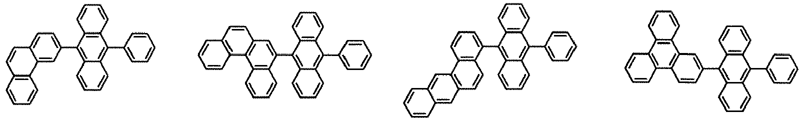

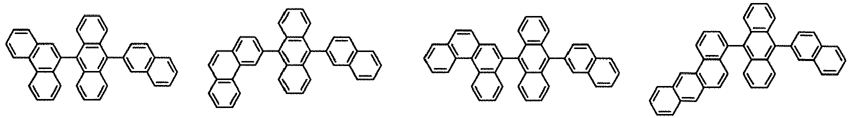

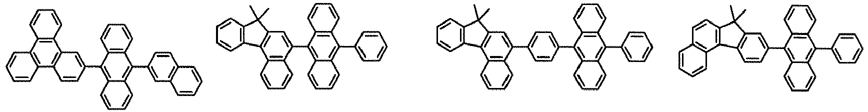

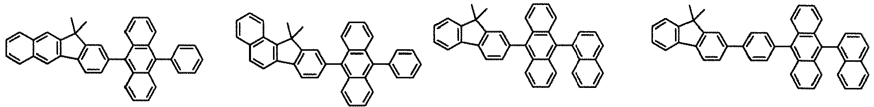

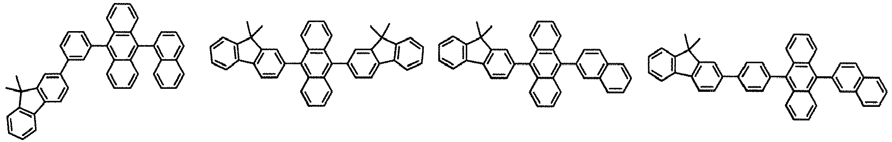

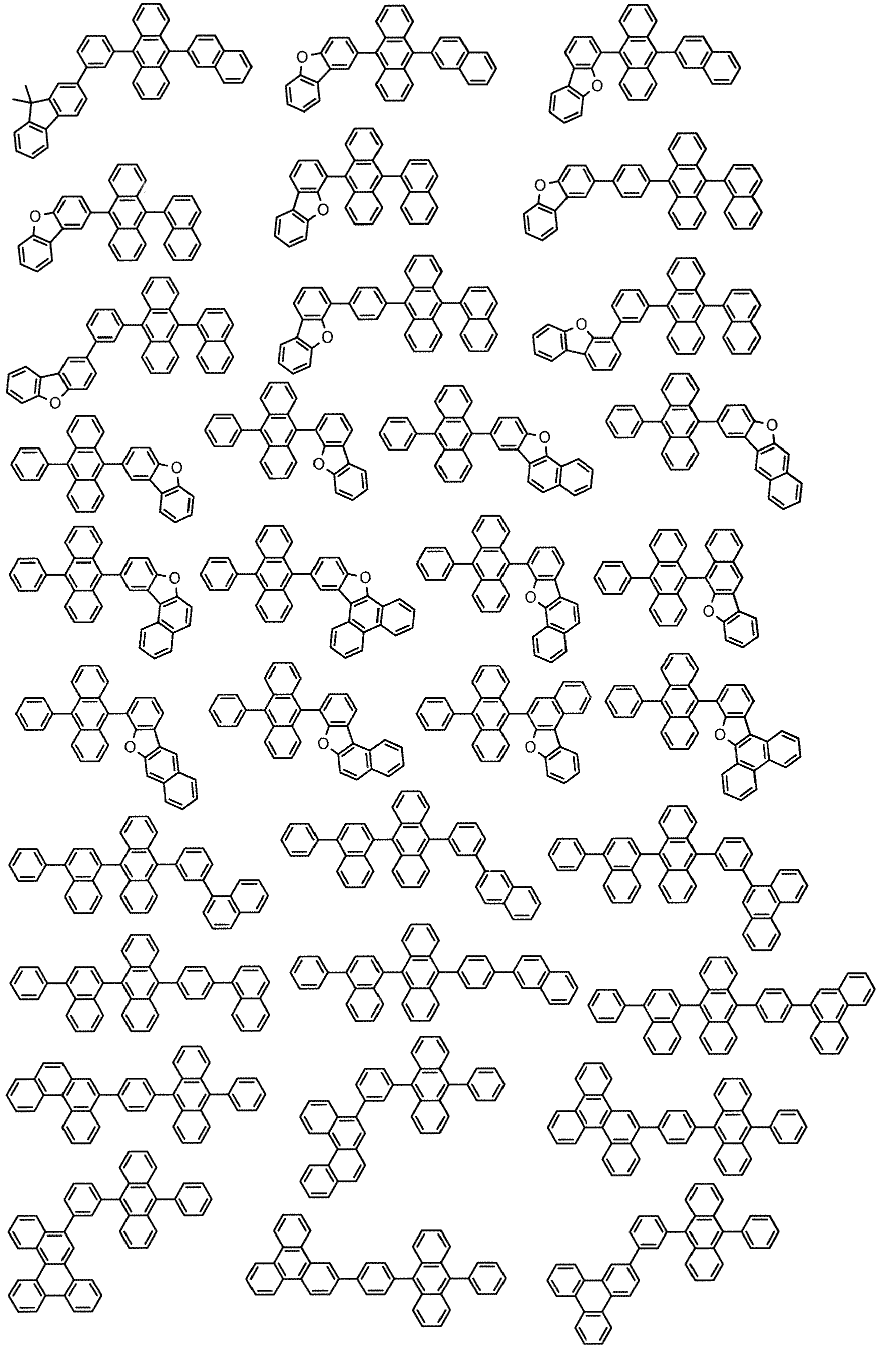

### Electron transporting layer

The electron transporting layer comprises a material having a high electron transporting ability (electron transporting material). The electron transporting layer may be formed, for example, by
(1) a metal complex, such as an aluminum complex, a beryllium complex, and a zinc complex;
(2) a heteroaromatic compound, such as an imidazole derivative, a benzimidazole derivative, an azine derivative, a carbazole derivative, and a phenanthroline derivative; and
(3) a macromolecular compound.

Examples of the metal complex include tris(8-quinolinolato)aluminum (III) (Alq), tris(4-methyl-8-quinolinolato)aluminum (Almq₃), bis(10-hydroxybenzo[h]quinolinato)beryllium (BeBq₂), bis(2-methyl-8-quinolinato)(4-phenylphenolato)aluminum (III) (BAlq), bis(8-quinolinato)zinc(II) (Znq), bis[2-(2-benzoxazolyl)phenolato]zinc(II) (ZnPBO), and bis[2-(2-benzothiazolyl)phenolato]zinc(II) (ZnBTZ).

Examples of the heteroaromatic compound include 2-(4-biphenylyl)-5-(4-tert-butylphenyl)-1,3,4-oxadiazole (PBD), 1,3-bis[5-(p-tert-butylphenyl)-1,3,4-oxadiazole-2-yl]benzene (OXD-7), 3-(4-tert-butylphenyl)-4-phenyl-5-(4-biphenylyl)-1,2,4-triazole (TAZ), 3-(4-tert-butylphenyl)-4-(4-ethylphenyl)-5-(4-biphenylyl)-1,2,4-triazole (p-EtTAZ), bathophenanthroline (BPhen), bathocuproine (BCP), and 4,4'-bis(5-methylbenzoxazole-2-yl)stilbene (BzOs).

Examples of the macromolecular compound include poly[(9,9-dihexylfluorene-2,7-diyl)-co-(pyridine-3,5-diyl)] (PF-Py), and poly[(9,9-dioctylfluorene-2,7-diyl)-co-(2,2'-bipyridine-6,6'-diyl)] (PF-BPy).

The above compounds have an electron mobility of 10⁻⁶ cm²/Vs or more. Materials other than those mentioned above are also usable in the electron transporting layer if their electron transporting ability is higher than their hole transporting ability.

The electron transporting layer may be a single layer or a multi-layer of two or more layers. For example, the electron transporting layer may be a two-layered structure comprising a first electron transporting layer (anode side) and a second electron transporting layer (cathode side). Each of two or more electron transporting layers is formed by the electron transporting material mentioned above.

### Electron injecting layer

The electron injecting layer is a layer comprising a material having a high electron injecting ability, for example, an alkali metal, an alkaline earth metal, and a compound of these metals, such as lithium (Li), cesium (Cs), calcium (Ca), lithium fluoride (LiF), cesium fluoride (CsF), calcium fluoride (CaF₂), and an oxide of lithium (LiOₓ). In addition, an electron transporting material which is doped with an alkali metal, an alkaline earth metal or a compound thereof, for example, Alq doped with magnesium (Mg), is also usable. By using such a material, electrons are efficiently injected from the cathode.

A composite material comprising an organic compound and an electron donor is also usable in the electron injecting layer. Such a composite material is excellent in the electron injecting ability and the electron transporting ability, because the organic compound receives electrons from the electron donor. The organic compound is preferably a compound excellent in transporting the received electrons. Examples thereof include the materials for the electron transporting layer mentioned above, such as the metal complex and the aromatic heterocyclic compound. Any compound capable of giving its electron to the organic compound is usable as the electron donor. Preferred examples thereof are an alkali metal, an alkaline earth metal, and a rare earth metal, such as lithium, cesium, magnesium, calcium, erbium, and ytterbium; an alkali metal oxide and an alkaline earth metal oxide, such as, lithium oxide, calcium oxide, and barium oxide; a Lewis base, such as magnesium oxide; and an organic compound, such as tetrathiafulvalene (TTF).

### Cathode

The cathode is formed preferably from a metal, an alloy, an electrically conductive compound, or a mixture thereof, each having a small work function, for example, a work function of 3.8 eV or less. Examples of the material for the cathode include an element belonging to a group 1 or group 2 of the periodic table, i.e., an alkali metal, such as lithium (Li) and cesium (Cs), an alkaline earth metal, such as magnesium (Mg), calcium (Ca), and strontium (Sr), an alloy containing these metals (for example, MgAg and AlLi), a rare earth metal, such as europium (Eu) and ytterbium (Yb), and an alloy containing a rare earth metal.

The alkali metal, the alkaline earth metal, and the alloy thereof is made into the cathode by a vacuum vapor deposition or a sputtering method. A coating method and an inkjet method are usable when a silver paste is used.

When the electron injecting layer is formed, the material for the cathode is selected irrespective of whether the work function is large or small and various electroconductive materials, such as Al, Ag, ITO, graphene, and indium oxide-tin oxide doped with silicon or silicon oxide, are usable. These electroconductive materials are made into films by a sputtering method, an inkjet method, and a spin coating method.

### Insulating layer

Since electric field is applied to the ultra-thin films of organic EL devices, the pixel defects due to leak and short circuit tends to occur. To prevent the defects, an insulating thin film layer may be interposed between the pair of electrodes.

Examples of the material for the insulating layer include aluminum oxide, lithium fluoride, lithium oxide, cesium fluoride, cesium oxide, magnesium oxide, magnesium fluoride, calcium oxide, calcium fluoride, aluminum nitride, titanium oxide, silicon oxide, germanium oxide, silicon nitride, boron nitride, molybdenum oxide, ruthenium oxide, and vanadium oxide. These materials may be used in combination or may be used in each layer of stacked layers.

### Space layer

For example, in an organic EL device having a fluorescent emitting layer and a phosphorescent emitting layer, a space layer is disposed between the fluorescent emitting layer and the phosphorescent emitting layer to prevent the diffusion of excitons generated in the phosphorescent emitting layer to the fluorescent emitting layer or to control the carrier (charge) balance. The space layer may be disposed between two or more phosphorescent emitting layers.

Since the space layer is disposed between the light emitting layers, a material combining the electron transporting ability and the hole transporting ability is preferably used for forming the space layer. To prevent the diffusion of triplet energy in the adjacent phosphorescent emitting layer, the triplet energy of the material for the space layer is preferably 2.6 eV or more. The materials described with respect to the hole transporting layer are usable as the material for the space layer.

### Blocking layer

A blocking layer, such as an electron blocking layer, a hole blocking layer, and an exciton blocking layer, may be provided in the portion adjacent to the light emitting layer. The electron blocking layer is a layer which prevents the diffusion of electrons from the light emitting layer to the hole transporting layer. The hole blocking layer is a layer which prevents the diffusion of holes from the light emitting layer to the electron transporting layer. The exciton blocking layer prevents the diffusion of excitons generated in the light emitting layer to adjacent layers and has a function of confining the excitons in the light emitting layer.

Each layer of the organic EL device is formed by a known method, such as a vapor deposition method and a coating method. For example, each layer is formed by a known vapor deposition method, such as a vacuum vapor deposition method and a molecular beam evaporation method (MBE method), and a known coating method using a solution of a compound for forming a layer, such as a dipping method, a spin coating method, a casting method, a bar coating method, and a roll coating method.

The thickness of each layer is not particularly limited and preferably 5 nm to 10 µm, more preferably 10 nm to 0.2 µm, because an excessively small thickness may cause defects such as pin holes and an excessively large thickness may require a high driving voltage.

The organic EL device can be used in an electronic device, for example, as display parts, such as organic EL panel module, display devices of television sets, mobile phones, personal computer, etc., and light emitting sources of lighting equipment and vehicle lighting equipment.

### EXAMPLES

The present invention will be described below in more details with reference to the examples. However, it should be noted that the scope of the invention is not limited thereto.

### Intermediate Synthesis A: synthesis of intermediate A

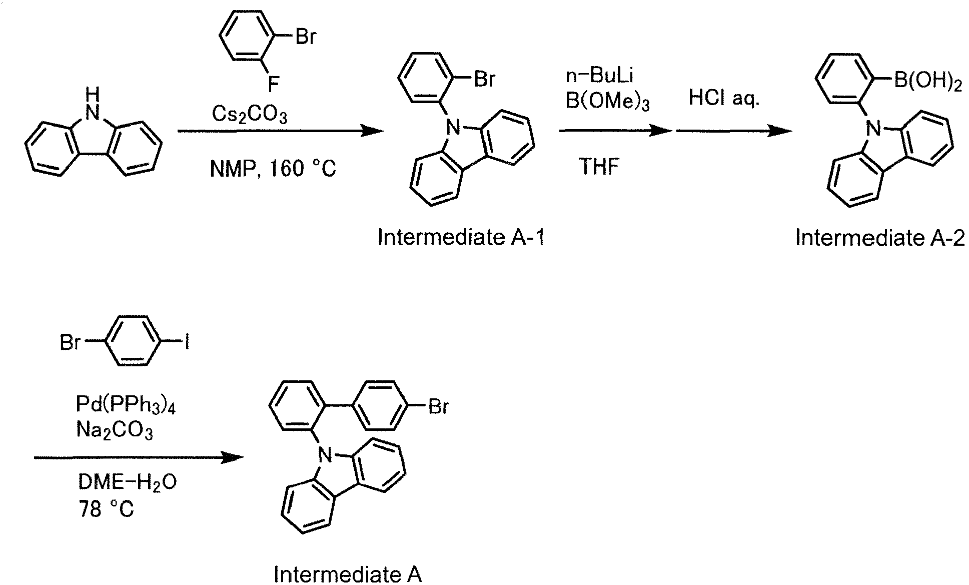

### (A-1) Synthesis of intermediate A-1

Into a solution of carbazole (10 g, 59.8 mmol) and cesium carbonate (39.0 g, 120 mmol) in N-methylpyrrolidone (NMP, 59.8 mL), 1-bromo-2-fluorobenzene (7.80 mL, 71.8 mmol) was added and the resultant mixture was stirred under heating at 160 °C for 16 h. The reaction solution was cooled to room temperature, toluene was added, and the insolubles were removed by filtration. The obtained solution was washed with water, dried over anhydrous sodium sulfate, and then purified by silica gel column chromatography. The obtained residue was crystallized to obtain intermediate A-1 (18.3 g) in a yield of 94%.

### (A-2) Synthesis of intermediate A-2

Under argon atmosphere, a solution of intermediate A-1 (8.0 g, 24.8 mmol) in tetrahydrofuran (THF, 124 mL) was cooled in a dry ice/acetone bath. Into the cooled solution, a 1.6 M solution of n-butyllithium in hexane (17.1 mL, 27.3 mmol) was added dropwise and then stirred for 2 h. After adding a solution of trimethyl borate (3.33 mL, 29.8 mmol) in THF (10 mL) dropwise and stirred for one hour, the solution was removed from the dry ice/acetone bath and returned to room temperature. The reaction solution was cooled in an iced water bath. After adding a 2 M hydrochloric acid, the solution was returned to room temperature and stirred for one hour. The obtained solution was extracted with ethyl acetate and the organic layer was washed with water, dried over anhydrous sodium sulfate, and concentrated under reduced pressure. The obtained residue was crystallized to obtain intermediate A-2 (4.91 g) in a yield of 69%.

### (A-3) Synthesis of intermediate A

Under argon atmosphere, a solution of intermediate A-2 (4.16 g, 14.5 mmol), 1-bromo-4-iodobenzene (4.92 g, 17.4 mmol), tetrakis(triphenylphosphine)palladium(0) (0.324 g, 0.28 mmol), and sodium carbonate (3.07 g, 29 mmol) in ethylene glycol dimethyl ether (DME, 40 mL)/water (7 mL) was stirred at 78 °C for 18 h. After cooling to room temperature, methanol was added and the generated solid was collected by filtration. The obtained solid was purified by silica gel column chromatography and recrystallization to obtain intermediate A (3.98 g) in a yield of 69%.

### Synthesis Example 1: synthesis of compound 1

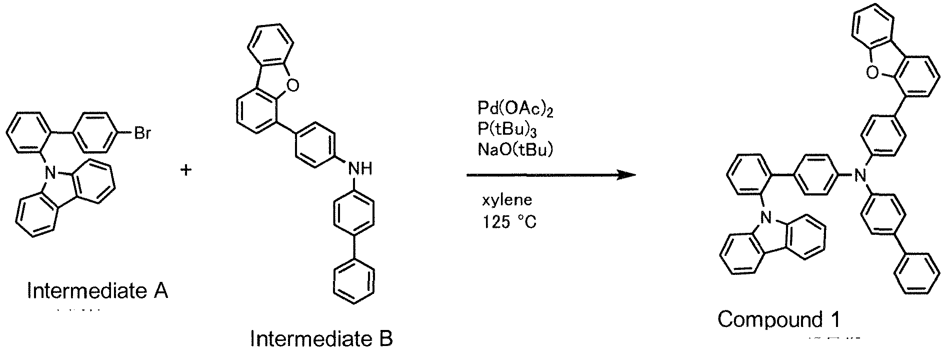

Under argon atmosphere, a mixture of intermediate A (3.18 g, 8.0 mmol), intermediate B (3.29 g, 8.0 mmol) synthesized in accordance with the method described in WO 2007/125714, palladium acetate (II) (0.036 g, 0.16 mmol), tri-t-butylphosphine (0.065 g, 0.32 mmol), sodium t-butoxide (0.93 g, 9.6 mmol), and xylene (50 mL) was stirred at 125 °C for 2.5 h. After cooling the reaction solution to room temperature, the solution was stirred upon adding methanol and the precipitated solid was collected by filtration. The obtained solid was purified by silica gel column chromatography and recrystallization to obtain a white solid (3.14 g) in a yield of 54%.

The obtained compound was identified as compound 1 by the result of mass spectroscopic analysis of m/e = 728 to the molecular weight of 728.28.

### Synthesis Example 2: synthesis of compound 2

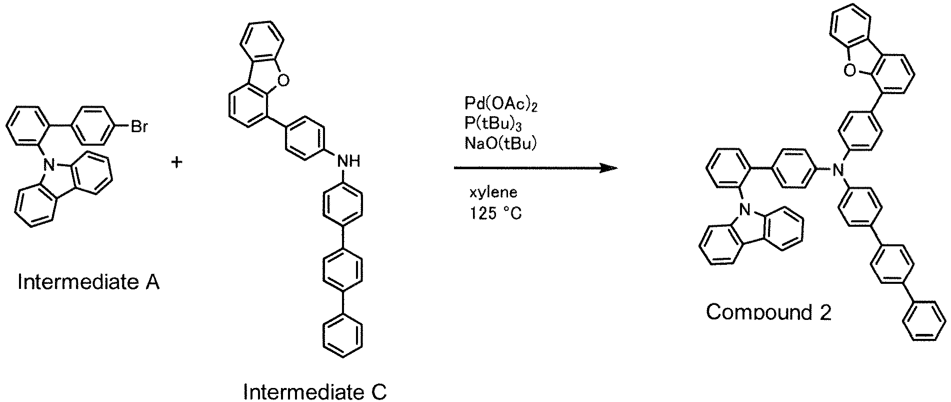

The procedures of Synthesis Example 1 were repeated except for using intermediate C synthesized in accordance with the method described in WO 2010/061824 in place of intermediate B to obtain a compound.

The obtained compound was identified as compound 2 by the result of mass spectroscopic analysis of m/e = 804 to the molecular weight of 804.31.

### Intermediate Synthesis D: synthesis of intermediate D

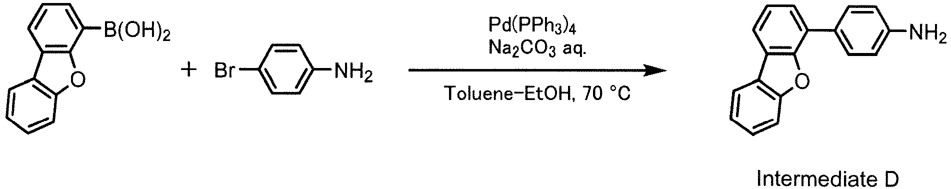

Under argon atmosphere, a mixture of 4-bromoaniline (23.7 g, 138 mmol), dibenzofuran-4-ylboronic acid (29.2 g, 138 mmol), tetrakis(triphenylphosphine)palladium(0) (3.18 g, 2.75 mmol), a 2 M aqueous solution of sodium carbonate (138 mL), toluene (300 mL), and ethanol (100 mL) was stirred at 70 °C for 2 h. After returning to room temperature, the reaction solution was extracted with toluene. The toluene layer was dried over anhydrous magnesium sulfate, filtered, and concentrated under reduced pressure. The obtained residue was purified by silica gel column chromatography and recrystallization to obtain intermediate D (20.9 g) in a yield of 58%.

### Synthesis Example 3: synthesis of compound 3

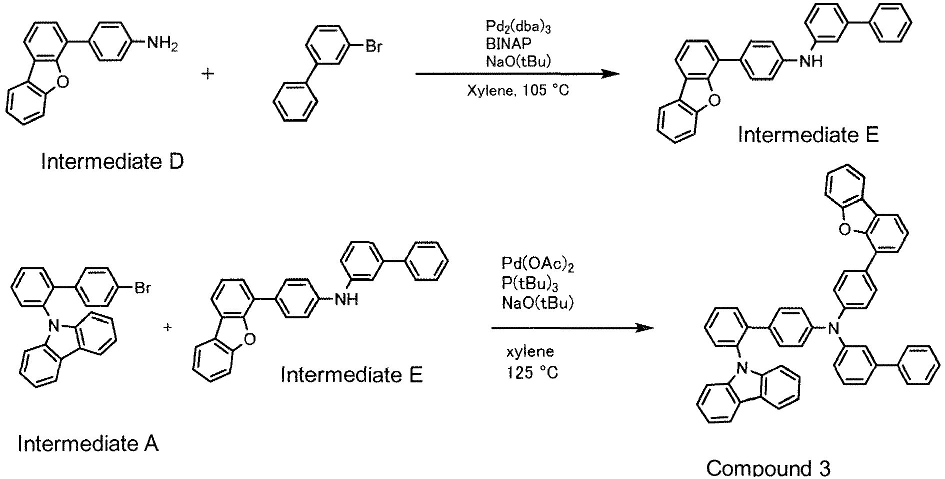

### (3-1) Synthesis of intermediate E

Under argon atmosphere, a solution of intermediate D (2.09 g, 8.06 mmol) and 3-bromobiphenyl (1.45 g, 6.2 mmol) in xylene (35 mL) was heated to 85 °C. After adding tris(dibenzylideneacetone)dipalladium(0) (85.2 mg, 0.093 mmol), 2,2'-bis(diphenylphosphino)-1,1'-binaphthyl (BINAP) (116 mg, 0.186 mmol), and sodium t-butoxide (1.19 g, 12.4 mmol), the solution was heated to 105 °C and stirred for 16 h. After returning to room temperature, water was added and the reaction solution was phase-separated. The organic layer was concentrated under reduced pressure and the residue was purified by column chromatography to obtain intermediate E (1.66 g) in a yield of 65%.

### (3-2) Synthesis of compound 3

The procedures of Synthesis Example 1 were repeated except for using intermediate E in place of intermediate B to obtain a compound.

The obtained compound was identified as compound 3 by the result of mass spectroscopic analysis of m/e = 728 to the molecular weight of 728.28.

### Synthesis Example 4: synthesis of compound 4

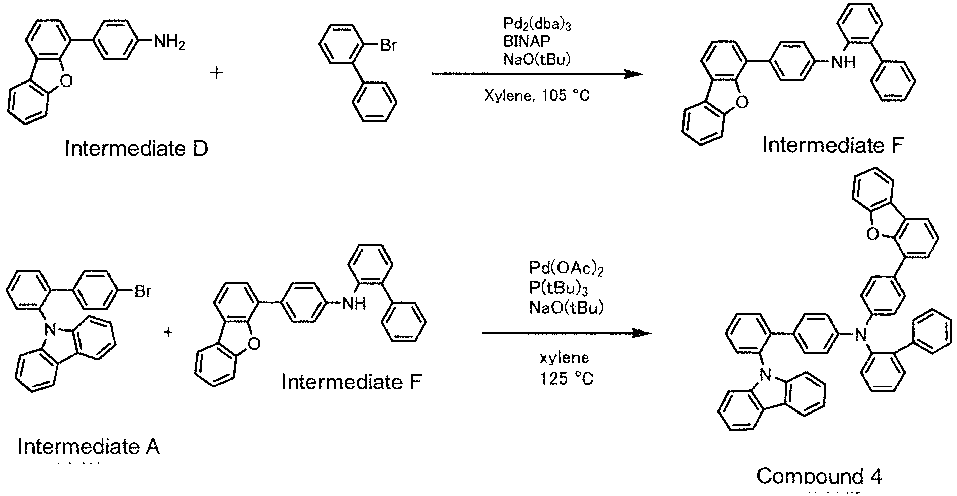

### (4-1) Synthesis of intermediate F

The procedures of (3-1) of Synthesis Example 3 were repeated except for using 2-bromobiphenyl in place of 3-bromobiphenyl to obtain intermediate F.

### (4-2) Synthesis of compound 4

The procedures of Synthesis Example 1 were repeated except for using intermediate F in place of intermediate B to obtain a compound.

The obtained compound was identified as compound 4 by the result of mass spectroscopic analysis of m/e = 728 to the molecular weight of 728.28.

### Synthesis Example 5: synthesis of compound 5

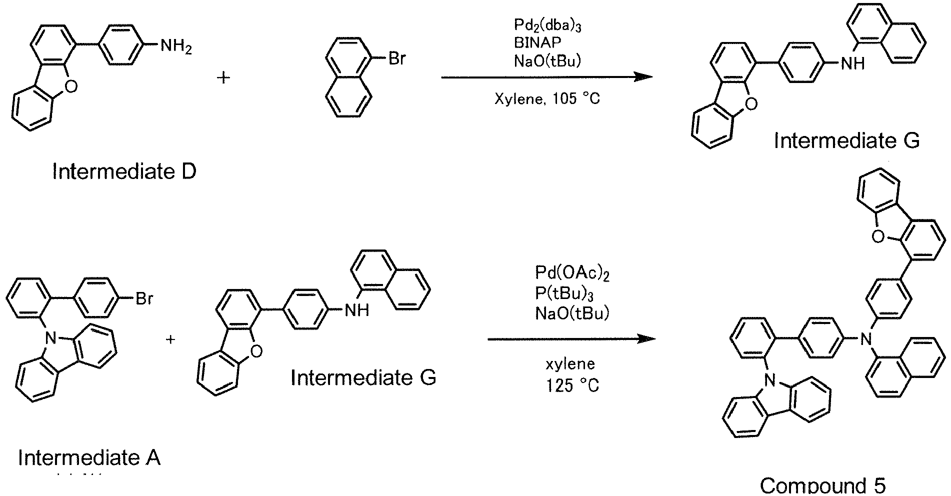

### (5-1) Synthesis of intermediate G

The procedures of (3-1) of Synthesis Example 3 were repeated except for using 1-bromonaphthalene in place of 3-bromobiphenyl to obtain intermediate G.

### (5-2) Synthesis of compound 5

The procedures of Synthesis Example 1 were repeated except for using intermediate G in place of intermediate B to obtain a compound.

The obtained compound was identified as compound 5 by the result of mass spectroscopic analysis of m/e = 702 to the molecular weight of 702.27.

### Synthesis Example 6: synthesis of compound 6

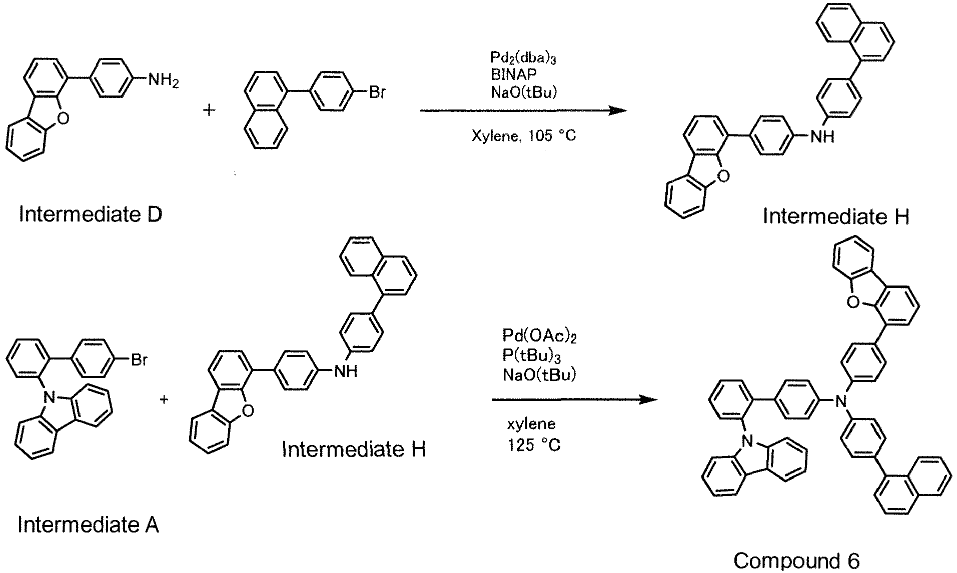

### (6-1) Synthesis of intermediate H

The procedures of (3-1) of Synthesis Example 3 were repeated except for using 1-(4-bromophenyl)naphthalene in place of 3-bromobiphenyl to obtain intermediate H.

### (6-2) Synthesis of compound 6

The procedures of Synthesis Example 1 were repeated except for using intermediate H in place of intermediate B to obtain a compound.

The obtained compound was identified as compound 6 by the result of mass spectroscopic analysis of m/e = 778 to the molecular weight of 778.30.

### Synthesis Example 7: synthesis of compound 7

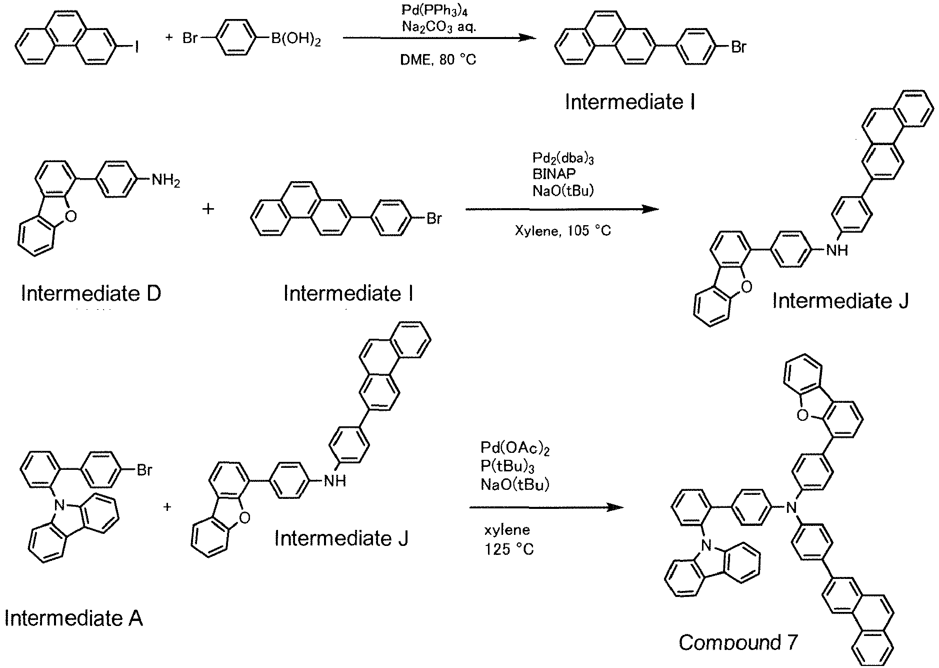

### (7-1) Synthesis of intermediate I

Under nitrogen atmosphere, a mixture of 2-iodophenanthrene synthesized in accordance with the method described in WO 2009/116628 (3.04 g, 10.0 mmol), 4-bromophenylboronic acid (2.00 g, 10.0 mmol), tetrakis(triphenylphosphine)palladium(0) (0.231 g, 0.200 mmol), a 2 M aqueous solution of sodium carbonate (60 mL, 120 mmol), 1,2-dimethoxyethane (DME) (50 mL), and water (60 mL) was stirred at 80 °C for 5 h. The reaction solution was cooled to room temperature. The precipitated crystal was collected by filtration, washed with hexane and then water, and dried to obtain the aimed intermediate I (3.28 g) in a yield of 99%.

### (7-2) Synthesis of intermediate J

The procedures of (3-1) of Synthesis Example 3 were repeated except for using intermediate I in place of 3-bromobiphenyl to obtain intermediate J.

### (7-3) Synthesis of compound 7

The procedures of Synthesis Example 1 were repeated except for using intermediate J synthesized in (7-2) in place of intermediate B to obtain a compound.

The obtained compound was identified as compound 7 by the result of mass spectroscopic analysis of m/e = 828 to the molecular weight of 828.31.

### Synthesis Example 8: synthesis of compound 8

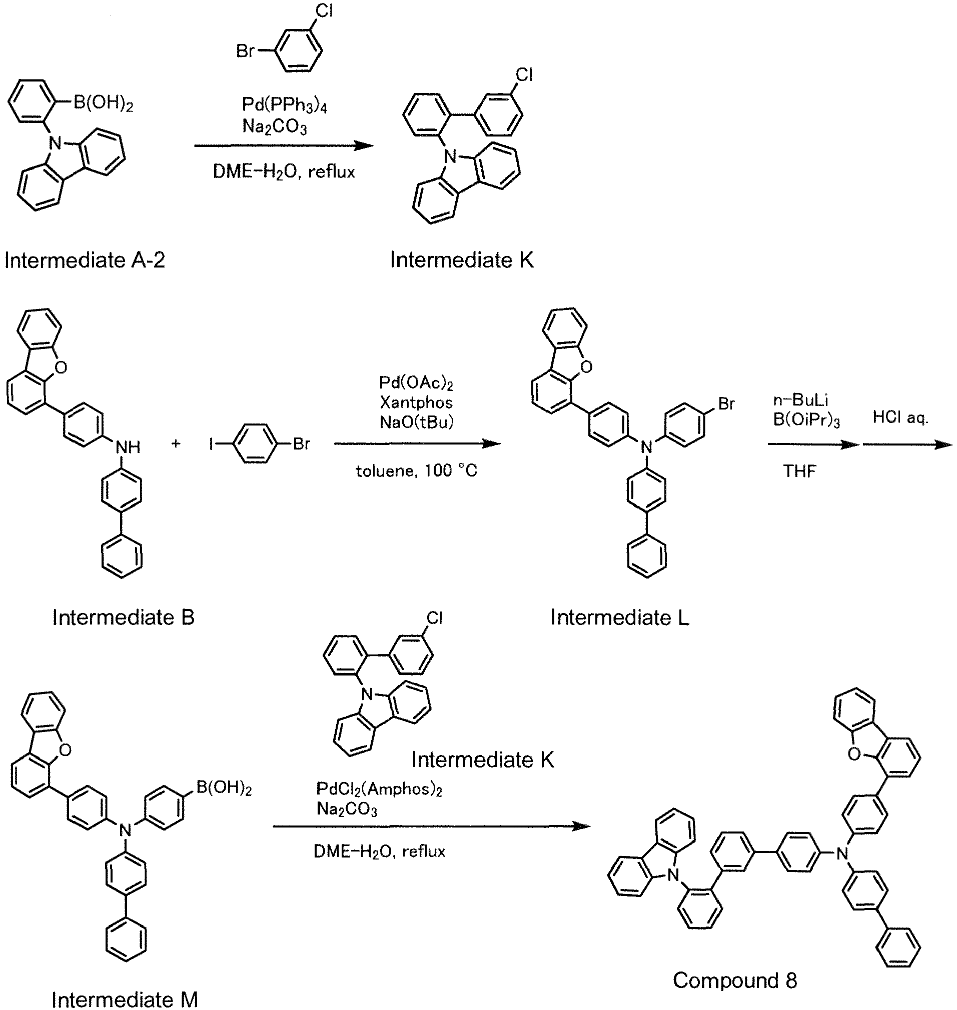

### (8-1) Synthesis of intermediate K

Under argon atmosphere, a mixture of intermediate A-2 synthesized in accordance with the method of (A-2) of Intermediate Synthesis A (2.18 g, 7.59 mmol),1-bromo-3-chlorobenzene (1.60 g, 8.36 mmol), tetrakis(triphenylphosphine)palladium(0) (0.17 g, 0.147 mmol), a 2 M aqueous solution of sodium carbonate (12 mL, 24 mmol), and 1,2-dimethoxyethane (DME) (40 mL) was refluxed for 7 h. The reaction solution was cooled to room temperature and extracted with ethyl acetate upon adding water. The ethyl acetate layer was washed with water and then a saturated brine, dried over anhydrous magnesium sulfate, and filtered. The filtrate was concentrated under reduced pressure and the obtained residue was purified by silica gel column chromatography to obtain intermediate K (2.52 g) in a yield of 94%.

### (8-2) Synthesis of intermediate L

Under argon atmosphere, a mixture of intermediate B synthesized in accordance with the method described in WO 2007/125714 (12.35 g, 30.0 mmol), 1-bromo-4-iodobenzene (8.49 g, 30.0 mmol), palladium acetate (II) (0.07 g, 0.313 mmol), Xantphos (0.17 g, 0.294 mmol), sodium t-butoxide (4.32 g, 45.0 mmol), and toluene (150 mL) was stirred at 100 °C for 23 h. The reaction solution was cooled to room temperature and extracted with ethyl acetate upon adding water. The obtained organic layer was filtered through celite. The filtrate was washed with water and then a saturated brine and dried over anhydrous magnesium sulfate. The dried solution was filtered and the filtrate was concentrated under reduced pressure. The obtained residue was purified by silica gel column chromatography to obtain intermediate L (14.65 g) in a yield of 86%.

### (8-3) Synthesis of intermediate M

Under argon atmosphere, a solution of intermediate L (8.4 g, 14.83 mmol) in THF (110 mL) was cooled in a dry ice/methanol bath. After adding a 1.5 M solution of n-butyllithium in hexane (12 mL, 18.6 mmol) dropwise, the solution was stirred for one hour. After adding triisopropyl borate (11 mL, 47.7 mmol) dropwise, the reaction solution was stirred for one hour and further stirred at room temperature for one hour. A 1 M hydrochloric acid (20 mL) and water (110 mL) were added to the reaction solution that was then stirred for 30 min. The obtained reaction solution was phase-separated and extracted with ethyl acetate. The organic layer was washed with a saturated brine, dried over anhydrous magnesium sulfate, and filtered. The filtrate was concentrated under reduced pressure and the obtained residue was washed with ethyl acetate to obtain intermediate M (3.74 g) in a yield of 48%.

### (8-4) Synthesis of compound 8

Under argon atmosphere, a mixture of intermediate M (2.33 g, 4.38 mmol), intermediate K (1.55 g, 4.38 mmol), PdCl₂(Amphos)₂ (0.06 g, 0.085 mmol), 1,2-dimethoxyethane (DME) (37 mL), and a 2 M aqueous solution of sodium carbonate (7 mL, 14 mmol) was stirred for 5 h. The reaction solution was cooled to room temperature. The precipitated solid was collected by filtration and washed with DME and then water. The obtained residue was purified by silica gel column chromatography and recrystallization to obtain a white solid (1.60 g) in a yield of 45%.

The obtained compound was identified as compound 8 by the result of mass spectroscopic analysis of m/e = 804 to the molecular weight of 804.31.

### Example 1

### Production of organic EL device

A 25 mm × 75 mm × 1.1 mm glass substrate having ITO transparent electrode (product of Geomatec Company) was ultrasonically cleaned in isopropyl alcohol for 5 min and then UV/ozone cleaned for 30 min. The thickness of ITO transparent electrode was 130 nm.

The cleaned glass substrate having the ITO transparent electrode was mounted to a substrate holder of a vacuum vapor deposition apparatus. First, the compound HI-1 was vapor-deposited so as to cover the ITO transparent electrode to form a hole injecting layer with a thickness of 5 nm.

On the hole injecting layer, the compound HT-1 (first hole transporting layer material) was vapor-deposited to form a first hole transporting layer with a thickness of 80 nm.

On the first hole transporting layer, the compound 1 synthesized in Synthesis Example 1 (second hole transporting layer material) was vapor-deposited to form a second hole transporting layer with a thickness of 10 nm.

Then, on the second hole transporting layer, the compound BH-1 (host material) and the compound BD-1 (dopant material) were vapor co-deposited to form a light emitting layer with a thickness of 25 nm. The concentration of the compound BD-1 in the light emitting layer was 4.0% by mass.

On the light emitting layer, the compound ET-1 was vapor-deposited to form a first electron transporting layer with a thickness of 10 nm.

On the first electron transporting layer, the compound ET-2 was vapor-deposited to form a second electron transporting layer with a thickness of 15 nm.

On the second electron transporting layer, LiF was vapor-deposited to form an electron injecting electrode (cathode) with a thickness of 1 nm.

Then, metallic Al was vapor-deposited on the LiF film to form a metallic cathode with a thickness of 80 nm.

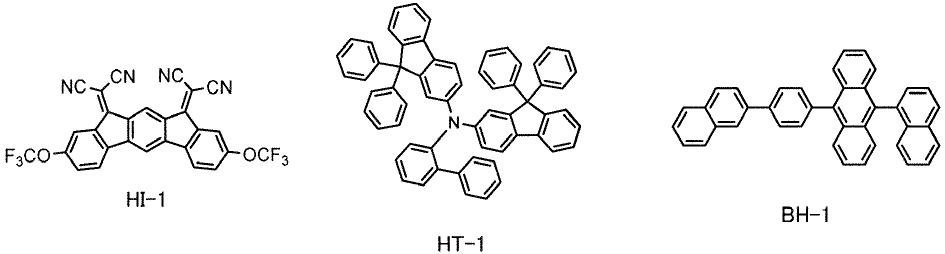

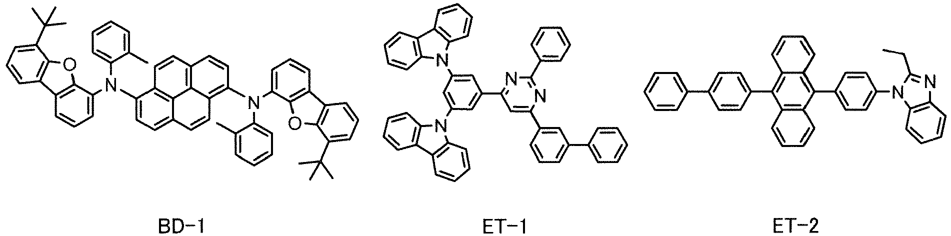

### Measurement of device lifetime

The organic EL device thus produced was operated by a direct current at a current density of 50 mA/cm² to measure the time taken until the luminance was reduced to 95% of the initial luminance. The result was taken as 95% lifetime (LT95) and shown in Table 1.

### Examples 2 to 8

Each organic EL device was produced in the same manner as in Example 1 except for using each of the compounds 2 to 8 as the second hole transporting layer material in place of the compound 1. Each organic EL device thus produced was measured for 95% lifetime (LT95) in the same manner as in Example 1. The results are shown in Table 1.

### Comparative Examples 1 and 2

Each organic EL device was produced in the same manner as in Example 1 except for using the comparative compound 1 (compound described in Patent Literature 3) or the comparative compound 2 (compound described in Patent Literature 4) as the second hole transporting layer material. Each organic EL device thus produced was measured for 95% lifetime (LT95) in the same manner as in Example 1. The results are shown in Table 1.

**Table 1**

| | Material of second hole transporting layer | LT95 (h) |
|---|---|---|
| Example 1 | Compound 1 | 140 |
| Example 2 | Compound 2 | 140 |
| Example 3 | Compound 3 | 120 |
| Example 4 | Compound 4 | 130 |
| Example 5 | Compound 5 | 120 |
| Example 6 | Compound 6 | 140 |
| Example 7 | Compound 7 | 145 |
| Example 8 | Compound 8 | 145 |
| Comparative example 1 | Comparative compound 1 | 85 |
| Comparative example 2 | Comparative compound 2 | 90 |

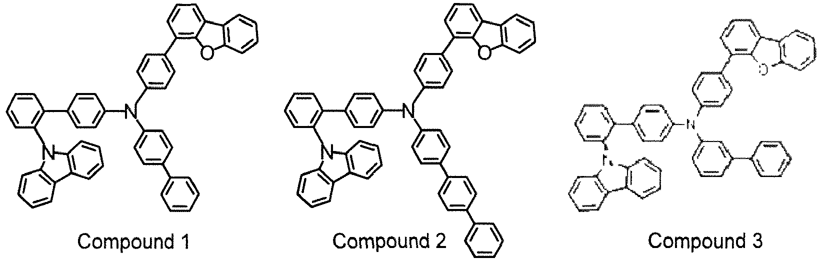

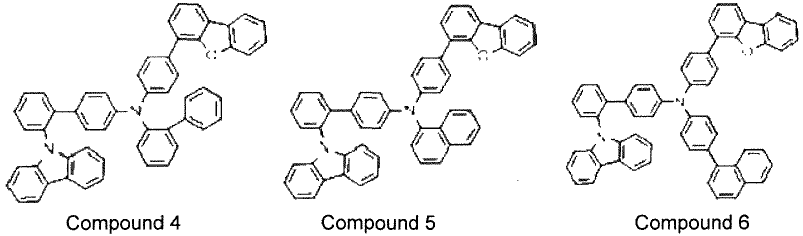

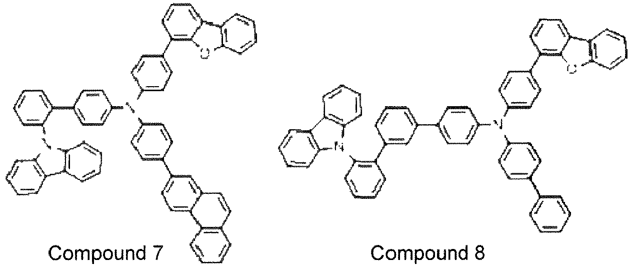

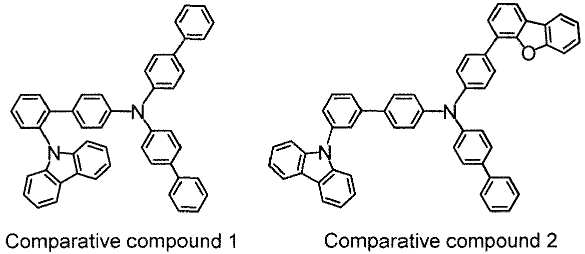

The compounds 1 to 8 of the invention meet both the requirements: Structural requirement A: 4-dibenzofuranyl group; and Structural requirement B: biphenylyl group having a N-carbazolyl group on the terminal benzene ring at its o-position.

The comparative compound 1 does not meet the structural requirement A. Since the N-carbazolyl group is bonded to the terminal benzene ring of the biphenylyl group at its m-position, the comparative compound 2 does not meet the structural requirement B.

As seen from Table 1, the compounds 1 to 8 of the invention which meet both the structural requirements A and B have device lifetimes extremely improved as compared with the comparative compounds 1 and 2 that meet only one the structural requirements A and B.

### REFERENCE SIGNS LIST

1, 11: Organic EL device
2: Substrate
3: Anode
4: Cathode
5: Light emission layer
6: Hole transporting region (hole transporting layer)
6a: Firs hole transporting layer
6b: Second hole transporting layer
7: Electron transporting region (electron transporting layer)
7a: First electron transporting layer
7b: Second electron transporting layer
10, 20: Emission unit

## Claims

1. A compound represented by formula (1): 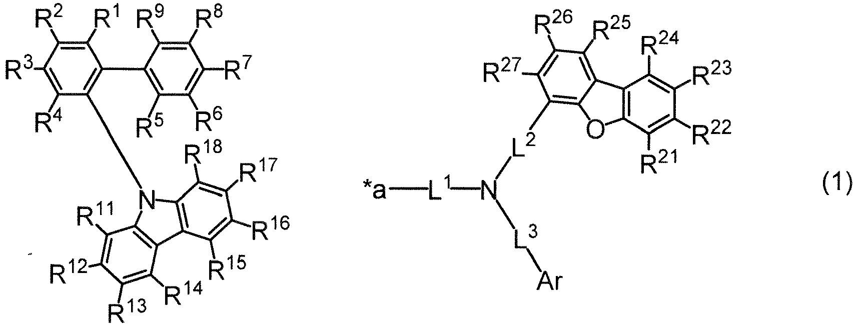 wherein:
one of R⁷, R⁸, and R⁹ is a single bond bonded to *a;
R¹ to R⁶ and R⁷ to R⁹ not the single bond bonded to *a are each independently a hydrogen atom or a substituent,
wherein the substituent is selected from the group consisting of a halogen atom, a cyano group, a nitro group, a substituted or unsubstituted alkyl group having 1 to 30 carbon atoms, a substituted or unsubstituted cycloalkyl group having 3 to 30 ring carbon atoms, a substituted or unsubstituted aryl group having 6 to 30 ring carbon atoms, a substituted or unsubstituted aralkyl group having 7 to 36 carbon atoms, a substituted or unsubstituted alkoxy group having 1 to 30 carbon atoms, a substituted or unsubstituted aryloxy group having 6 to 30 ring carbon atoms, a mono, di or tri-substituted silyl group having a substituent selected from a substituted or unsubstituted alkyl group having 1 to 30 carbon atoms and a substituted or unsubstituted aryl group having 6 to 30 ring carbon atoms, a substituted or unsubstituted haloalkyl group having 1 to 30 carbon atoms, a substituted or unsubstituted haloalkoxy group having 1 to 30 carbon atoms, and a substituted or unsubstituted heteroaryl group having 5 to 30 ring atoms,
provided that adjacent two selected from R¹ to R⁶ and R⁷ to R⁹ not the single bond bonded to *a are each independently a hydrogen atom or the substituent, or the adjacent two are bonded to each other to form a ring structure;
R¹¹ to R¹⁸ are each independently a hydrogen atom or a substituent,
wherein the substituent is selected from the group consisting of a halogen atom, a cyano group, a nitro group, a substituted or unsubstituted alkyl group having 1 to 30 carbon atoms, a substituted or unsubstituted cycloalkyl group having 3 to 30 ring carbon atoms, a substituted or unsubstituted aryl group having 6 to 30 ring carbon atoms, a substituted or unsubstituted aralkyl group having 7 to 36 carbon atoms, a substituted or unsubstituted alkoxy group having 1 to 30 carbon atoms, a substituted or unsubstituted aryloxy group having 6 to 30 ring carbon atoms, a mono, di or tri-substituted silyl group having a substituent selected from a substituted or unsubstituted alkyl group having 1 to 30 carbon atoms and a substituted or unsubstituted aryl group having 6 to 30 ring carbon atoms, a substituted or unsubstituted haloalkyl group having 1 to 30 carbon atoms, a substituted or unsubstituted haloalkoxy group having 1 to 30 carbon atoms, and a substituted or unsubstituted heteroaryl group having 5 to 30 ring atoms,
provided that adjacent two selected from R¹¹ to R¹⁸ are each independently a hydrogen atom or the substituent, or the adjacent two are bonded to each other to form a ring structure;
R²¹ to R²⁷ are each independently a hydrogen atom or a substituent,
wherein the substituent is selected from the group consisting of a halogen atom, a cyano group, a nitro group, a substituted or unsubstituted alkyl group having 1 to 30 carbon atoms, a substituted or unsubstituted cycloalkyl group having 3 to 30 ring carbon atoms, a substituted or unsubstituted aryl group having 6 to 30 ring carbon atoms, a substituted or unsubstituted aralkyl group having 7 to 36 carbon atoms, a substituted or unsubstituted alkoxy group having 1 to 30 carbon atoms, a substituted or unsubstituted aryloxy group having 6 to 30 ring carbon atoms, a mono, di or tri-substituted silyl group having a substituent selected from a substituted or unsubstituted alkyl group having 1 to 30 carbon atoms and a substituted or unsubstituted aryl group having 6 to 30 ring carbon atoms, a substituted or unsubstituted haloalkyl group having 1 to 30 carbon atoms, a substituted or unsubstituted haloalkoxy group having 1 to 30 carbon atoms, and a substituted or unsubstituted heteroaryl group having 5 to 30 ring atoms,
provided that adjacent two selected from R²¹ to R²⁷ are each independently a hydrogen atom or the substituent, or the adjacent two are bonded to each other to form a ring structure;
Ar is a substituted or unsubstituted aryl group having 6 to 30 ring carbon atoms;
L¹ is a single bond or L¹¹, wherein L¹¹ is a substituted or unsubstituted arylene group having 6 to 30 ring carbon atoms;
L² is a single bond or L²², wherein L²² is a substituted or unsubstituted arylene group having 6 to 30 ring carbon atoms; and
L³ is a single bond or L³³, wherein L³³ is a substituted or unsubstituted arylene group having 6 to 30 ring carbon atoms.

2. The compound according to claim 1, wherein the compound is represented by formula (2): 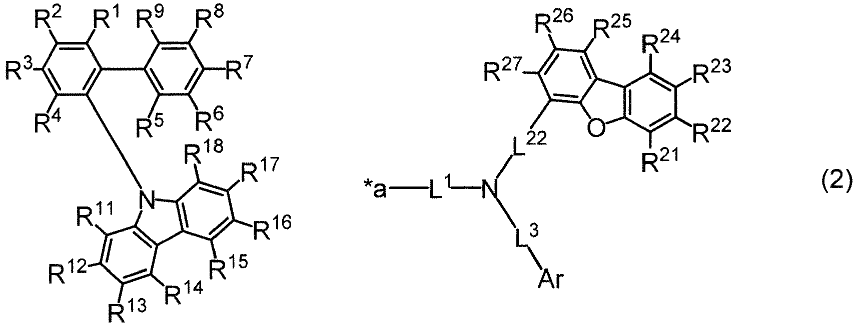 wherein R¹ to R⁹, R¹¹ to R¹⁸, R²¹ to R²⁷, Ar, L¹, L²², and L³ are as defined in formula (1).

3. The compound according to claim 1 or 2, wherein the compound is represented by any of formulae (3) to (6): 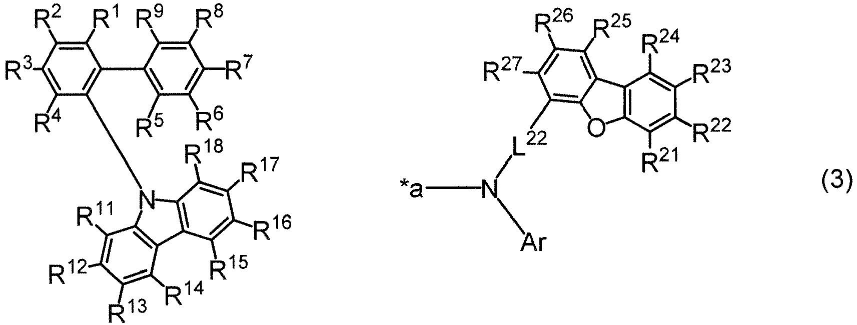 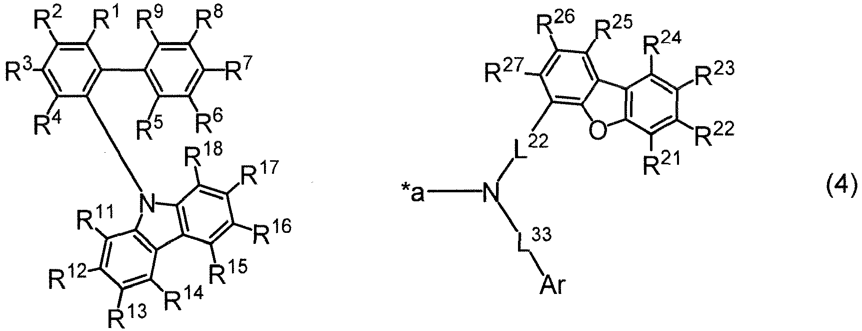 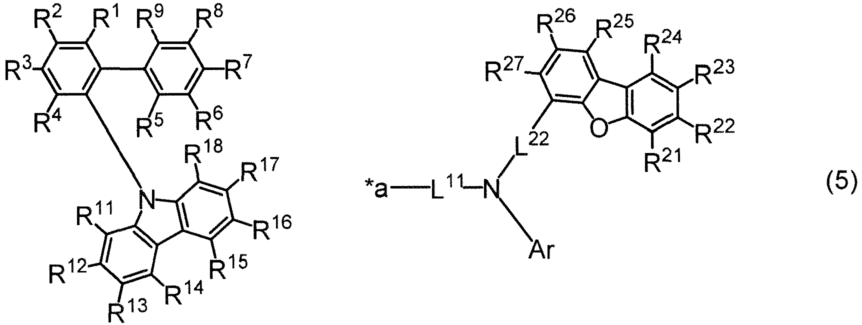 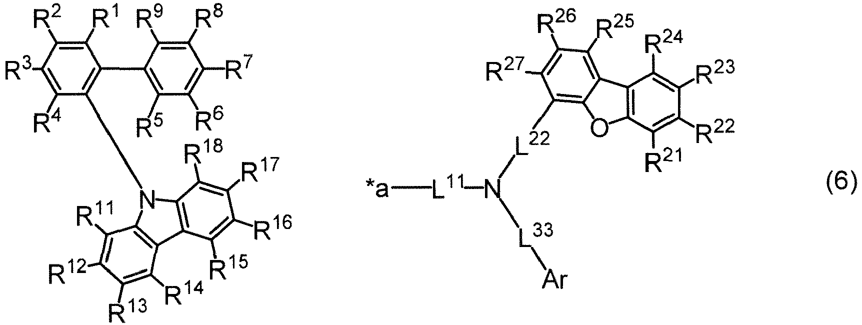 wherein R¹ to R⁹, R¹¹ to R¹⁸, R²¹ to R²⁷, Ar, L¹¹, L²², and L³³ are as defined in formula (1).

4. The compound according to any one of claims 1 to 3, wherein the compound is represented by formula (3) or (4).

5. The compound according to any one of claims 1 to 4, wherein R⁷ or R⁸ is a single bond bonded to *a.

6. The compound according to claim 1 or 5, wherein the compound is represented by formula (7): 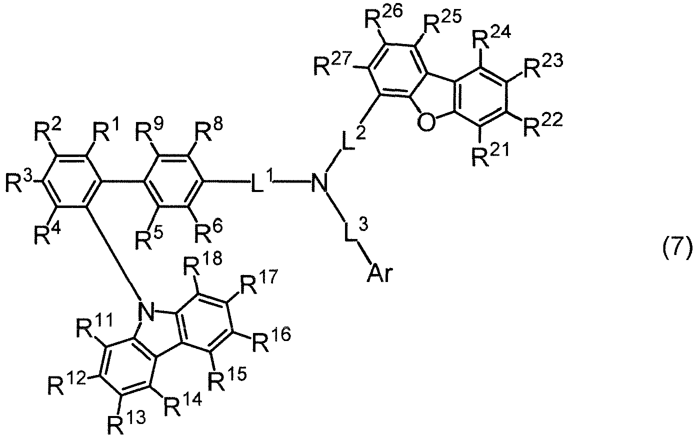 wherein R¹ to R⁶, R⁸, R⁹, R¹¹ to R¹⁸, R²¹ to R²⁷, Ar, L¹, L², and L³ are as defined in formula (1).

7. The compound according to any one of claims 1 to 6, wherein the compound is represented by formula (8): 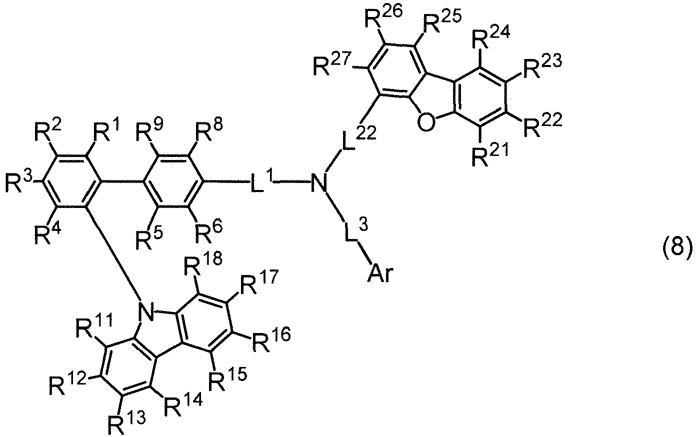 wherein R¹ to R⁶, R⁸, R⁹, R¹¹ to R¹⁸, R²¹ to R²⁷, Ar, L¹, L²², and L³ are as defined in formula (1).

8. The compound according to any one of claims 1 to 7, wherein the aryl group having 6 to 30 ring carbon atoms of the substituted or unsubstituted aryl group having 6 to 30 ring carbon atoms represented by Ar is selected from the group consisting of a phenyl group, a biphenylyl group, a terphenylyl group, a biphenylenyl group, a naphthyl group, an anthryl group, a benzanthryl group, a phenanthryl group, a benzophenanthryl group, a phenalenyl group, a picenyl group, a pentaphenyl group, a pyrenyl group, a chrysenyl group, a benzochrysenyl group, a fluorenyl group, a fluoranthenyl group, a perylenyl group, and a triphenylenyl group.

9. The compound according to any one of claims 1 to 8, wherein the aryl group having 6 to 30 ring carbon atoms of the substituted or unsubstituted aryl group having 6 to 30 ring carbon atoms represented by Ar is selected from the group consisting of a phenyl group, a biphenylyl group, a terphenylyl group, a naphthyl group, a phenanthryl group, and a triphenylenyl group.

10. The compound according to any one of claims 1 to 9, wherein the arylene group having 6 to 30 ring carbon atoms of the substituted or unsubstituted arylene group having 6 to 30 ring carbon atoms represented by any of L¹¹ to L³³ are each independently selected from the group consisting of a phenylene group, a biphenylene group, a terphenylene group, a biphenylenylene group, a naphthylene group, an anthrylene group, a benzanthrylene group, a phenanthrylene group, a benzophenanthrylene group, a phenalenylene group, a picenylene group, a pentaphenylene group, a pyrenylene group, a chrysenylene group, a benzochrysenylene group, a fluorenylene group, a fluoranthenylene group, a perylenylene group, and a triphenylenylene group.

11. The compound according to any one of claims 1 to 10, wherein the arylene group having 6 to 30 ring carbon atoms of the substituted or unsubstituted arylene group having 6 to 30 ring carbon atoms represented by L¹¹ is selected from the group consisting of a phenylene group, a biphenylene group, and a naphthylene group.

12. The compound according to any one of claims 1 to 11, wherein the arylene group having 6 to 30 ring carbon atoms of the substituted or unsubstituted arylene group having 6 to 30 ring carbon atoms represented by L²² is selected from the group consisting of a phenylene group, a biphenylene group, a naphthylene group, and a phenanthrylene group.

13. The compound according to any one of claims 1 to 12, wherein the arylene group having 6 to 30 ring carbon atoms of the substituted or unsubstituted arylene group having 6 to 30 ring carbon atoms represented by L³³ is a phenylene group.

14. The compound according to any one of claims 1 to 13, wherein L¹ is a single bond.

15. The compound according to any one of claims 1 to 14, wherein R¹ to R⁶ and R⁷ to R⁹ not the single bond bonded to *a are each independently selected from the group consisting of a hydrogen atom or a substituent,
wherein the substituent is selected from the group consisting of a substituted or unsubstituted alkyl group having 1 to 30 carbon atoms, a substituted or unsubstituted cycloalkyl group having 3 to 30 ring carbon atoms, a substituted or unsubstituted aryl group having 6 to 30 ring carbon atoms, a mono, di or tri-substituted silyl group having a substituent selected from a substituted or unsubstituted alkyl group having 1 to 30 carbon atoms and a substituted or unsubstituted aryl group having 6 to 30 ring carbon atoms, and a substituted or unsubstituted heteroaryl group having 5 to 30 ring atoms.

16. The compound according to any one of claims 1 to 15, wherein R¹¹ to R¹⁸ are each independently a hydrogen atom or a substituent,
wherein the substituent is selected from the group consisting of a substituted or unsubstituted alkyl group having 1 to 30 carbon atoms, a substituted or unsubstituted cycloalkyl group having 3 to 30 ring carbon atoms, a substituted or unsubstituted aryl group having 6 to 30 ring carbon atoms, a mono, di or tri-substituted silyl group having a substituent selected from a substituted or unsubstituted alkyl group having 1 to 30 carbon atoms and a substituted or unsubstituted aryl group having 6 to 30 ring carbon atoms, and a substituted or unsubstituted heteroaryl group having 5 to 30 ring atoms.

17. The compound according to any one of claims 1 to 16, wherein R²¹ to R²⁷ are each independently a hydrogen atom or a substituent,
wherein the substituent is selected from the group consisting of a substituted or unsubstituted alkyl group having 1 to 30 carbon atoms, a substituted or unsubstituted cycloalkyl group having 3 to 30 ring carbon atoms, a substituted or unsubstituted aryl group having 6 to 30 ring carbon atoms, a mono, di or tri-substituted silyl group having a substituent selected from a substituted or unsubstituted alkyl group having 1 to 30 carbon atoms and a substituted or unsubstituted aryl group having 6 to 30 ring carbon atoms, and a substituted or unsubstituted heteroaryl group having 5 to 30 ring atoms.

18. The compound according to any one of claims 1 to 17, wherein adjacent two selected from R¹ to R⁶ and R⁷ to R⁹ not the single bond bonded to *a do not form a ring structure.

19. The compound according to any one of claims 1 to 18, wherein adjacent two selected from R¹¹ to R¹⁸ do not form a ring structure.

20. The compound according to any one of claims 1 to 19, wherein adjacent two selected from R²¹ to R²⁷ do not form a ring structure.

21. The compound according to any one of claims 1 to 20, wherein R¹ to R⁶ and R⁷ to R⁹ not the single bond bonded to *a are all hydrogen atoms.

22. The compound according to any one of claims 1 to 21, wherein R¹¹ to R¹⁸ are all hydrogen atoms.

23. The compound according to any one of claims 1 to 22, wherein R²¹ to R²⁷ are all hydrogen atoms.

24. A material for organic electroluminescence device which comprises the compound according to any one of claims 1 to 23.

25. An organic electroluminescence device comprising a cathode, an anode, and an organic layer disposed between the cathode and the anode, wherein the organic layer comprises a light emitting layer and at least one layer of the organic layer comprises the compound according to any one of claims 1 to 23.

26. The organic electroluminescence device according to claim 25, wherein:
the organic layer comprises a hole transporting region between the anode and the light emitting layer; and
the hole transporting region comprises the compound.

27. The organic electroluminescence device according to claim 26, wherein:
the hole transporting region comprises a first hole transporting layer at anode side and a second hole transporting layer at cathode side; and
the first hole transporting layer, the second hole transporting layer, or both thereof comprises the compound.

28. The organic electroluminescence device according to claim 27, wherein the first hole transporting layer comprises the compound.

29. The organic electroluminescence device according to claim 27, wherein the second hole transporting layer comprises the compound.

30. The organic electroluminescence device according to any one of claims 27 to 29, wherein the second hole transporting layer is in contact with the light emitting layer.

31. The organic electroluminescence device according to any one of claims 25 to 30, wherein the light emitting layer comprises a fluorescent dopant material.

32. The organic electroluminescence device according to any one of claims 25 to 30, wherein the light emitting layer comprises a phosphorescent dopant material.

33. An electronic device comprising the organic electroluminescence device according to any one of claims 25 to 32.
